# EUROPÄISCHE PATENTSCHRIFT

(11) **EP 1 971 581 B1**
(45) Veröffentlichungstag und Bekanntmachung des Hinweises auf die Patenterteilung: **07.04.2010**
(21) Anmeldenummer: 07703628.3
(22) Anmeldetag: 04.01.2007
(51) Int. Cl.: C07D 241/08, C07D 241/18, C07D 403/06, C07D 409/06, C07F 7/08, A01N 37/46

(54) **PIPERAZINVERBINDUNGEN MIT HERBIZIDER WIRKUNG**
PIPERAZINE COMPOUNDS WITH A HERBICIDAL ACTION
COMPOSES A BASE DE PIPERAZINE A ACTION HERBICIDE

(30) Priorität: 05.01.2006 EP 06000185
(43) Veröffentlichungstag der Anmeldung: 24.09.2008
(73) Patentinhaber: BASF SE, 67056 Ludwigshafen (DE)
(72) Erfinder: HUPE, Eike, 67067 Ludwigshafen (DE); ZAGAR, Cyrill, Kowloon Hong Kong (CN); WITSCHEL, Matthias, 67098 Bad Dürkheim (DE); KÜHN, Toralf, 68199 Mannheim (DE); MOBERG, William Karl, 67454 Hassloch (DE); PARRA RAPADO, Liliana, 77654 Offenburg (DE); STELZER, Frank, 68309 Mannheim (DE); VESCOVI, Andrea, 68167 Mannheim (DE); REINHARD, Robert, 67117 Limburgerhof (DE); SIEVERNICH, Bernd, 67454 Hassloch (DE); GROSSMANN, Klaus, 67141 Neuhofen (DE); EHRHARDT, Thomas, 67346 Speyer (DE)
(74) Vertreter: Reitstötter - Kinzebach
(86) Internationale Anmeldenummer: PCT/EP2007/050067
(87) Internationale Veröffentlichungsnummer: WO 2007/077247

(56) Entgegenhaltungen:
- RUSSELL R. KING, C. HAROLD LAWRENCE: "Herbicidal Properties of the Thaxtomin Group of Phytotoxins" J. AGRIC. FOOD CHEM., Bd. 49, 2001, Seiten 2298-2301, XP002370714 in der Anmeldung erwähnt

## Beschreibung

Die vorliegende Erfindung betrifft die Verwendung von Piperazinverbindungen der Formel I 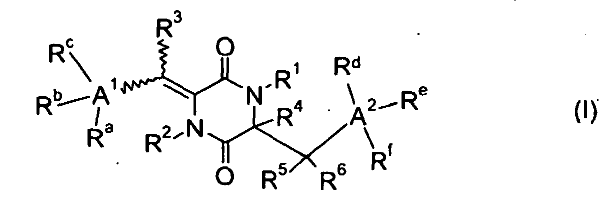 oder der landwirtschaftlich brauchbaren Salze von Piperazinverbindungen der Formel I als Herbizide, wobei die Variablen in Formel I die folgenden Bedeutungen haben:
R¹ und R² unabhängig voneinander
   Cyano, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocyclyl-(C₁-C₆)-alkyl; oder
   COR²¹, wobei
      R²¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet; oder
   NR²²R²³, wobei
      R22 und R²³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl oder C₁-C₆-Alkylcarbonyl bedeuten; oder
   OR²⁴, wobei
      R²⁴ C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl oder Phenyl-(C₁-C₆)-Alkyl bedeutet; oder
   SO₂R²⁵, wobei R²⁵ C₁-C₆-Alkyl oder Phenyl bedeutet;
   wobei die genannten aliphatischen, cyclischen oder aromatischen Teile der Substituenten von R¹ und R² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
   und wobei R¹ zusätzlich Wasserstoff bedeuten kann;
   - R³: Wasserstoff, Halogen, Cyano, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl; oder ein Rest COR²⁶, NR²⁷R²⁸, OR²⁹, SO₂R³⁰ oder N(OR³¹)R³², wobei
   R²⁶ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet;
   R²⁷ und R²⁸ unabhängig voneinander Wasserstoff, C₁-C₆-Alkyl, Aryl oder Heteroaryl-bedeuten;
   R²⁹ C₁-C₆-Alkyl bedeutet;
   R³⁰ C₁-C₆-Alkyl oder Phenyl bedeutet;
   R³¹ Wasserstoff, C₁-C₆-Alkyl, Phenyl oder Phenyl-(C₁-C₆)-alkyl bedeutet;
   R³² C₁-C₆-Alkyl, Phenyl oder Phenyl-(C₁-C₆)-alkyl bedeutet;
   wobei die genannten aliphatischen, cyclischen oder aromatischen Teile der Substituenten von R³ bzw. R²⁶, R²⁷, R²⁸, R²⁹, R³⁰, R³¹ und R³² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
   - R⁴, R⁵, R⁶: unabhängig voneinander Wasserstoff, Hydroxy, C₁-C₆-Alkyl, C₁-C₆-Alkoxy, wobei die genannten aliphatischen Teile der Substituenten von R⁴, R⁵ oder R⁶ partiell oder vollständig halogeniert seln können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
   - A¹: Aryl oder Heteroaryl;
   - A²: Aryl oder Heteroaryl, ausgenommen Indolyl;
   - R^{a}: Halogen, Cyano, Nitro, C₁-C₆-Alkyl, C₃-C₆-Cycloalkyl, C₂-C₆-Alkenyl, C₃-C₆-Cycloalkenyl, C₄-C₁₀-Alkadienyl, C₂-C₆-Alkinyl, [Tri-(C₁-C₆)-alkylsilyl]-(C₂-C₆)-alkinyl, C₃-C₆-Cycloalkinyl, C₁-C₆-Alkylthio, C₁-C₆-Alkylsulfinyl, Aryl, Phenyl-(C₁-C₆)-Alkyl, Phenyl-(C₂-C₆)-Alkenyl, Phenylsulfonyl-(C₁-C₆)-Alkyl, Heterocyclyl, Heterocyclyl-(C₁-C₆)-Alkyl oder Phenyl-[C₁-C₆-alkoxycarbonyl]-(C₁-C₆)-alkyl,
   Z¹P(O)(OR⁹)₂, Z²B(OR¹⁰)₂, wobei
   R⁹ und R¹⁰ jeweils Wasserstoff oder C₁-C₆-Alkyl bedeuten und R¹⁰ in Z²B(OR¹⁰)₂ zusammen eine C₂-C₄-Alkylenkette bilden können; oder
   Z³COR¹¹, wobei
   R¹¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, [Di-(C₁-C₆)-Alkoxy]amino, C₁-C₆-Alkylsulfonylamino, C₁-C₆-Alkylaminosulfonylamino, [Di-(C₁-C₆)-alkylamino]sulfonylamino, C₃-C₆-Alkenylamino, C₃-C₆-Alkinylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenoxy, Phenylamino, Naphthyl oder Heterocyclyl bedeutet; oder
   Z⁴NR¹²R¹³, wobei
   R¹² und R¹³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₃-C₆-Cycloalkylcarbonyl, [Di-(C₁-C₆)-alkylamino]carbonyl, C₁-C₆-Alkoxycarbonyl, C₁-C₆-Alkoxycarbonyl-(C₁-C₆)-alkyl, C₁-C₆-Alkylsulfonyl, C₁-C₆-Alkylaminosulfonyl, [Di-(C₁-C₆)-alkylamino]sulfonyl, Phenylcarbonyl, Phenylaminocarbonyl, Phenylsulfonyl, Phenylsulfonylaminocarbonyl oder Heterocyclylcarbonyl bedeuten; oder
   Z⁵CH=N-O-R¹⁴, wobei R¹⁴ Wasserstoff oder C₁-C₆-Alkyl bedeutet; oder
   Z⁶OR¹⁵, wobei
   R¹⁵ Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₁-C₆-Alkoxycarbonyl-(C₁-C₆)-alkyl, [Di-(C₁-C₆)-Alkoxycarbonyl]-(C₁-C₆)-alkyl, Phenyl oder Phenyl-(C₁-C₆)-Alkyl bedeutet; oder
   Z⁷SO₂R¹⁶, wobei R¹⁶ C₁-C₆-Alkyl oder Phenyl bedeutet; und wobei
   Z¹, Z², Z³, Z⁴, Z⁵, Z⁶, Z⁷ unabhängig voneinander eine Bindung, -CH₂-, -CH₂-CH₂-, -O-CH(R¹⁷)-, -S-CH(R¹⁸)-, -S(O)-CH(R¹⁹)- oder -SO₂CH(R²⁰)- bedeutet, und wobei R¹⁷, R'8, R¹⁹ und R²⁰ unabhängig voneinander Wasserstoff oder C₁-C₆-Alkyl bedeuten; und
wobei die genannten aliphatischen, cyclischen oder aromatischen Teile des Substituenten R^{a} partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy; und
- R^{b}, R^{c}, R^{d}, R^{e} und R^{f}: jeweils unabhängig voneinander für Wasserstoff stehen oder eine der für R^{a} angegebenen Bedeutungen aufweisen; und
worin zwei an benachbarte Ringatome von A¹ gebundene Reste R^{a}, R^{b} oder R^{c} oder zwei an benachbarte Ringatome von A² gebundene Reste R^{d}, Re oder R^{f} auch für lineares C₃-C₆-Alkylen stehen können, das teilweise oder vollständig halogeniert sein kann und das eine bis drei der folgenden Gruppen tragen kann: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy, worin eine CH₂-Gruppe in C₃-C₆-Alkylen durch eine Carbonylgruppe, Thiocarbonylgruppe oder Sulfonylgruppe ersetzt sein kann und worin eine oder zwei nicht benachbarte CH₂-Gruppen in C₃-C₆-Alkylen jeweils durch Sauerstoff, Schwefel oder eine Gruppe NR³⁴ ersetzt sein können, wobei R³⁴ eine für R¹² angegebenen Bedeutungen aufweist.

Bei den von dem Pflanzenpathogen S. scabies produzierten Thaxtominen A und B (King R. R. et al., J. Agric. Food Chem. (1992) 40, 834-837) handelt es sich um Naturstoffe mit einem zentralen Piperazin-2,5-dion-Ring, der in der 3-Position einen 4-Nitro-indol-3-ylmethyl-Rest und in der 2-Position einen gegebenenfalls durch OH substituierten Benzyl-Rest trägt. Aufgrund ihrer pflanzenschädigenden Wirkung wurde auch die Verwendungsmöglichkeit dieser Verbindungsklasse als Herbizide untersucht (King R. R. et al., J. Agric. Food Chem. (2001) 49, 2298-2301).

J. Gelin et al., J. Org. Chem. 58; 1993, S. 3473-3475, und J. Moyroud et al., Tetrahedron 52, 1996, S. 8525-8543 beschreiben im Rahmen von synthetischen Untersuchungen zur Herstellung von Thaxtomin A und B Dehydrothaxtomin-Derivate. Unter anderem werden Verbindungen der Formel beschrieben 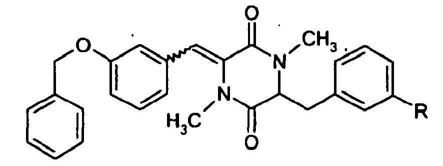 worin R für Wasserstoff oder NO₂ steht.

N. Saito et al., J. Chem. Soc. Perkin Trans 1997, S. 53-69 beschreiben unter anderem Verbindungen der folgenden Formel 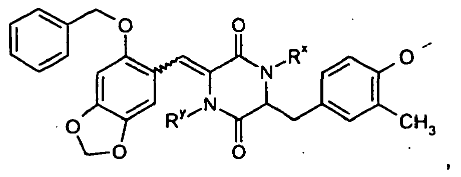 worin R^{y} für Wasserstoff oder Benzyl steht und R^{x} für Wasserstoff, Acetyl oder Isopropyloxycarbonyl steht, als Vorstufen für die Herstellung von Ecteinascidinen.

Z.Z. Liu et al., Chinese Chem. Lett. 13(8) 2002, S. 701-704 beschreiben im Rahmen synthetischer Untersuchungen zur Herstellung von Phthalascidin eine Zwischenstufe der folgenden Formel, worin Bn für Benzyl steht: 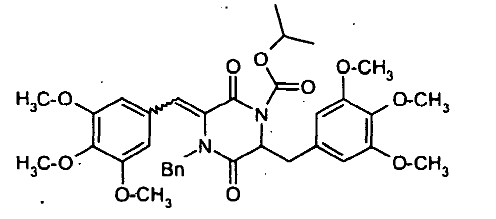 J. Bryans et al., Journal of Antibiotics 49(10), 1996,S. 1014-1021 beschreiben die Verbindung der folgenden Formel:

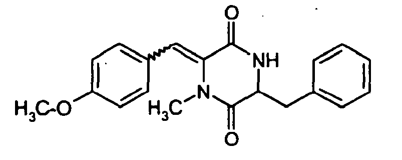

Die WO 99/48889, WO 01/53290 und WO 2005/011699 beschreiben 2,5-Diketopiperazinverbindungen, die in der 3- bzw. 6-Position einen über eine Methylen- oder Methingruppe gebundenen 4-Imidazolyl-Rest und in der anderen 3- bzw. 6-Position einen Benzyl- oder Benzylidenrest aufweisen. Bei diesen Verbindungen handelt es sich um Antitumor-Wirkstoffe.

Eine Aufgabe der vorliegenden Erfindung ist die Bereitstellung von Verbindungen mit herbizider Wirkung. Insbesondere sollen Verbindungen zur Verfügung gestellt werden, die eine hohe herbizide Wirkung, insbesondere bereits bei niedrigen Aufwandmengen, aufweisen und deren Verträglichkeit gegenüber Kulturpflanzen für eine kommerzielle Verwertung hinreichend ist.

Diese und weitere Aufgaben werden durch die eingangs definierten Verbindungen der Formel I und durch ihre landwirtschaftlich geeigneten Salze gelöst.

Dementsprechend betrifft die vorliegende Erfindung die Verwendung von Piperazinverbindungen der allgemeinen Formel I oder der landwirtschaftlich brauchbaren Salze von Piperazinverbindungen der Formel I als Herbizide, d.h. zur Bekämpfung von Schadpflanzen.

Die Erfindung betrifft auch Mittel, welche eine herbizid wirksame Menge mindestens einer Piperazinverbindung der Formel I oder eines landwirtschaftlich brauchbaren Salzes von I und für die Formulierung von Pflanzenschutzmitteln übliche Hilfsmittel enthalten.

Die Erfindung betrifft außerdem ein Verfahren zur Bekämpfung von unerwünschtem Pflanzenwuchs, bei dem man eine herbizid wirksame Menge mindestens einer Piperazinverbindung der Formel I oder eines landwirtschaftlich brauchbaren Salzes von I auf Pflanzen, deren Samen und/oder deren Lebensraum einwirken läßt.

Die Piperazinverbindungen der Formel I sind neu und ebenfalls Gegenstand der vorliegenden Erfindung,
- ausgenommen Verbindungen der Formel I, worin A¹ für Phenyl steht und A² 4-Imidazolyl bedeutet oder A¹ für 4-Imidazolyl steht und A² Phenyl bedeutet,
- weiterhin ausgenommen die Verbindung der Formel I, worin R¹ für Wasserstoff steht und R² Methyl bedeutet, R³, R⁴, R⁵ und R⁶ Wasserstoff bedeuten, und die Gruppe A¹(R^{a}R^{b}R^{c}) für 4-Methoxyphenyl steht und die Gruppe A²(R^{d}R^{e}R^{f}) für Phenyl steht,
- weiterhin ausgenommen Verbindungen der Formel I, worin A¹ für Phenyl steht, R¹ und R² für Methyl stehen, R³, R⁴, R⁵ und R⁶ Wasserstoff bedeuten, R^{a} Benzyloxy, das in der 3-Position gebunden ist, bedeutet, R^{b} und R^{c} für Wasserstoff stehen, und die Gruppe A²(R^{d}R^{e}R^{f}) für Phenyl oder 3-Nitrophenyl steht,
- weiterhin ausgenommen Verbindungen der Formel I, worin R¹ für Wasserstoff, Acetyl oder Isopropyloxycarbonyl und steht, R² für Wasserstoff oder Benzyl steht, R³, R⁴, R⁵ und R⁶ Wasserstoff bedeuten, die Gruppe A¹(R^{a}R^{b}R^{c}) für einen Rest der Formel 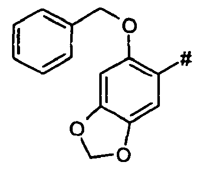 steht, wobei # die Verknüpfung mit dem Methinkohlenstoff, welcher R³ trägt, angibt, und die Gruppe A²(R^{d}R^{e}R^{f}) für 3-Methyl-4-methoxyphenyl steht,
- weiterhin ausgenommen die Verbindung der Formel I, worin R¹ für Isopropyloxycarbonyl steht und R² für Benzyl steht R³, R⁴, R⁵ und R⁶ Wasserstoff bedeuten, und die Gruppen A¹(R^{a}R^{b}R^{c}) und A²(R^{d}R^{e}R^{f}) jeweils für für 3,4,5-Trimethoxyphenyl stehen.

Die Erfindung betrifft außerdem Verfahren und Zwischenprodukte zur Herstellung von Verbindungen der Formel I.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können bezüglich der exocyclischen Doppelbindung als E-Isomer oder als Z-Isomer vorliegen. Gegenstand der Erfindung sind sowohl die reinen E-Isomere und Z-Isomere als auch deren Gemische.

Die Verbindungen der Formel I können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium verwendet werden, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄-alkyl)sulfonium oder Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat oder Butyrat.

Die für die Substituenten der erfindungsgemäßen Verbindungen genannten organischen Molekülteile stellen Sammelbegriffe für individuelle Aufzählungen der einzelnen Gruppenmitglieder dar. Sämtliche Kohlenwasserstoffketten, wie
Alkyl-, Halogenalkyl-, sowie die Alkylteile in Cyanoalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl, N,N-Dialkylaminosulfonyl, Dialkylamino-, N-Alkylsulfonylamino, N-Halogenalkylsulfonylamino, N-Alkyl-N-alkylsulfonylamino, N-Alkyl-N-halogenalkylsulfonylamino, Alkylcarbonyl-, Halogenalkylcarbonyl-, Alkoxycarbonyl-, Halogenalkoxycarbonyl, Alkylcarbonyloxy-, Alkylaminocarbonyl-, Dialkylaminocarbonyl-, Dialkylaminothiocarbonyl, Alkoxyalkyl-, Dialkoxyalkyl-, Alkylthioalkyl-, Dialkylaminoalkyl-, Dialkylhydrazinoalkyl-, Alkyliminooxyalkyl-, Alkylcarbonylalkyl, Alkoxyiminoalkyl, N-(Alkylamino)-iminoalkyl, N-(Dialkylamino)-iminoalkyl, Alkoxycarbonylalkyl-, Dialkylaminocarbonylalkyl-, Phenylalkenylcarbonyl, Heterocyclylalkenylcarbonyl, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl- und Afkoxyalkoxy-Teile
können geradkettig oder verzweigt sein. Das Präfix Cₙ-Cₘ- gibt die jeweilige Kohlenstoffzahl der Kohlenwasserstoffeinheit an. Sofern nicht anders angegeben tragen halogenierte Substituenten vorzugsweise ein bis fünf gleiche oder verschiedene Halogenatome, insbesondere Fluoratome oder Chloratome.

Die Bedeutung Halogen steht jeweils für Fluor, Chlor, Brom oder Iod.

### Ferner bedeuten beispielsweise:

Alkyl sowie die Alkylteile beispielsweise in Alkoxy, Alkylthio, Alkylsulfinyl und Alkylsulfonyl, Alkylcarbonyl, Alkylamino, Alkylsilyl, Phenylalkyl, Phenylsulfonylalkyl, Heterocyclylalkyl : gesättigte, geradkettige oder verzweigte Kohlenwasserstoffreste mit einem oder mehr C-Atomen, z.B. 1 bis 2, 1 bis 4, oder 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl. In einer erfindungsgemäßen Ausführungsform steht Alkyl für kleine Alkylgruppen wie C₁-C₄-Alkyl. In einer anderen erfindungsgemäßen Ausführungsform steht Alkyl für größere Alkylgruppen wie C₅-C₆-Alkyl.

Halogenalkyl (auch als Haloalkyl bezeichnet): einen Alkylrest wie vorstehend genannt, dessen Wasserstoffatome partiell oder vollständig durch Halogenatome wie Fluor, Chlor, Brom und/oder Iod substituiert sind, z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl.

Cycloalkyl sowie die Cycloalkylteile beispielsweise in Cycloalkoxy oder Cycloalkylcarbonyl: monocyclische, gesättigte Kohlenwasserstoffgruppen mit drei oder mehr C-Atomen, z.B. 3 bis 6 Kohlenstoffringgliedern, wie Cyclopropyl, Cyclobutyl, Cyclopentyl und Cyclohexyl.

Alkenyl sowie Alkenylteile beispielsweise in Phenyl-(C₂-C₆)-Alkenyl oder Alkenylamino: einfach ungesättigte, geradkettige oder verzweigte Kohlenwasserstoffreste mit zwei oder mehr C-Atomen, z. B. 2 bis 4, 2 bis 6 oder 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1 ,2-Dimethyl-1-propenyl, 1,2-Dimethyl-2-propenyl, 1-Ethyl-1 propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

In einer erfindungsgemäßen Ausführungsform werden Alkenylgruppen wie C₂-C₆-Alkenyl verwendet. In einer anderen erfindungsgemäßen Ausführungsform werden Alkenylgruppen wie C₃-C₆-Alkenyl eingesetzt.

Cycloalkenyl sowie Cycloalkenylteile: monocyclische, einfach ungesättigte Kohlenwasserstoffgruppen mit drei oder mehr C-Atomen, z. B. 3 bis 6, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl.

Alkinyl sowie Alkinylteile beispielsweise in [Tri-(C₁-C₆)-alkylsilyl-(C₂-C₆)-alkinyl oder Alkinylamino: geradkettige oder verzweigte Kohlenwasserstoffgruppen mit zwei oder mehr C-Atomen, z. B. 2 bis 4, 2 bis 6, oder 3 bis 6 Kohlenstoffatomen und einer oder zwei Dreifachbindungen in beliebiger, jedoch nicht benachbarter Position, z. B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Cycloalkinyl sowie Cycloalkinylteile: monocyclische Kohlenwasserstoffgruppen mit drei oder mehr C-Atomen, z. B. 3 bis 6, vorzugsweise 5 bis 6 Kohlenstoffringgliedern und einer Dreifachbindung, wie Cyclohexin-1-yl, Cyclohexin-3-yl, Cyclohexin-4-yl.

C₄-C₁₀-Alkadienyl: zweifach ungesättigte, geradkettige oder verzweigte Kohlenwasserstoffreste mit vier oder mehr C-Atomen und zwei Doppelbindungen in einer beliebigen, jedoch nicht benachbarten Position, z. B. 4 bis 10 Kohlenstoffatomen und zwei Doppelbindungen in einer beliebigen, jedoch nicht benachbarten Position, z. B. 1,3-Butadienyl, 1-Methyl-1,3-butadienyl, 2-Methyl-1,3-butadienyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl.

Alkoxy oder Alkoxyteile beispielsweise in Phenylalkoxy, Alkoxyamino, Alkoxycarbonyl: Alkyl, wie vorstehend definiert, das über ein O-Atom gebunden ist: z. B. Methoxy, Ethoxy, n-Propoxy, 1-Methylethoxy, Butoxy, 1-Methylpropoxy, 2-Methylpropoxy oder 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

In einer erfindungsgemäßen Ausführungsform werden kleine Alkoxygruppen wie C₁-C₄-Alkoxy verwendet. In einer anderen erfindungsgemäßen Ausführungsform werden größere Alkoxygruppen wie C₅-C₆-Alkoxy eingesetzt.

Alkenyloxy: Alkenyl wie vorstehend genannt, das über ein Sauerstoffatom gebunden ist, z. B. C₃-C₆-Alkenyloxy wie 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy. In einer erfindungsgemäßen Ausführungsform werden kleine Alkenyloxygruppen wie C₃-C₄-Alkenyloxy verwendet. In einer anderen erfindungsgemäßen Ausführungsform werden größere Alkenyloxygruppen wie C₅-C₆-Alkenyloxy eingesetzt.

Alkinyloxy: Alkinyl wie vorstehend genannt, das über ein Sauerstoffatom gebunden ist, z. B. C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy. In einer erfindungsgemäßen Ausführungsform werden kleine Alkinyloxygruppen wie C₃-C₄-Alkinyloxy verwendet. In einer anderen erfindungsgemäßen Ausführungsform werden größere Alkinyloxygruppen wie C₅-C₆-Alkinyloxy eingesetzt.

Alkylthio: Alkyl, wie vorstehend definiert, das über ein S-Atom gebunden ist.

Alkylsulfinyl: Alkyl, wie vorstehend definiert, das über eine SO-Gruppe gebunden ist.

Alkylsulfonyl: Alkyl, wie vorstehend definiert, das über eine S(O)₂-Gruppe gebunden ist.

Alkylcarbonyl: Alkyl, wie vorstehend definiert, das über eine (C=O)-Gruppe gebunden ist, z. B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl, Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl.

Alkenylcarbonyl: Alkenyl, wie vorstehend definiert, das über eine (C=O)-Gruppe gebunden ist, z.B. 1-Ethenylcarbonyl.

Alkinylcarbonyl: Alkinyl, wie vorstehend definiert, das über eine (C=O)-Gruppe gebunden ist, z.B. 1-Propinylcarbonyl.

Heterocyclyl: ein mono- oder bicyclischer, gesättigter, partiell ungesättigter oder aromatischer heterocyclischer Ring mit drei oder mehr, z.B. 3 bis 10 Ring-Atomen, z. B. ein monocyclischer 3-, 4-, 5-, 6-oder 7-gliedriger heterocyclischer Ring, der ein bis vier gleiche oder verschiedene Heteroatome, ausgewählt aus der Gruppe Sauerstoff, Schwefel oder Stickstoff enthält, und über C oder N gebunden sein kann, z. B.
C-gebundene 3-4-gliedrige, gesättigte oder ungesättigt Ringe wie 2-Oxiranyl, 2-Oxetanyl, 3-Oxetanyl, 2-Aziridinyl, 3-Thiethanyl, 1-Azetidinyl, 2-Azetidinyl.
C-gebundene, 5-gliedrige, gesättigte Ringe wie Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl.
C-gebundene, 6-gliedrige, gesättigte Ringe wie:
   Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl.
N-gebundene, 5-gliedrige, gesättigte Ringe wie:
   Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl.
N-gebundene, 6-gliedrige, gesättigte Ringe wie:
   Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl.
C-gebundene, 5-gliedrige, partiell ungesättigte Ringe wie:
   2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydro-thien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1H-pyrazol-3-yl, 4,5-Dihydro-1H-pjrrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ ³-1,2-Dithiol-3-yl, Δ ³-1,2-Dithiol-4-yl, Δ ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, 1,2,3-Δ ²-Oxadiazolin-4-yl, 1,2,3-Δ ²-Oxadiazolin-5-yl, 1,2,4-Δ ⁴-Oxadiazolin-3-yl, 1,2,4-Δ ⁴-Oxadiazolin-5-yl, 1,2,4-Δ ²-Oxadiazolin-3-yl, 1,2,4-Δ ²-Oxadiazolin-5-yl, 1,2,4-Δ ³-Oxadiazolin-3-yl, 1,2,4-Δ ³-Oxadiazolin-5-yl, 1,3,4-Δ ²-Oxadiazolin-2-yl, 1,3,4-Δ ²-Oxadiazolin-5-yl, 1,3,4-Δ ³-Oxadiazolin-2-yl, 1,3,4-Oxadiazolin-2-yl, 1,2,4-Δ ⁴-Thiadiazolin-3-yl, 1,2,4-Δ ⁴-Thiadiazolin-5-yl, 1,2,4-Δ ³-Thiadiazolin-3-yl, 1,2,4-Δ ³-Thiadiazolin-5-yl, 1,2,4-Δ ²-Thiadiazolin-3-yl, 1,2,4-Δ ²-Thiadiazolin-5-yl, 1,3,4-Δ ²-Thiadiazolin-2-yl, 1,3,4-Δ ²-Thiadiazolin-5-yl, 1,3,4-Δ ³-Thiadiazolin-2-yl, 1,3,4-Thiadiazolin-2-yl, 1,2,3-Δ ²-Triazolin-4-yl, 1,2,3-Δ ²-Triazolin-5-yl, 1,2,4-Δ ²-Triazolin-3-yl, 1,2,4-Δ ²-Triazolin-5-yl, 1,2,4-Δ ³-Triazolin-3-yl, 1,2,4-Δ ³-Triazolin-5-yl, 1,2,4-Δ ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl.
C-gebundene, 6-gliedrige, partiell ungesättigte Ringe wie:
   2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin 3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6 -Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1 ,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl.
N-gebundene, 5-gliedrige, partiell ungesättigte Ringe wie:
   2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ ⁴-Oxadiazolin-2-yl, 1,2,4-Δ ²-Oxadiazolin-4-yl, 1,2,4-Δ ³-Oxadiazolin-2-yl, 1,3,4-Δ ²-Oxadiazolin-4-yl, 1,2,4-Δ ⁵-Thiadiazolin-2-yl, 1,2,4-Δ ³-Thiadiazolin-2-yl, 1,2,4-Δ ²-Thiadiazolin-4-yl, 1,3,4-Δ ²-Thiadiazolin-4-yl, 1,2,3-Δ ²-Triazolin-1-yl, 1,2,4-Δ ²-Triazolin-1-yl, 1,2,4-Δ ²-Triazolin-4-yl, 1,2,4-Δ ³-Triazolin-1-yl, 1,2,4-Δ ¹-Triazolin-4-yl.
N-gebundene, 6-gliedrige, partiell ungesättigte Ringe wie:
   1,2,3,4-Tetrahydropyridin-1-yl, 1,2,5,6-Tetrahydropyridin-1-yl, 1,4-Dihydropyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-d-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl.
C-gebundene, 5-gliedrige, heteroaromatische Ringe mit in der Regel 1, 2, 3 oder 4 Stickstoffatomen oder einem unter Sauerstoff und Schwefel ausgewählten Heteratom und gegebenenfalls mit 1, 2 oder 3 Stickstoffatomen als Ringglieder wie: 2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl.
C-gebundene, 6-gliedrige, heteroaromatische Ringe mit in der Regel 1, 2, 3 oder 4 Stickstoffatomen als Ringglieder wie :
   Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl.
N-gebundene, 5-gliedrige, heteroaromatische Ringe mit in der Regel 1, 2, 3 oder 4 Stickstoffatomen als Ringglieder wie:
   Pyrrol-1-yl, Pyrazol-1-yl, Imidazol-1-yl, 1,2,3-Triazol-1-yl, 1,2,4-Triazol-1-yl, Tetrazol-1-yl.
oder ein bicyclischer Heterocyclus, der einen der vorgenannten 5- oder 6-gliedrigen heterocyclischen Ringe und einen weiteren, daran ankondensierten, gesättigten, ungesättigten oder aromatischen Carbocyclus, beispielsweise einen Benzol-, Cyclohexan, Cyclohexen oder Cyclohexadien-Ring, oder einen weiteren, daran ankondensierten 5- oder 6-gliedrigen heterocyclischen Ring aufweist, wobei letzterer ebenfalls gesättigt, ungesättigt oder aromatisch sein kann.

Ein Schwefelatom in den genannten Heterocyclen kann zu S=O oder S(=O)₂ oxidiert sein.

Dementsprechend stehen Hetaryl bzw. Hetaryl für einen 5- oder 6-gliedrigen heteroaromatischen Rest, der 1, 2, 3 oder 4 gleiche oder verschiedene Heteroatome, ausgewählt aus der Gruppe Sauerstoff, Schwefel oder Stickstoff, als Ringglieder aufweist, der über C oder N gebunden sein kann, und der mit einem weiteren ankondensierten Benzolring oder einem 5- bis 6-gliedrigen Heteroaromaten ein bicyclisches Ringsystem ausbilden kann. Beispiele für Hetaryl sind die vorgenannten C-gebundenen, 5- und 6-gliedrigen, heteroaromatischen Ringe, die vorgenannten N-gebundenen, 5-gliedrigen, heteroaromatischen Ringe, und bicyclische heteroaramatische Reste wie Chinolinyl, Isochinolinyl, Chinazolinyl, Chinoxalinyl, Indolyl, Benzothienyl, Benzofuryl, Benzoxazolyl, Benzthiazolyl, Benzimidazolyl, Benzpyrazolyl, Benzotriazol, Indolizinyl, 1,2,4-Triazolo[1,5-a]pyrimidinyl, 1,2,4-Triazolo[4,3-a]pyridinyl, Pyrazolo[3,4-b]pyridinyl, 1,2,4-Triazolo[1,5-a]pyridinyl, Imidazo[1,2-a]pyridyl, Imidazo[3,4-a]pyrimidinyl, und dergleichen.

Aryl: ein- oder mehrkerniger aromatischer Carbocyclus, z. B. ein- bis zweikerniger oder ein- bis dreikerniger aromatischer Carbocyclus mit 6 bis 14 Ringgliedern, wie z. B. Phenyl, Naphthyl oder Anthracenyl.

Arylalkyl: ein über eine Alkylengruppe, insbesondere über eine Methylen-, 1,1-Ethylen-oder 1,2-Ethylengruppe gebundener Arylrest, z.B. Benzyl, 1-Phenylethyl und 2-Phenylethyl.

Heterocyclylalkyl sowie Hetarylalkyl: ein über eine Alkylengruppe, insbesondere über eine Methylen-, 1,1-Ethylen-oder 1,2-Ethylengruppe gebundener Heterocyclyl-bzw. Hetarylrest.

In einer besonderen Ausführungsform haben die Variablen der Verbindungen der Formel I folgende Bedeutungen, wobei diese sowohl für sich allein betrachtet als auch in Kombination miteinander besondere Ausgestaltungen der Verbindungen der Formel I darstellen:
- R¹: Wasserstoff, Amino, Cyano, C₁-C₆-Alkyl, C₃-C₆-Alkenyl, C₃-C₆-Alkinyl, Phenyl-(C₁-C₆)-alkyl, Heterocyclyl-(C₁-C₆)-alkyl oder COR²¹, wobei R²¹ die zuvor genannten Bedeutungen aufweist und insbesondere für C₁-C₆-Alkyl, C₁-C₆-Alkoxy, Phenyl, Phenylamino oder Heterocyclyl steht; wobei die genannten aliphatischen, cyclischen oder aromatischen Teile der Substituenten partiell oder vollständig halogeniert sein können. Besonders bevorzugt hat R¹ die Bedeutungen Wasserstoff oder C₁-C₆-Alkyl, insbesondere Methyl.
- R²: Amino, Cyano, C₁-C₆-Alkyl, C₃-C₆-Alkenyl, C₃-C₆-Alkinyl, Phenyl-(C₁-C₆)-alkyl, Heterocyclyl-(C₁-C₆)-alkyl oder COR²¹, wobei R²¹ die zuvor genannten Bedeutunge aufweist und insbesondere für C₁-C₆-Alkyl, C₁-C₆-Alkoxy, Phenyl, Phenylamino oder Heterocyclyl steht; wobei die genannten aliphatischen, cyclischen oder aromatischen Teile der Substituenten partiell oder vollständig halogeniert sein können. Besonders bevorzugt hat R² die Bedeutung C₁-C₆-Alkyl, insbesondere Methyl.
- R³: Wasserstoff, Halogen, C₁-C₆-Alkyl oder Halo-C₁-C₆-alkyl.
- R⁴, R⁵: und/oder R⁶ Wasserstoff.
- A¹: steht für Aryl oder Heteroaryl, ausgewählt aus der Gruppe Phenyl, Naphthyl, Furyl, Thienyl, Pyrrolyl, Pyrazolyl, Thiazolyl, Isothiazolyl, Oxazolyl, Isoxazolyl, Triazolyl, Tetrazolyl, Pyridinyl, Pyridazinyl, Pyrimidinyl, Pyrazinyl, Triazinyl oder Tetrazinyl.
Insbesondere ist A¹ ausgewählt aus der Gruppe Phenyl, Furyl, Thienyl, Triazolyl, Tetrazolyl oder Pyridinyl.
Besonders bevorzugt hat A¹ die Bedeutung Phenyl oder Pyridinyl, insbesondere Phenyl.
In einer anderen bevorzugten Ausführungsform steht A¹ für einen bicyclischen aromatischen Rest, insbesondere für Napththyl oder einen der vorgenannten bicyclischen heteroaromatischen Reste wie Chinolinyl, Isochinolinyl, Chinazolinyl, Chinoxalinyl, Indolyl, Benzothienyl, Benzofuryl, Benzoxazolyl, Benzthiazolyl, Benzimidazolyl, Benzpyrazolyl, Benzotriazol, Indolizinyl, 1,2,4-Triazolo[1,5-a]pyrimidinyl, 1,2,4-Triazolo[4,3-a]pyridinyl, Pyrazolo[3,4-b]pyridinyl, 1,2,4-Triazolo[1,5-a]pyridinyl, Imidazo[1,2-a]pyridyl, Imidazo[3,4-a]pyrimidinyl, speziell für Indolyl, ganz speziell für 3-Indolyl.
- A²: steht für Aryl oder Heteroaryl, ausgewählt aus der Gruppe Phenyl, Naphthyl, Furyl, Thienyl, Pyrrolyl, Pyrazolyl, Thiazolyl, Isothiazolyl, Oxazolyl, Isoxazolyl, Triazolyl, Tetrazolyl, Pyridinyl, Pyridazinyl, Pyrimidinyl, Pyrazinyl, Triazinyl oder Tetrazinyl, insbesondere ausgewählt aus der Gruppe Phenyl, Furyl, Thienyl, Triazolyl, Tetrazolyl oder Pyridinyl. Besonders bevorzugt hat A² die Bedeutung Phenyl oder Thienyl, insbesondere Phenyl.

Erfindungsgemäß trägt A¹ einen von Wasserstoff verschiedenen Rest R^{a} wie zuvor definiert. Vorzugsweise ist dieser Rest in der ortho-Position zur Verknüpfungsstelle von A¹ an ein N- oder insbesondere an ein C-Atom von A¹ gebunden; diese ortho-Position ist unabhängig davon bevorzugt, ob es sich bei A¹ um einen ein- oder mehrkernigen aromatischen oder heteroaromatischen Ring handelt.

In einer alternativ bevorzugten Ausführungsform der Erfindung ist für den Fall, dass A¹ für einen mehrkernigen aromatischen oder heteroaromatischen Ring steht, der Rest R^{a} vorzugsweise nicht an denjenigen Kern des mehrkernigen Ringsystems gebunden, der die Verknüpfungsstelle von A¹ trägt, d.h. über den A¹ an die exocyclische Doppelbindung, die zum Piperazinring führt, gebunden ist (= "Verknüpfungskern"). Bei einem drei- oder höherkernigen Ringsystem ist R^{a} vorzugsweise an einen Kern, der dem "Verknüpfungskern" direkt benachbart ist, gebunden. Besonders bevorzugt ist R^{a} möglichst nahe zur Verknüpfungsstelle von A¹ gebunden. Insbesondere befindet sich R^{a} dabei in α-Position zu demjenigen Brückenkopfatom, das der Verknüpfungsstelle von A¹ zur exocyclischen Doppelbindung am nächsten kommt.

Sofern R^{a} an ein Stickstoffatom gebunden ist, ist R^{a} vorzugsweise von Halogen, C₁-C₆-Alkylthio, C₁-C₆-Alkylsulfinyl, Z¹P(O)(OR⁹)₂, worin Z¹ für eine Bindung steht, verschieden. In einer bevorzugten Ausführungsform der Erfindung ist R^{a} an ein C-Atom gebunden.

R^{a} weist bevorzugt eine der folgenden Bedeutungen auf:
- Halogen, Cyano, Nitro, C₁-C₆-Alkyl, C₃-C₆-Cycloalkyl, C₂-C₆-Alkenyl, C₂-C₆-Alkinyl, [Tri-(C₁-C₆)-alkylsilyl]-(C₂-C₆)-alkinyl, C₁-C₆-Alkylthio, C₁-C₆-Alkylsulfinyl, Aryl, Heterocyclyl, insbesondere 5- oder 6-gliedriges Heterocyclyl, wobei Aryl und Heterocyclyl unsubstituiert sind oder einen oder 2 Reste aufweisen können, die unter C₁-C₄-Alkyl, C₁-C₄-Halolkyl, C₁-C₄-Alkoxy, C₁-C₄-Haloalkoxy, CN und Halogen ausgewählt sind;
- Z¹P(O)(OR⁹)₂, wobei Z¹ eine Bindung oder -CH₂- bedeutet und R⁹ jeweils Wasserstoff oder C₁-C₆-Alkyl bedeuten;
- Z³COR¹¹, wobei Z³ eine Bindung bedeutet und R¹¹ die zuvorgenannten Bedeutungen hat und insbesondere Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, N-C₁-C₆-Alkoxy-N-C₁-C₆-alkylamino, [Di-(C₁-C₆)-Alkoxy]amino, C₁-C₆-Alkylsulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl, speziell C-gebundenes 5- oder 6-gliedriges Heteroaryl, bedeutet;
- Z⁴NR¹²R¹³, wobei Z⁴ eine Bindung oder -CH₂- bedeutet und R¹² und R¹³ die zuvorgenannten Bedeutungen haben und insbesondere unabhängig voneinander Wasserstoff, C₁-C₆-Alkyl, C₃-C₆-Cycloalkyl, C₃-C₆-Alkenyl, C₃-C₆-Alkinyl, C₁-C₆-Alkylcarbonyl, [Di-(C₁-C₆)-alkylamino]carbonyl, C₁-C₆-Alkoxycarbonyl, C₁-C₆-Alkylsulfonyl, C₁-C₆-Alkylaminosulfonyl, [Di-(C₁-C₆)-alkylamino]sulfonyl, C₃-C₆-Cycloalkylcarbonyl, Phenylcarbonyl, Phenylaminocarbonyl, Phenylsulfonyl, Phenylsulfonylaminocarbonyl,oder Heterocyclylcarbonyl, speziell C-gebundenes 5- oder 6-gliedriges Heteroarylcarbonyl, bedeuten;
- Z⁵CH=N-O-R¹⁴, wobei Z⁵ eine Bindung bedeutet und R¹⁴ Wasserstoff oder C₁-C₆-Alkyl bedeutet; oder
- Z⁶OR¹⁵, wobei Z⁶ eine Bindung oder -CH₂- bedeutet und R¹⁵ die zuvorgenannten Bedeutungen hat und insbesondere C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Di-(C₁-C₆-Alkoxycarbonyl)-C₁-C₆-alkyl, Phenylcarbonyl oder Phenyl bedeutet, R¹⁵ auch Wassestoff, C₁-C₆-Alkyl oder Phenyl-C₁-C₆-alkyl bedeuten kann; oder
- Z⁷SO₂R¹⁶, wobei Z⁷ eine Bindung oder CH₂ bedeutet und R¹⁶ C₁-C₆-Alkyl oder Phenyl;
wobei die genannten aliphatischen, cyclischen oder aromatischen Teile der Substituenten R^{a} partiell oder vollständig halogeniert sein können.

Sofern R^{a} an ein Stickstoffatom gebunden ist, ist R^{a} vorzugsweise von Halogen, C₁-C₆-Alkylthio, C₁-C₆-Alkylsulfinyl, Z¹P(O)(OR⁹)₂, worin Z¹ für eine Bindung steht, verschieden. In einer bevorzugten Ausführungsform der Erfindung ist R^{a} an ein C-Atom gebunden.

Insbesondere weist R^{a} eine der folgenden Bedeutungen auf:
- Halogen, Cyano, Nitro, C₁-C₆-Alkylthio, C₁-C₆-Alkylsulfinyl, Aryl, Heterocyclyl, wobei die zwei letzgenannten Reste unsubstituiert sind oder einen oder 2 Reste aufweisen können, die unter C₁-C₄-Alkyl, C₁-C₄-Halolkyl, C₁-C₄-Alkoxy, C₁-C₄-Haloalkoxy, CN und Halogen ausgewählt sind,
- C₂-C₆-Alkenyl, C₂-C₆-Alkinyl,
- Z¹P(O)(OR⁹)₂, wobei Z¹ eine Bindung oder -CH₂- bedeutet und R⁹ jeweils Wasserstoff oder C₁-C₆-Alkyl bedeuten; oder
- Z³COR¹¹, wobei Z³ eine Bindung bedeutet und R¹¹ Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, N-C₁-C₆-Alkoxy-N-C₁-C₆-alkylamino, C₁-C₆-Alkylsulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl, speziell C-gebundenes 5- oder 6-gliedriges Heteroaryl, bedeutet; oder
- Z⁴NR¹²R¹³, wobei Z⁴ eine Bindung oder -CH₂- bedeutet und R¹² und R¹³ unabhängig voneinander Wasserstoff, C₁-C₆-Alkyl, C₃-C₆-Cycloalkyl, C₃-C₆-Alkenyl, C₃-d₆-Alkinyl, C₁-C₆-Alkylcarbonyl, [Di-(C₁-C₆)-alkylamino]carbonyl, C₁-C₆-Alkoxycarbonyl, C₁-C₆-Alkyisulfonyl, Phenylcarbonyl, Phenylsulfonyl, oder Heterocyclylcarbonyl, speziell C-gebundenes 5- oder 6-gliedriges Heteroarylcarbonyl, bedeuten; oder
- Z⁵CH=N-O-R¹⁴, wobei Z⁵ eine Bindung bedeutet und R¹⁴ Wasserstoff oder C₁-C₆-Alkyl bedeutet; oder
- Z⁶OR¹⁵, wobei Z⁶ eine Bindung oder -CH₂- bedeutet und R¹⁵ Wasserstoff, C₁-C₆-Alkyl, C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Phenyl oder Phenyl-(C₁-C₆)-Alkyl-bedeutet; oder
- Z⁷SO₂R¹⁶, wobei Z⁷ eine Bindung oder CH₂ bedeutet und R¹⁶ C₁-C₆-Alkyl oder Phenyl;
und wobei die genannten aliphatischen, cyclischen oder aromatischen Teile der Substituenten R^{a} partiell oder vollständig halogeniert sein können.

Ganz speziell bevorzugt steht R^{a} für einen Rest, der ausgewählt ist unter Halogen, Cyano, Nitro, C₂-C₄-Alkenyl und C₂-C₄-Alkinyl, NH-C(O)-C₁-C₆-Alkyl, NH-S(O)₂-C₁-C₆-Alkyl und 5 gliedrigem Heteroaryl, z.B. Oxazolyl, Thiazolyl, Isoxazolyl, Isothiazolyl, wobei die vorgeanntne Heteroarylreste einen oder 2 Reste aufweisen können, die unter C₁-C₂-Alkyl, C₁-C₂-Haloalkyl und Halogen ausgewählt sind, und der insbesondere in einer der ortho-Postionen von A¹ gebunden ist.

R^{b}, R^{c}, R^{d}, R^{e} und R^{f} stehen vorzugsweise für Wasserstoff oder haben unabhängig voneinander eine der für R^{a} als bevorzugt oder besonders bevorzugt genannten Bedeutungen.

Insbesondere sind die Reste R^{b}, R^{c}, R^{d}, R^{e} und R^{f} unabhängig voneinander ausgewählt unter Wasserstoff, Halogen, C₁-C₄-Alkyl, C₂-C₄-Alkenyl, C₂-C₄-Alkinyl, C₁-C₄-Alkoxy, C₁-C₄-Halogenalkyl und C₁-C₄-Halogenalkoxy, wobei zwei an benachbarte C-Atome von A¹ oder A² gebundene Gruppen R^{b}, R^{c}, R^{d}, R^{e} oder R^{f} auch für eine Gruppe O-CH₂-O stehen können.

Insbesondere ist R^{b} ein von Wasserstoff verschiedener Rest. Vorzugsweise ist R^{b} ein in der ortho-Position von A¹, gebundener Rest, d.h. wenn R^{a} ebenfalls in der ortho-Position gebunden ist, befindet sich R^{b} in der zweiten ortho-Position.

Sofern einer oder beide Reste R^{b}, R^{c} für einen von Wasserstoff verschiedenen Substituenten stehen, sind sie insbesondere ausgewählt unter den als bevorzugt angegebenen Substituenten, und speziell unter Halogen, C₁-C₄-Alkyl, C₁-C₄-Alkoxy, C₁-C₄-Halogenalkyl, C₂-C₄-Alkenyl, C₂-C₄-Alkinyl oder C₁-C₄-Halogenalkoxy oder R^{b} und R^{c} stehen gemeinsam für eine Gruppe O-CH₂-O.

Insbesondere ist A² unsubstituiert oder einer oder zwei der Substituenten R^{d}, Re bzw. R^{f} stehen für einen von Wasserstoff verschiedenen Substituenten. Sofern 1 oder 2 der Substituenten R^{d}, R^{e} und R^{f} von Wasserstoff verschieden sind , sind sie insbesondere ausgewählt unter Halogen, C₁-C₄-Alkyl, C₁-C₄-Alkoxy, C₁-C₄-Halogenalkyl und C₁-C₄-Halogenalkoxy.

Die Bedeutung, die ein Substituent im Rahmen der Erfindung annehmen kann, ist vollkommen unabhängig von der Bedeutung, die ein anderer Substituent im Rahmen der Erfindung annehmen kann. Besonders bevorzugt sind jedoch Verbindungen der Formel I, worin mehrere oder insbesondere alle Variablen eine als bevorzugt oder besonders bevorzugt angegebene Bedeutung aufweisen.

Weiterhin hat es sich als vorteilhaft herausgestellt, wenn die exocyclische Doppelbindung am Piperazin-Ring Z-Konformation aufweist, d.h. die Gruppe A¹(R^{a}R^{b}R^{c}) und der Ringstickstoff, welcher die Gruppe R² trägt, weisen bezüglich dieser Doppelbindung eine cis-Anordnung auf. Dementsprechend betrifft eine bevorzugte Ausführungsform der Erfindung Piperazinverbindungen der Formel (Z)-I, worin die Variablen A¹, A², R¹ - R⁶ und R^{a} - R^{f} die zuvor genannten Bedeutungen aufweisen, sowie Mischungen des Z-Isomers (Z)-I mit dem E-Isomer der Formel (E)-I, worin das E/Z-Verhältnis < 1:1, insbesondere < 1:2, besonders bevorzugt < 1:4 und speziell < 1:10 ist. In Formel (E)-I haben ebenfalls die Variablen A¹, A², R¹ - R⁶ und R^{a} - R^{f} die zuvor genannte Bedeutungen.

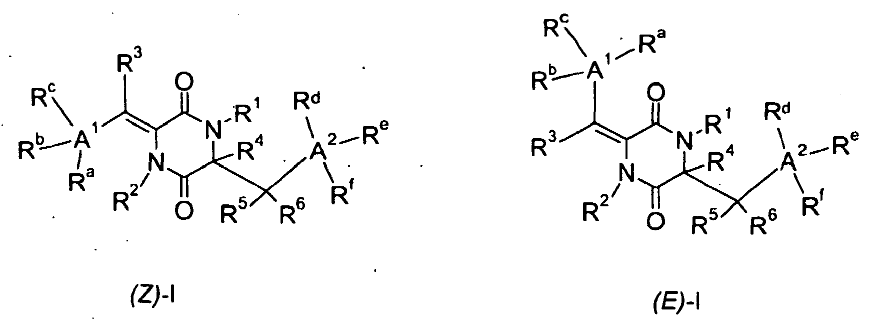

Weiterhin hat es sich als vorteilhaft herausgestellt, wenn das Kohlenstoffatom, welches R⁴ trägt, S-Konfiguration aufweist. Dementsprechend betrifft eine bevorzugte Ausführungsform der Erfindung Piperazinverbindungen der Formel I-S, worin die Variablen A¹, A², R¹ - R⁶ und R^{a} - R^{f} die zuvor genannten Bedeutungen aufweisen, sowie Mischungen des S-Enantiomers (S)-I mit dem R-Enantiomer der Formel (R)-I, worin das R/S-Verhältnis < 1:1, insbesondere < 1:2, besonders bevorzugt < 1:4 und speziell < 1:10 ist. In Formel (R)-I haben ebenfalls die Variablen A¹, A², R¹ - R⁶ und R^{a} - R^{f} die zuvor genannte Bedeutungen.

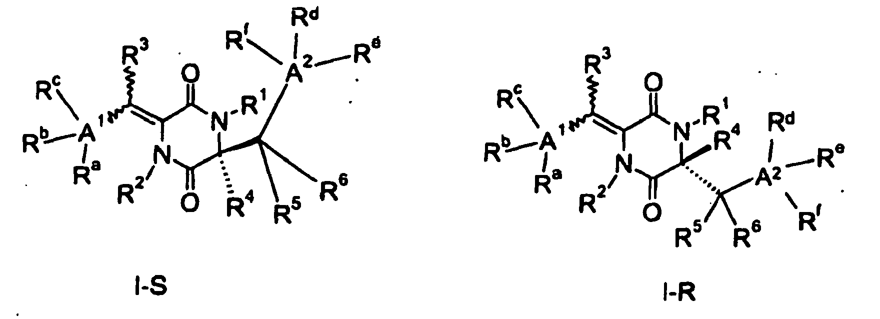

Bevorzugt sind insbesondere Verbindungen der Formel **I,** worin sowohl die exocyclische Doppelbindung Z-Konformation hat als auch das C-Atom, welches R⁴ trägt, S-Konfiguraion aufweist. Diese Verbindungen werden im Folgenden auch als Verbindungen (Z,S)-I bezeichnet.

Bevorzugt sind insbesondere die Verbindungen der Formel I.1, bei denen R¹, R³, R⁴, R⁵, und R⁶ Wasserstoff sind und R² CH₃ ist, besonders bevorzugt die Verbindungen (Z,S)-I.1 die an den gekennzeichneten Positionen (1) und (2) Z- bzw. S-Konfiguration haben.

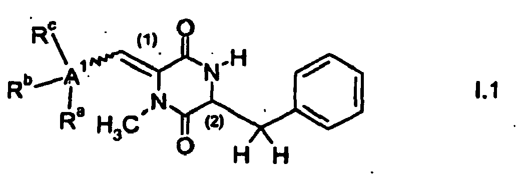

Die Piperazinverbindungen der Formel **I** können nach Standardmethoden der Synthese organischer Verbindungen auf verschiedene Art und Weise herstellt werden, beispielsweise nach den im Folgenden näher erläuterten Verfahren:

### Verfahren A

Die Verbindungen der Formel I können beispielsweise in Analogie zu literaturbekannten Verfahren durch Dehydratisierung der entsprechenden Alkoholvorstufe der Formel II hergestellt werden.

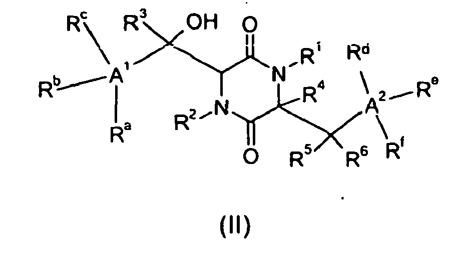

Dementsprechend ist ein weiterer Gegenstand der Erfindung ein Verfahren zur Herstellung erfindungsgemäßer Piperazinverbindungen der allgemeinen Formel I, bei dem man eine Verbindung der Formel II 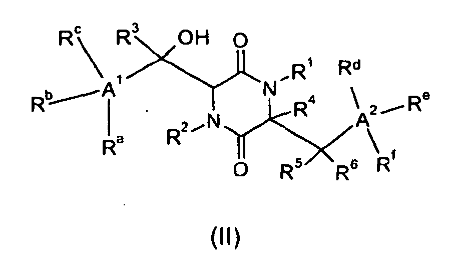 worin die Variablen A¹, A², R¹, R², R³, R⁴, R⁵, R⁶, R^{a}, R^{b}, R^{c}, R^{d}, Re und R^{f} wie vorstehend für die Verbindung der Formel I definiert sind, entweder

### (A.1) zu einer Verbindung der Formel III

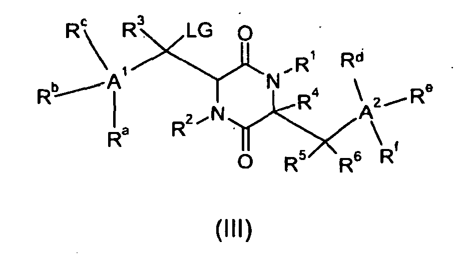

worin LG für eine Abgangsgruppe steht,
umsetzt und aus der Verbindung III unter Erhalt der entsprechenden Verbindung I formal die Verbindung H-LG eliminiert;
oder
- (A.2): entsprechenden Verbindung I dehydratisiert, wenn LG für OH steht, wobei die Dehydratisierung gegebenenfalls in Gegenwart eines Dehydratisierungsmittels erfolgt.

Bezüglich bevorzugter Bedeutungen der Variablen A¹, A², R¹, R², R³, R⁴, R⁵, R⁶, R^{a}, R^{b}, R^{c}, R^{d}, Re und R^{f} wird auf die vorstehend gemachten Ausführungen verwiesen. Bevorzugte Ausgestaltungen des erfindungsgemäßen Verfahrens sind nachfolgend beschrieben.

### Variante A.1

In einer ersten Variante (Variante A.1) kann die Alkoholfunktion der Verbindung II zunächst in eine geeignete Abgangsgruppe überführt werden und diese anschließend formal als Verbindung H-LG eliminiert werden. Vorzugsweise findet die Eliminierungsreaktion in Gegenwart einer geeigneten Base statt. Diese Reaktion ist im nachfolgenden Schema skizziert.

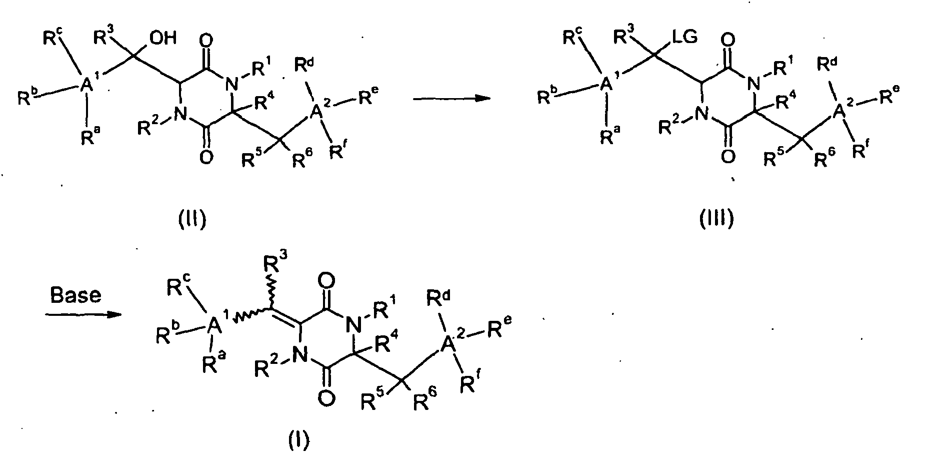

In Formel II haben die Variablen A¹, A², R¹- R⁶, R^{a}, R^{b}, R^{c}, R^{d}, Re und R^{f} die für Formel I angegebene Bedeutung. LG steht für eine Abgangsgruppe.

Bei der Abgangsgruppe LG handelt es sich um eine übliche, aus einer Hydroxygruppe leicht herzustellende Abgangsgruppe. Beispiele hierfür sind 4-Toluolsulfonyloxy (LG = -O-SO₂C₆H₄CH₃), Trifluormethansulfonyloxy (LG = -O-SO₂CF₃) und Methansulfonyloxy (LG = -O-SO₂CH₃), wobei letzteres besonders geeignet ist. Die Einführung einer solchen Abgangsgruppe erfolgt gemäß herkömmlicher Verfahren, z.B. durch Umsetzung des Alkohols II mit einer Base und anschließend mit dem entsprechenden Sulfonsäurechlorid, z.B. mit Methansulfonsäurechlorid oder Trifluormethansulfonsäurechlorid. Geeignete Basen sind die nachfolgend bei der Eliminierung aufgeführten Basen. Bevorzugt verwendet man jedoch Basen, die in organischen Lösungsmitteln löslich sind, z.B. die unten genannten Amine oder Stickstoff-Heterocyclen. Insbesondere verwendet man Pyridin oder substituierte Pyridine, wie Dimethylaminopyridin, Lutidin oder Collidin, oder Gemische davon. Günstigerweise werden die organischen Basen so gewählt, dass sie auch als Lösungsmittel fungieren.

Für die Eliminierung kommen als Basen allgemein anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin 2-Hydroxypyridin und N-Methylpiperidin, Pyridin, substituierte Pyridine wie Collidin, Lutidin und 4-Dimethylaminopyridin sowie bicyclische Amine in Betracht. Selbstverständlich kann auch eine Mischung verschiedener Basen verwendet werden.

Besonders geeignet sind jedoch Basen, die zwar eine ausreichende Basizität, jedoch im Wesentlichen keine Nucleophilie aufweisen, z.B. sterisch gehinderte Alkalialkoholate, beispielsweise Alkali-tert-butanolate, wie Kalium-tert-butanolat, und insbesondere cyclische Amidine, wie DBU (1,8-Diazabicyclo[5.4.0]undec-7-en) und DBN (1,5-Diazabicyclo[3.4.0]non-5-en). Bevorzugt verwendet man die zuletzt genannten Amidine.

Die Eliminierung erfolgt in der Regel in einem Lösungsmittel, bevorzugt in einem inerten organischen Lösungsmittel. Geeignete inerte organische Lösungsmittel umfassen aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, diethylketon und tert-Butylmethylketon, Alkohole wie Methanol, Ethanol, n-Propanol, Isopropanol, n-Butanol, tert-Butanol, Wasser sowie Dimethylsulfoxid, Dimethylformamid und Dimethylacetamid sowie Morpholin und N-Methylmorpholin. Es können auch Gemische der genannten Lösungsmittel verwendet werden. Bevorzugt verwendet man Tetrahydrofuran.

Die Dehydratisierung von Alkoholen II durch Überführung der Alkoholfunktion in eine gute Abgangsgruppe und anschließende Eliminierung kann in Analogie zu bekannten Verfahren des Standes der Technik erfolgen, z.B. analog zu den in Helv. Chim. Acta 1947, 30, 1454; Liebigs Ann. Chem 1992, (7), 687-692, Carbanions. 24. Rearrangements of (E)- and (Z)-2,2-diphenyl-3-pentenylalkali metal compounds; Sch. Chem., Georgia Inst. Technol., Atlanta, GA, USA; J. Org. Chem. 1989, 54(7), 1671-1679; Chemical & Pharmaceutical Bulletin 1986, 34(7), 2786-2798 beschriebenen Verfahren, worauf hiermit in vollem Umfang Bezug genommen wird.

### Variante A.2

In einer zweiten Variante (Variante A.2) erfolgt die Herstellung der Verbindung I durch Dehydratisierung der Verbindung II in Gegenwart eines geeigneten Dehydratisierungsmittels, wie folgendes Schema veranschaulicht.

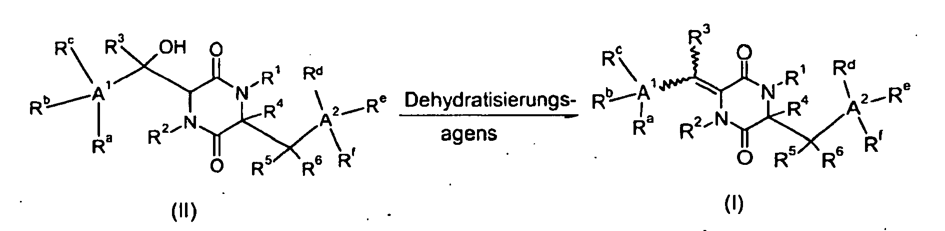

Geeignete Dehydratisierungsagenzien sind z.B. das System Triphenylphosphin/DEAD (DEAD = Diethylazodicarboxylat) und das Burgess-Reagens. Die Kombination aus Triphenylphosphin und DEAD wird zwar in der Regel zur gezielten Inversion an einem Hydroxy-substituierten Chiralitätszentrum eingesetzt (sogenannte Mitsunobu-Reaktion); in Abwesenheit von Nucleophilen wirkt es jedoch als mildes Dehydratisierungsmittel. Das System wird in Bezug auf die Verbindung II vorzugsweise im Überschuss eingesetzt, wobei die beiden Komponenten Triphenylphosphin und DEAD geeigneterweise in einem etwa äquimolaren Verhältnis zueinander vorliegen.

Beim Burgess-Reagens handelt es sich um das Zwitterion Methyl-N-(triethylammoniumsulfonylcarbamat ((C₂H₅)₃N⁺-SO₂-N-COOCH₃), einem milden Dehydratisierungsmittel. Dieses kann in Bezug auf den Alkohol II äquimolar oder in molarem Überschuss eingesetzt werden. Die Umsetzung mit dem Burgess-Reagens erfolgt in der Regel in einem inerten organischen Lösungsmittel. Geeignete inerte organische Lösungsmittel umfassen aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, und Ketone wie Aceton, Methylethylketon, Diethylketon und tert-Butylmethylketon Bevorzugt verwendet man aromatische Kohlenwasserstoffe oder Gemische davon und speziell Toluol.

Die Dehydratisierung von Alkoholen II unter Verwendung von Dehydratisierungsmitteln kann in Analogie zu bekannten Verfahren des Standes der Technik erfolgen, beispielsweise analog zu den in Synthesis 2003, 201 und J. Indian Sci. 2001, 81, 461 beschriebenen Verfahren, worauf hiermit in vollem Umfang Bezug genommen wird.

### Verfahren B

Die in den beiden Varianten (A.1 und A.2) des Verfahrens A eingesetzten Alkohole der Formel II können beispielsweise in Analogie zu literaturbekannten Verfahren durch Cyclisierung entsprechender Dipeptid-Vorstufen der Formel IV hergestellt werden, beispielsweise in Analogie zu der von T. Kawasaki et al., Org. Lett. 2(19) (2000), 3027-3029, Igor L. Rodionov et al., Tetrahedron 58(42) (2002), 8515-8523 oder A. L. Johnson et al., Tetrahedron 60 (2004), 961-965 beschriebenen Methode. Die Cyclisierung von Dipeptiden der Formel IV zu den Alkoholen 11 wird im Folgenden auch als Verfahren B bezeichnet und ist im nachfolgenden Schema veranschaulicht.

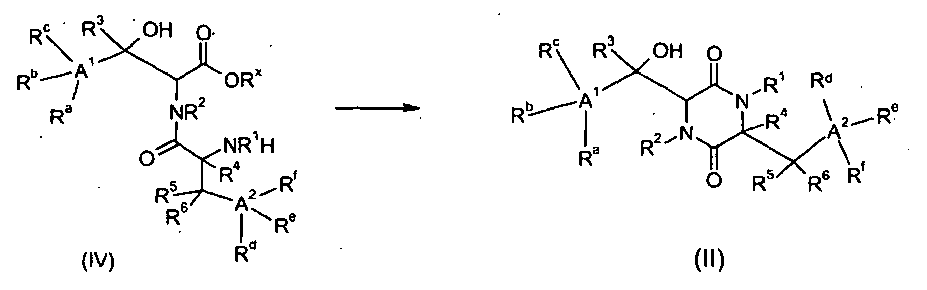

In Formel IV haben die Variablen A¹, A², R¹ - R⁶, R^{a}, R^{b}, R^{c}, R^{d}, Re und R^{f} die für Formel angegebene Bedeutung. Die Gruppe OR^{x} steht für eine geeignete, über Sauerstoff gebundene Abgangsgruppe. R^{x} ist hierbei z.B. C₁-C₆-Alkyl, insbesondere Methyl oder Ethyl, oder Phenyl-C₁-C₆-alkyl, z.B. Benzyl.

Die Cyclisierung kann beispielsweise durch Umsetzung eines Dipeptids der Formel IV, entweder in Gegenwart von Säure oder Base (saure oder basische Cyclisierung) oder durch Erhitzen der Reaktionsmischung (thermische Cyclisierung) bewirkt werden.

Die Basen oder Säuren werden entweder in äquimolaren Mengen zum Dipeptid IV oder im Überschuss eingesetzt. In einer besonderen Ausführungsform des erfindungsgemäßen Verfahrens werden die Basen oder Säuren in einem Überschuss bezogen auf das Dipeptid verwandt.

Die Umsetzung des Dipeptids IV in Gegenwart einer Base erfolgt üblicherweise bei Temperaturen im Bereich von 0°C bis zum Siedepunkt der Reaktionsmischung, bevorzugt von 10°C bis 50°C, insbesondere bevorzugt von 15°C bis 35°C. In der Regel wird die Umsetzung in einem Lösungsmittel vorgenommen, bevorzugt in einem inerten organischen Lösungsmittel.

Geeignete inerte organische Lösungsmittel umfassen aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol, tert.-Butanol, Wasser sowie Dimethylsulfoxid, Dimethylformamid und Dimethylacetamid sowie Morpholin und N-Methylmorpholin. Es können auch Gemische der genannten Lösungsmittel verwendet werden.

In einer bevorzugten Ausgestaltungsform der Erfindung wird die Reaktion in einem Tetrahydrofuran-Wasser Gemisch durchgeführt, beispielsweise mit einem Mischungsverhältnis von 1 : 10 bis 10 : 1 (Volumenteile).

Als Basen kommen allgemein anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin, 2-Hydroxypyridin und N-Methylpiperidin, Pyridin, substituierte Pyridine wie Collidin, Lutidin und 4-Dimethylaminopyridin sowie bicyclische Amine in Betracht. Selbstverständlich kann auch eine Mischung verschiedener Basen verwendet werden.

In einer Ausführungsform des erfindungsgemäßen Verfahrens wird die Reaktion von IV in Gegenwart von Basen durchgeführt, bevorzugt in Gegenwart der Basen Kalium-tert.-Butanolat, 2-Hydroxypyridin oder einer wässrigen Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 w/v %ig sein kann, durchgeführt.

Die Umsetzung von IV in Gegenwart einer Säure erfolgt üblicherweise bei Temperaturen im Bereich von 10°C bis zum Siedepunkt der Reaktionsmischung, bevorzugt von 50°C bis zum Siedepunkt, insbesondere bevorzugt beim Siedepunkt unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die auch für die basische Cyclisierung verwendet werden können, insbesondere Alkohole. In einer bevorzugten Ausführungsform wird die Reaktion in n-Butanol durchgeführt.

Als Säuren für die Cyclisierung von IV kommen grundsätzlich sowohl Brönstedt- als auch Lewis-Säuren in Betracht. Insbesondere können anorganische Säuren, z.B. Halogenwasserstofisäuren wie Fluorwasserstoffsäure, Salzsäure, Bromwasserstoffsäure, anorganische Oxosäuren wie Schwefelsäure und Perchlorsäure, weiterhin anorganische Lewis-Säuren wie Bortrifluorid, Aluminiumtrichlorid, Eisen-III-chlorid, Zinn=IV-chlorid, Titan-IV-chlorid und Zink-II-chlorid, sowie organische Säuren, beispielsweise Carbonsäuren und Hydroxycarbonsäuren wie Ameisensäure, Essigsäure, Propionsäure, Oxalsäure, Zitronensäure und Trifluoressigsäure, sowie organische Sulfonsäuren wie Toluolsulfonsäure, Benzolsulfonsäure, Camphersulfonsäure und dergleichen, Verwendung finden. Selbstverständlich kann auch eine Mischung verschiedener Säuren eingesetzt werden.

In einer Ausführungsform des erfindungsgemäßen Verfahrens wird die Reaktion in Gegenwart von organischen Säuren durchgeführt, beispielsweise in Gegenwart von Carbonsäuren wie Ameisensäure, Essigsäure oder Trifluoressigsäure oder einer Mischung dieser Säuren. Bevorzugt wird nur eine dieser Säuren verwendet. In einer bevorzugten Ausführungsform wird die Reaktion in Essigsäure durchgeführt.

Eine besonders bevorzugte Ausführungsform der sauren Cyclisierung wird in Gegenwart von n-Butanol, N-Methyl-Morpholin und Essigsäure unter Rückflussbedingungen durchgeführt.

In einer weiteren Ausführungsform der Erfindung wird die Umsetzung ausschließlich durch Erhitzen der Reaktionsmischung durchgeführt (thermische Cyclisierung). Die Umsetzung erfolgt hierbei üblicherweise bei Temperaturen im Bereich von 10°C bis zum Siedepunkt der Reaktionsmischung, bevorzugt von 50°C bis zum Siedepunkt der Reaktionsmischung, insbesondere bevorzugt beim Siedepunkt der Reaktionsmischung unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die bei der basischen Cycilsierung verwendet werden. Bevorzugt werden polar aprotische Lösungsmittel, z.B. Dimethylsulfoxid oder Dimethylformamid oder deren Mischungen. In einer bevorzugten Ausführungsform wird die Reaktion in Dimethylsulfoxid durchgeführt.

Die in Verfahren B erhaltenen Reaktionsgemische, aber auch die in den Verfahren A1 und A2 erhaltenen Reaktionsgemische sowie die Reaktionsgemische der im Folgenden beschriebenen Verfahren, können in üblicher Weise aufgearbeitet werden. Das kann z.B. durch Mischen mit Wasser, Trennung der Phasen und gegebenenfalls chromatographische Reinigung der Rohprodukte erfolgen. Die Zwischen- und Endprodukte fallen z.T. in Form zäher Öle an, die in der Regel unter vermindertem Druck und bei mäßig erhöhter Temperatur von flüchtigen Anteilen befreit oder gereinigt werden können. Sofern die Zwischen- und Endprodukte als Feststoffe erhalten werden, kann die Reinigung auch durch Umkristallisieren oder Digerieren erfolgen.

Die für die Herstellung der Verbindungen der Formel II benötigen Vorstufen und Zwischenprodukte sind teilweise kommerziell erhältlich, aus der Literatur bekannt oder können nach literaturbekannten Verfahren hergestellt werden.

Die Dipeptid-Verbindungen der Formel IV können beispielsweise aus N-geschützten Dipeptiden der allgemeinen Formel V in Analogie zu literaturbekannten Verfahren hergestellt werden, beispielsweise nach Glenn L. Stahl et al., J. Org. Chem. 43(11), (1978), 2285-6 oder A. K. Ghosh et al., Org. Lett. 3(4), (2001), 635-638.

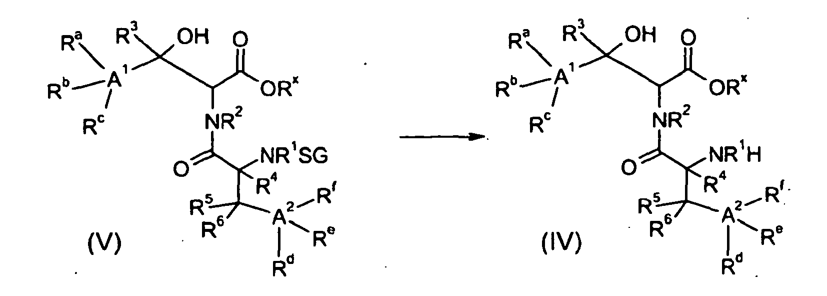

In den Formeln IV und V haben die Variablen A¹, A², R¹ - R⁶, R^{a}, R^{b}, R^{c}, R^{d}, Re und R^{f} die für Formel I angegebene Bedeutung, SG bedeutet eine Stickstoff-Schutzgruppe wie Boc (= tert-Butoxycarbonyl) und ORx steht für eine über ein Sauerstoffatom gebundene Abgangsgruppe. Selbstverständlich gelten die bevorzugten Bedeutungen für die Verbindungen der Formel II sinngemäß jeweils für die Verbindungen der Formel IV oder V. Bezüglich der Abgangsgruppe ORx gilt das zuvor für die Dipeptide der Formel IV gesagte.

So kann beispielsweise ein Dipeptid der Formel V, worin SG für Boc steht und OR^{x} eine geeignete Abgangsgruppe, bei der R^{x} z.B. C₁-C₆-Alkyl, insbesondere Methyl, Ethyl oder Benzyl ist, in Gegenwart einer Säure zu einer Verbindung der Formel IV umgesetzt werden.

Die Umsetzung erfolgt üblicherweise bei Temperaturen im Bereich von -30°Cbis zum Siedepunkt der Reaktionsmischung, bevorzugt von 0°C bis 50°C, insbesondere bevorzugt von 20°C bis 35°C.

Die Reaktion kann in einem Lösungsmittel stattfinden, inbesondere in einem inerten organischen Lösungsmittel. Als Lösungsmittel kommen prinzipiell die bei der basischen Cyclisierung zitierten Verbindungen in Betracht, insbesondere Tetrahydrofuran oder Dichlormethan oder deren Mischungen. In einer bevorzugten Ausführungsform wird die Reaktion in Dichlormethan durchgeführt. '

Als Säuren finden die bei der Cyclisierung des Dipeptids IV zum Piperazin II zitierten Säuren Verwendung.

In einer Ausführungsform des erfindungsgemäßen Verfahrens wird die Reaktion in Gegenwart von organischen Säuren durchgeführt, beispielsweise in Gegenwart starker organischer Säuren, wie Ameisensäure, Essigsäure oder Trifluoressigsäure oder deren Mischungen. In einer bevorzugten Ausführungsform wird die Reaktion in Gegenwart von Trifluoressigsäure durchgeführt.

Die Aufarbeitung kann in Analogie zu der bei der Cyclisierung des Dipeptids IV zum Piperazin II beschriebenen Vorgehensweise erfolgen.

Die geschützten Dipeptide der Formel V können in Analogie zu literaturbekannten Verfahren hergestellt werden, beispielsweise nach Wilford L. Mendelson et al., Int. J.Peptide & Protein Research 35(3), (1990), 249-57. Ein typischer Zugang ist die Amidierung einer Boc-geschützen Aminosäure VI mit einem Aminosäureester der Formel VII wie in dem folgenden Schema dargestellt:

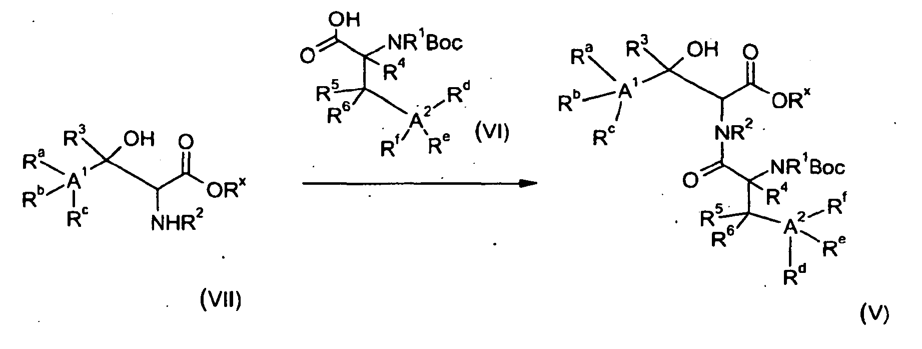

In diesem Schema haben die Variablen die zuvor genannten Bedeutungen. Anstelle von Boc können auch andere Amino-Schutzgruppen eingesetzt werden.

In der Regel erfolgt die Umsetzung von VII mit VI bei Temperaturen in einem Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgeführt werden. Geeignet sind die im Zusammenhang mit der basischen Cyclisierung von IV zu II genannten Lösungsmittel.

Im Allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenz EDAC oder DCC bevorzugt.

Vorzugsweise erfolgt die Umsetzung von VII mit VI in Gegenwart einer Base. Geeignete Basen sind die bei der Cyclisierung des Dipeptids IV zum Piperazin II zitierten Verbindungen. In einer Ausführungsform werden als Base Triethylamin oder N-Ethyldiisopropylamin oder deren Mischungen, besonders bevorzugt N-Ethyldiisopropylamin verwendet.

Die Aufarbeitung kann in Analogie zu der bei der Cyclisierung des Dipeptids IV zum Piperazin II beschriebenen Vorgehensweise erfolgen.

Die Verbindungen der Formel VII können ihrerseits durch Entschützen entsprechender geschützter Aminosäureverbindungen VIII in Analogie zu literaturbekannten Verfahren hergestellt werden, beispielsweise nach Glenn L. Stahl et al., J. Org. Chem. 43(11), (1978), 2285-6. oder A. K. Ghosh et al., Org. Lett. 3(4), (2001), 635-638. Die Herstellung von VII aus einer Boc-geschützten Aminosäureverbindung VIII ist im folgenden Schema dargestellt.

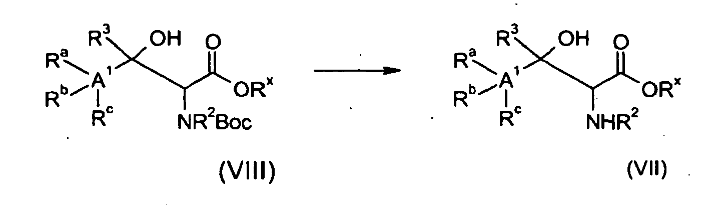

In diesem Schema haben die Variablen die zuvor genannten Bedeutungen. Anstelle von Boc können auch andere Amino-Schutzgruppen eingesetzt werden.

Die Umsetzung einer Verbindung der Formel VIII zur Verbindung VII erfolgt typischerweise in Gegenwart einer Säure bei Temperaturen in einem Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel.

Als Lösungsmittel kommen prinzipiell die unter der basischen Cyclisierung zitierten Verbindungen in Betracht, insbesondere Tetrahydrofuran oder Dichlormethan oder deren Mischungen. In einer bevorzugten Ausführungsform wird die Reaktion in Dichlormethan durchgeführt.

Als Säuren und saure Katalysatoren finden die bei der Cyclisierung des Dipeptids IV zum Piperazin II zitierten Stoffe Verwendung.

In einer Ausführungsform des erfindungsgemäßen Verfahrens wird die Reaktion in Gegenwart von organischen Säuren durchgeführt, beispielsweise in Gegenwart von starken organischen Säuren, wie Ameisensäure, Essigsäure oder Trifluoressigsäure oder deren Mischungen. In einer bevorzugten Ausführungsform wird die Reaktion in Gegenwart von Trifluoressigsäure durchgeführt.

Die Aufarbeitung kann in Analogie zu der bei der Cyclisierung des Dipeptids IV zum Piperazin II beschriebenen Vorgehensweise erfolgen.

Die Verbindungen der Formel VIII können entsprechend der im folgenden Schema dargestellten Umsetzung hergestellt werden. Die Umsetzung von Verbindung XI mit der geschützten Aminosäureverbindung X kann in Analogie zu literaturbekannten Verfahren erfolgen, beispielsweise nach I. Ojima et al., J. Am. Chem. Soc., 109(21), (1987), 6537-6538 oder J. M. McIntosh et al., Tetrahedron 48(30), (1992), 6219-6224.

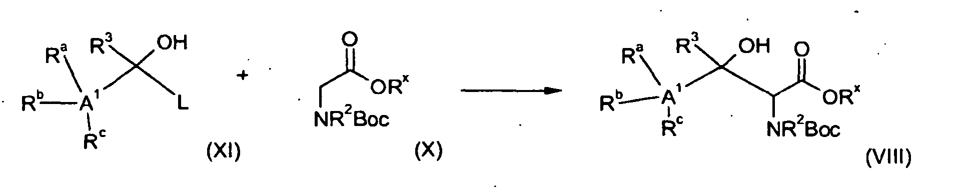

In diesem Schema haben die Variablen die zuvor genannten Bedeutungen. L steht für eine Abgangsgruppe. Anstelle von Boc können auch andere Amino-Schutzgruppen eingesetzt werden.

Die Umsetzung von XI mit X erfolgt in der Regel in Gegenwart von Base. Geeignete Basen sind die bei der Cyclisierung des Dipeptids IV zum Piperazin II zitierten Verbindungen. In einer weiteren bevorzugten Ausführungsform wird Lithiumdiisopropylamid, besonders bevorzugt in im Wesentlichen äquimolarer Menge, insbesondere äquimolar als Base verwendet.

Üblicherweise wird die Reaktion bei Temperaturen im Bereich von - 78°C und dem Siedepunkt der Reaktionsmischung, bevorzugt von - 78°C und dem Siedepunkt, insbesondere bevorzugt von - 78°C bis 30°C durchgeführt.

Die Reaktion kann in einem Lösungsmittel durchgeführt werden, bevorzugt in einem inerten organischen Lösungsmittel. Als Lösungsmittel kommen prinzipiell die unter der basischen Cyclisierung genannten Lösungsmittel in Betracht, insbesondere Dichlormethan oder Tetrahydrofuran oder deren Mischungen. In einer bevorzugten Ausführungsform wird die Reaktion in Tetrahydrofuran durchgeführt.

Die Aufarbeitung kann in Analogie zu der bei der Cyclisierung des Dipeptids IV zum Piperazin II beschriebenen Vorgehensweise erfolgen.

Verbindungen der Formel XI sind zum Teil kommerziell erhältlich oder durch literaturbeschriebene Transformationen der entsprechenden, kommerziell erhältlichen Vorprodukte herstellbar.

### Verfahren C

Verbindung der Formel II.1, worin R³ für H steht, können auch dadurch hergestellt werden, dass man in einer Aldolreaktion den Aldehyd XII mit dem Piperazin XIII koppelt, wie im folgenden Schema skizziert ist:

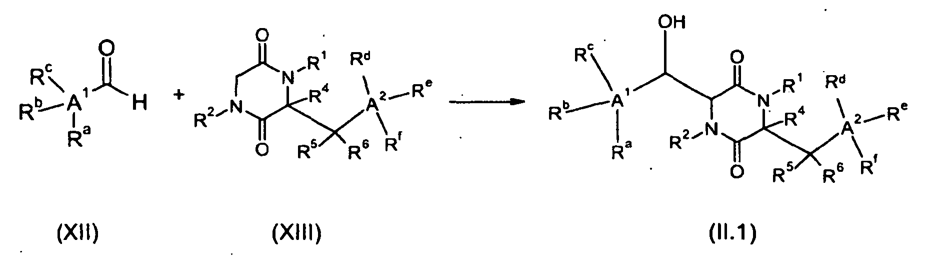

In den Formeln II.1, XII und XII haben die Variablen A¹, A², R¹ - R⁶, R^{a}, R^{b}, R^{c}, R^{d}, R^{e} und R^{f} die für Formel I angegebene Bedeutung.

Die Umsetzung erfolgt in der Regel in Gegenwart geeigneter Basen. Geeignete Basen sind solche, die üblicherweise bei Aldolreaktionen eingesetzt werden. Beispiele hierfür sind bei der Cyclisierung von IV zu II genannten Verbindungen. Bevorzugt verwendet man Alkaliamide wie Lithiumdiisopropylamid. Geeignete Reaktionsbedingungen sind aus dem Stand der Technik bekannt und beispielsweise in J. Org. Chem. 2000, 65 (24), 8402-8405 beschrieben, worauf hiermit in vollem Umfang Bezug genommen wird.

Die Aldolreakion kann auch direkt zum entsprechenden Aldolkondensationsprodukt, d.h. zu Verbindungen der Formel I.1, worin R³ für H steht, führen. Dies ist insbesondere dann der Fall, wenn die Umsetzung bei höheren Temperaturen und unter längeren Reaktionszeiten verläuft.

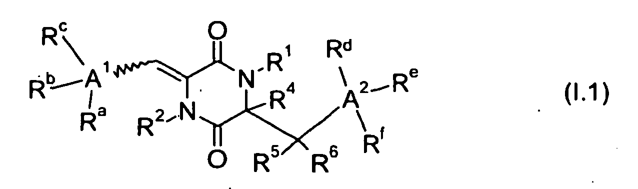

Der Aldehyd XII ist entweder kommerziell erhältlich oder kann gemäß bekannten Verfahren zur Herstellung von Aldehyden synthetisiert werden. Solche Aldolkondensationen können analog zu den in J. Org. Chem. 2000, 65 (24), 8402-8405 beschriebenen Verfahren durchgeführt werden, worauf hiermit in vollem Umfang Bezug genommen wird.

Grundsätzlich lässt sich die Aldolreaktion oder -kondensation auch für die Herstellung von Verbindungen I einsetzen, in denen R³ nicht für Wasserstoff stehen muss, sondern auch für C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl stehen kann. In diesem Fall wird anstelle des Aldehyds XII das Keton IX eingesetzt 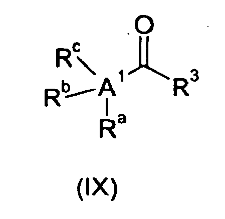 worin R³ für C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl steht.

Allerdings ist hier die Bildung komplexer Reaktionsgemische möglich, insbesondere wenn R³ für eine Gruppe steht, in der das Kohlenstoffatom, das in α-Position zur Verknüpfungsstelle gebunden ist, ein Wasserstoffatom trägt. Zudem sind meist auch drastischere Reaktionsbedingungen nötig, so dass die Aldolisierung vorzugsweise nur für die Herstellung von Verbindungen I.1 angewandt wird.

Die Verbindungen der Formel XIII können durch intramolekulare Cyclisierung von Verbindungen der allgemeinen Formel XIV in Analogie zu weiteren literaturbekannten Verfahren hergestellt werden, beispielsweise nach T. Kawasaki et al., Org. Lett. 2(19) (2000), 3027-3029, Igor L. Rodionov et al., Tetrahedron 58(42) (2002), 8515-8523 or A. L. Johnson et al., Tetrahedron 60 (2004), 961-965.

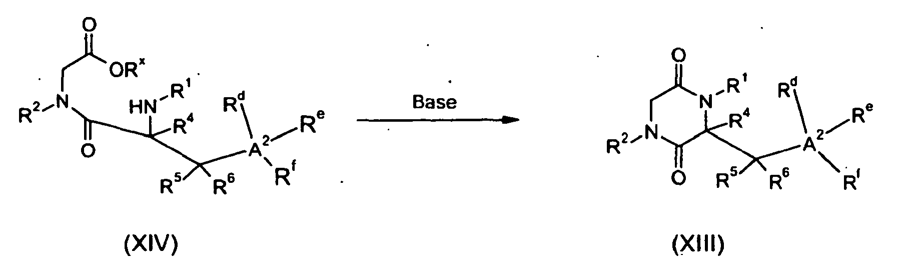

In Formel XIV haben die Variablen R^{x}, A², R¹, R², R⁴, R⁵, R⁶, R^{d}, Re und R^{f} die für Formel IV angegebene Bedeutung. Die Gruppe OR^{x} steht für eine geeignete, über Sauerstoff gebundene Abgangsgruppe. R^{x} ist hierbei z.B. C₁-C₆-Alkyl, insbesondere Methyl, Ethyl oder Phenyl-C₁-C₆-alkyl, z.B. Benzyl.

Die Cyclisierung der Verbindungen der Formel XIV kann in Gegenwart einer Base erfolgen. Die Reaktion erfolgt dann in der Regel bei Temperaturen im Bereich von 0°C bis zum Siedepunkt der Reaktionsmischung, bevorzugt von 10°C bis 50°C, insbesondere bevorzugt von 15°C bis 35°C. Die Umsetzung kann in einem Lösungsmittel durchgeführt werden, bevorzugt in einem inerten organischen Lösungsmittel.

Als Lösungsmittel kommen prinzipiell die unter der thermischen Cyclisierung zitierten Verbindungen in Betracht, insbesondere ein Tetrahydrofuran - Wasser Gemisch mit einem Mischungsverhältnis von 1 : 10 bis 10 : 1.

Geeignete Basen sind die bei der basischen Cyclisierung von IV zu II (Verfahren B) genannten Basen, insbesondere Kalium-tert-butanolat, 2-Hydroxypyridin oder eine wässrige Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 w/v %ig sein kann, durchgeführt.

Die Verbindungen der Formel XIV können ihrerseits nach der im folgenden Schema dargestellten Synthese in Analogie zu literaturbekannten Verfahren hergestellt werden, beispielsweise nach Wilford L. Mendelson et al., Int. J.Peptide & Protein Research 35(3), (1990), 249-57, Glenn L. Stahl et al., J. Org. Chem. 43(11), (1978), 2285-6. oder A. K. Ghosh et al., Org. Lett. 3(4), (2001), 635-638.

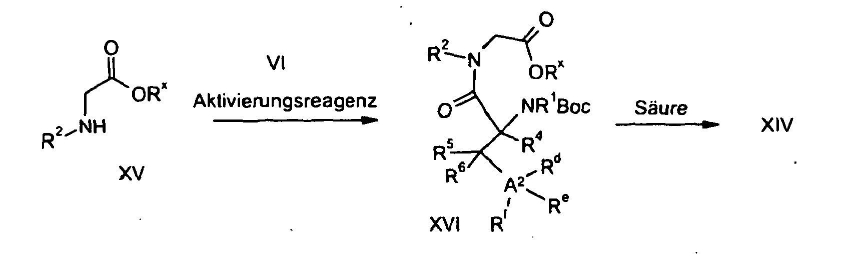

In dem Schema haben die Variablen R^{x}, A², R¹, R², R⁴, R⁵, R⁶, R^{d}, Re und R^{f} die für Formel IV bzw. XIV angegebenen Bedeutungen. Die Synthese umfasst in einem ersten Schritt die Kupplung von Aminosäureverbindungen XV mit Boc-geschützten Aminosäuren VI in Gegenwart eines Aktivierungsreagenz. Anstelle von Boc kann auch eine andere Aminoschutzgruppe eingesetzt werden.

Die Umsetzung einer Verbindung der Formel XV mit einer Verbindung der Formel VI erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel. Wegen weiterer Details wird diesbezüglich auf die Herstellung von Verbindung V durch Amidierung der Aminosäureverbindung VI mit der Verbindung VII verwiesen.

Im Allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenz EDAC oder DCC bevorzugt.

Vorzugsweise erfolgt die Umsetzung von XV mit VI in Gegenwart einer Base. Geeignete Basen sind die unter Verfahren B (Cyclisierung des Dipeptids IV zum Piperazin II) zitierten Verbindungen. In einer Ausführungsform werden als Base Triethylamin oder N-Ethyldiisopropylamin oder deren Mischungen, besonders bevorzugt N-Ethyldiisopropylamin verwendet.

Die Aufarbeitung kann in Analogie zu der bei Verfahren B (Cyclisierung des Dipeptids IV zum Piperazin II) beschriebenen Vorgehensweise erfolgen.

Das Entschützen der Verbindung XVI zur Verbindung XIV erfolgt typischerweise durch Behandlung mit einer Säure. Die Umsetzung erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgeführt werden.

Als Lösungsmittel kommen prinzipiell die unter Verfahren B im Zusammenhang mit der basischen Cyclisierung genannten Lösungsmittel in Betracht, insbesondere Tetrahydrofuran oder Dichlormethan oder deren Mischungen. In einer bevorzugten Ausführungsform wird die Reaktion in Dichlormethan durchgeführt.

Als Säuren finden die bei Verfahren B (Cyclisierung des Dipeptids IV zum Piperazin II) genannten Säuren Verwendung. Wegen weitere Details wird auch auf die Entschützung von V zur Verbindung IV verwiesen. Die dort genannten Reaktionsbedingungen eignen sich auch zum Entschützen von Verbindung XVI. In einer Ausführungsform des erfindungsgemäßen Verfahrens wird die Reaktion in Gegenwart von organischen Säuren, insbesondere starker organischer Säuren durchgeführt, beispielsweise in Gegenwart von Ameisensäure, Essigsäure oder Trifluoressigsäure oder deren Mischungen. In einer bevorzugten Ausführungsform wird die Reaktion in Gegenwart von Trifluoressigsäure durchgeführt.

Wenn anstelle von Boc eine andere Schutzgruppe eingesetzt wird, so wird selbstverständlich ein für die jeweilige Schutzgruppe geeignetes Entschützungsverfähren angewendet.

Die Aufarbeitung kann in Analogie zu der bei Verfahren B (Cyclisierung des Dipeptids IV zum Piperazin II) beschriebenen Vorgehensweise erfolgen.

### Verfahren D

Ein weiterer Gegenstand der Erfindung ist ein Verfahren zur Herstellung von Piperazinverbindungen der allgemeinen Formel I, bei dem man eine Verbindung der Formel IX 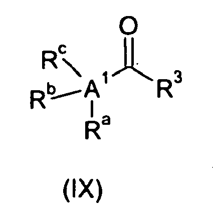 worin die Variablen A¹, R^{a}, R^{b} und R^{c} wie vorstehend definiert sind und R³ für Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl, Phenyl-[C₁-C₆-alkoxycarbonyl]-(C₁-C₆)-alkyl oder Phenylheterocyclyl-(C₁-C₆)-alkyl steht,
mit einem Phosphonsäureester der Formel XVII 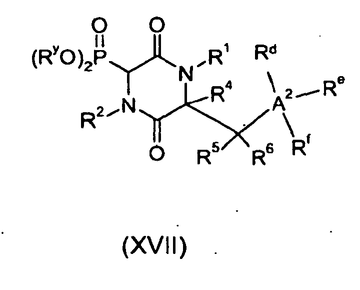 worin die Variablen A², R¹ R², R⁴, R⁵, R⁶, R^{d}, R^{e} und R^{f} wie vorstehend definiert sind und R^{y} für C₁-C₄-Alkyl, z.B. Methyl, Ethyl, Propyl, Isopropyl, Butyl oder Isobutyl, Aryl, z.B. Phenyl, oder Aryl-C₁-C₄-alkyl, z.B. Benzyl, steht,
in Gegenwart einer Base umsetzt.

Die oben genannte Reaktion wird auch als Wittig-Horner-Emmons-Reaktion bezeichnet.

Vorzugsweise steht R^{y} für C₁-C₄-Alkyl und insbesondere für Methyl oder Ethyl.

Vorzugsweise wird dieses Verfahren eingesetzt, um Verbindungen der Formel I.1, worin R³ für H steht, herzustellen. Hierzu geht man gemäß der oben beschriebenen Vorgehensweise vor, wobei man als Verbindung IX den Aldehyd XII (entspricht Verbindung IX, worin R³ für H steht) verwendet und diesen mit dem Phosphonsäureester XVII umsetzt. Dies wird im folgenden Schema skizziert:

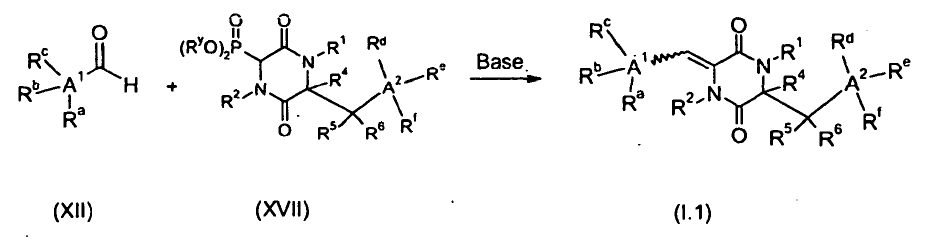

In dem Schema haben die Variablen A¹, A², R¹, R², R⁴, R⁵, R⁶, R^{a}, R^{b}, R^{c}, R^{d}, R^{e} und R^{f} die zuvor genannten Bedeutungen. R^{y} steht für C₁-C₄-Alkyl, z.B. Methyl, Ethyl, Propyl, Isopropyl, n-Butyl, oder Isobutyl, Aryl, z.B. Phenyl, oder Aryl-C₁-C₄-alkyl, z.B. Benzyl. Vorzugsweise steht R^{y} für C₁-C₄-Alkyl und insbesondere für Methyl oder Ethyl.

Geeignete Reaktionsbedingungen sind aus dem Stand der Technik bekannt und beispielsweise in J. Org. Chem. 2001, 66(11), 3984-3997 und in Tetrahedron Lett. 1987, 28, 4275 beschrieben, worauf hiermit in vollem Umfang Bezug genommen wird.

So sind als Basen alle bei Wittig-Horner-Emmons üblichen Basen geeignet, z.B. Alkalihydride wie Natriumhydrid und Alkaliamide wie Lithiumdiisopropylamid. Bevorzugt verwendet man jedoch schwächere Basen wie beispielsweise die zuvor genannten nicht nucleophilen Basen, z.B. die Amidine DBU und DBN oder sterisch gehinderte Alkalialkoholate wie Kalium-tert-butanolat. Geeignete Lösungsmittel sind selbstverständlich aprotisch und beispielsweise unter halogenierten Kohlenwasserstoffen, wie Methylenchlorid, Chloroform und Chlorbenzol, und aromatischen Kohlenwasserstoffen wie Benzol und Toluol ausgewählt.

Grundsätzlich lässt sich die Wittig-Horner-Emmons-Reaktion auch für die Herstellung von Verbindungen I einsetzen, in denen R³ nicht für Wasserstoff stehen muss, sondern auch für C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl stehen kann. In diesem Fall wird anstelle des Aldehyds XII das Keton IX.1 (entspricht Verbindung IX, worin R³ nicht für Wasserstoff steht) eingesetzt 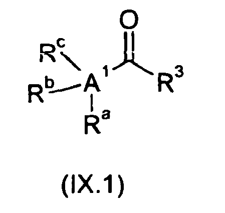 worin R³ für C₁-C₆Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl steht.

Die zuvor gemachten Ausführungen zur Herstellung der Piperazinverbindung der Formel I.1 gelten hier entsprechend. Allerdings sind in diesem Fall meist drastischere Reaktionsbedingungen nötig, z.B. stärkere Basen, höhere Temperaturen, längere Reaktionszeiten, etc., so dass die beschriebene Umsetzung nach Wittig, Horner und Emmons vorzugsweise für die Herstellung von Verbindungen I.1 angewandt wird.

Der Phosphonsäureester XVII ist beispielsweise dadurch erhältlich, dass man das Phosphonat XVIII cyclisiert:

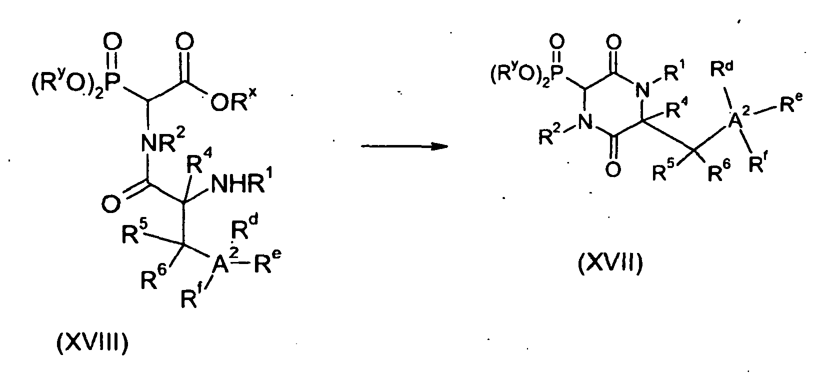

In dem Schema haben die Variablen R^{y}, A², R¹, R², R⁴, R⁵, R⁶, R^{d}, Re und R^{f} die zuvor genannten Bedeutungen. OR^{x} steht für eine über Sauerstoff gebundene Abgangsgruppe, wobei R^{x} vorzugsweise für C₁-C₆-Alkyl, wie Methyl, Ethyl, Propyl, Isopropyl, n-Butyl, Isobutyl, Pentyl oder Hexyl, oder Phenyl-C₁-C₆-alkyl, wie Benzyl, steht.

Die Cyclisierung kann analog zur zuvor beschriebenen Cyclisierung der Verbindung IV zur Verbindung II durchgeführt werden.

Das Phosphonat XVIII kann durch Kopplung des zuvor N-entschützten Phosphonats XIX mit der N-geschützten Aminosäure XX und anschließender Abspaltung der Schutzgruppe hergestellt werden:

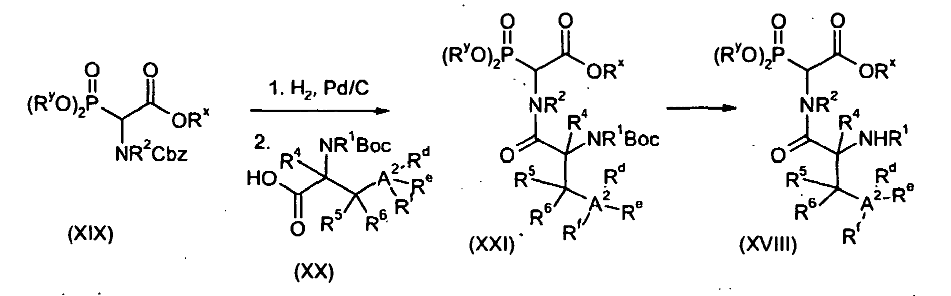

In dem Schema haben die Variablen R^{x}, R^{y}, A², R¹, R², R⁴, R⁵, R⁶, R^{d}, R^{e} und R^{f} die zuvor genannten Bedeutungen. Cbz steht für die Benzyloxycarbonyl-Schutzgruppe. Anstelle von Cbz und Boc können auch andere Aminoschutzgruppen eingesetzt werden.

Die Abspaltung der Cbz-Schutzgruppe erfolgt in der Regel reduktiv mit Wasserstoff in Gegenwart von Palladium auf Kohle. Auch andere herkömmliche Verfahren zur Abspaltung von Cbz-Gruppen können angewendet werden. Wenn andere Schutzgruppen eingesetzt werden, so erfolgt deren Abspaltung in der Regel gemäß den für die jeweilige Gruppe geeigneten Verfahren.

Die obige Umsetzung kann gemäß dem in Tetrahedron 2004, 60, 961-965 beschriebenen Verfahren erfolgen. So kann die Kupplung in Schritt 2 mit der Aminosäure XX in Analogie zur Herstellung der Verbindung V aus den Verbindungen VI und VII erfolgen. Das Entschützen der Verbindung XXI zur Verbindung XVIII kann analog zum Entschützen der Verbindung V zur Verbindung IV durchgeführt werden.

Die Phosphonate XIX sind entweder kommerziell erhältlich oder sie können gemäß den in Synthesis 1986, 53-60 beschriebenen Verfahren hergestellt werden.

### Verfahren E

Ein weiterer Gegenstand der Erfindung ist ein Verfahren zur Herstellung von Piperazinverbindungen der allgemeinen Formel I, bei dem man eine Verbindung der Formel IX 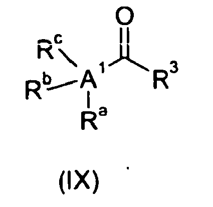 worin die Variablen A¹, R^{a}, R^{b} und R^{c} wie vorstehend definiert sind und R³ für Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl, Phenyl-[C₁-C₆-alkoxycarbonyl]-(C₁-C₆)-alkyl oder Phenylheterocyclyl-(C₁-C₆)-alkyl steht,
mit einem Phosphonsäureester der Formel XXI 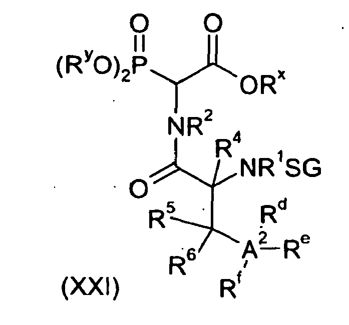 worin die Variablen A², R¹, R², R⁴, R⁵, R⁶, R^{d}, Re und R^{f} wie vorstehend definiert sind, ORₓ für eine über Sauerstoff gebundene Abgangsgruppe steht, wobei R^{x} vorzugsweise für C₁-C₆-Alkyl, wie Methyl, Ethyl, Propyl, Isopropyl, n-Butyl, Isobutyl, Pentyl oder Hexyl, oder Phenyl-C₁-C₆-alkyl, wie Benzyl, steht, R^{y} für C₁-C₄-Alkyl, z.B. Methyl, Ethyl, Propyl, Isopropyl, n-Butyl, oder Isobutyl, Aryl, z.B. Phenyl, oder Aryl-C₁-C₄-alkyl, z.B. benzoyl, steht und SG für eine Aminoschutzgruppe, z.B: Boc, steht,
in Gegenwart einer Base zu der Verbindung der Formel XXII 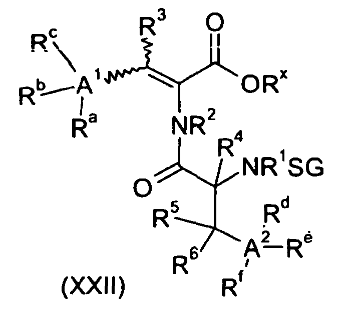 umsetzt,
die Aminoschutzgruppe unter Erhalt der Verbindung der Formel XXIII entfernt 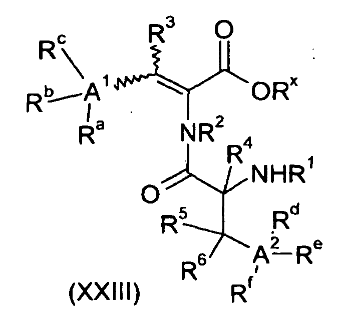 und die Verbindung der Formel XXIII zur Verbindung der Formel I cyclisiert.

Der erste Schritt des erfindungsgemäßen Verfahrens wird auch als Wittig-Horner-Emmons-Reaktion bezeichnet.

Vorzugsweise steht R^{y} für C₁-C₄-Alkyl, insbesondere für Methyl oder Ethyl.

Vorzugsweise wird das erfindungsgemäße Verfahren zur Herstellung von Verbindungen der Formel I.1, worin R³ für H steht, eingesetzt. Hierzu geht man analog zur oben beschriebenen Vorgehensweise vor, wobei man in die Wittig-Horner-Emmons-Reaktion den Aldehyd XII ( entspricht Verbindung IX, worin R³ für H steht) einsetzt. Dieser wird mit dem offenkettigen Phosphonsäureester XXI.1 in das Olefin der Formel XXII.1 überführt, welches nach Entfernen der Schutzgruppe zum Piperazin I.1 cyclisiert wird, wie das folgende Schema veranschaulicht:

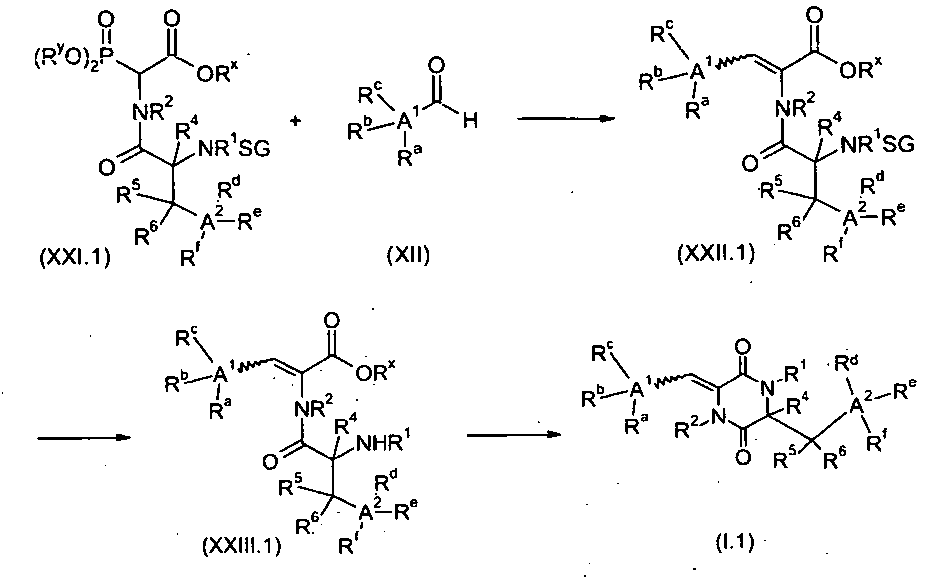

In den Formeln I.1, XII, XXI, XXII.1 und XXIII.1 haben die Variablen A¹, A², R¹- R⁶, R^{a}, R^{b}, R^{c}, R^{d}, R^{e} und R^{f} die für Formel I angegebene Bedeutung. OR^{x} steht für eine über Sauerstoff gebundene Abgangsgruppe, wobei R^{x} vorzugsweise für C₁-C₆-Alkyl, wie Methyl, Ethyl, Propyl, Isopropyl, n-Butyl, Isobutyl, Pentyl oder Hexyl, oder Phenyl-C₁-C₆-alkyl, wie Benzyl, steht. R^{y} steht für C₁-C₄-Alkyl, z.B. Methyl, Ethyl, Propyl, Isopropyl, n-Butyl, oder Isobutyl, Aryl, z.B. Phenyl, oder Aryl-C₁-C₄-alkyl, z.B. Benzyl. Vorzugsweise steht R^{y} für C₁-C₄-Alkyl, insbesondere für Methyl oder Ethyl. SG steht für eine geeignete Aminoschutzgruppe, insbesondere für Boc.

Die Umsetzung des Phosphonats XXI mit dem Aldehyd XII kann analog zur oben beschriebenen Reaktion des Phosphonats XVII mit dem Aldehyd XII erfolgen. Die Umsetzung kann auch analog zu dem in Synthesis 1992, 487-490, Synthesis 1992, 482-486 und Synthesis 1984, 53-60 beschriebenen Verfahren durchgeführt werden, worauf hiermit in vollem Umfang Bezug genommen wird.

Die Abspaltung der Schutzgruppe erfolgt im Allgemeinen nach literaturbekannten Verfahren. Wenn beispielsweise SG für Boc steht, kann die Schutzgruppe wie zuvor für Verbindung V beschrieben, z.B. durch Umsetzung der Verbindung XXII.1 mit einer Säure, erfolgen. Die Durchführung der Entschützungsreaktion kann auch analog zu dem in Tetrahedron 2004, 60, 961-965 beschriebenen Verfahren durchgeführt werden, worauf hiermit in vollem Umfang Bezug genommen wird.

Die Cyclisierung des Dipeptids XXIII.1 zum Piperazin I.1 kann analog zur Umsetzung der Verbindung IV zu Verbindung II erfolgen. In diesem Fall ist jedoch aufgrund der Anwesenheit der Doppelbindung nur die Cyclisierung in Gegenwart einer Säure geeignet. Die bei der Cyclisierung der Verbindung IV gemachten Aussagen zu geeigneten und bevorzugten Säuren und Lösungsmitteln gelten hier entsprechend. Die saure Cyclisierung kann auch gemäß dem in Tetrahedron 2004, 60, 961-965 beschriebenen Verfahren durchgeführt werden, worauf hiermit in vollem Umfang Bezug genommen wird.

In die Wittig-Horner-Emmons-Reaktion kann anstelle der N-geschützten, z.B. der Boc-geschützten Verbindung XXI.1, auch die entsprechende entschützte Verbindung eingesetzt werden, so dass direkt Verbindung XXIII.1 gebildet wird. Die Abspaltung der Schutzgruppe erfolgt im Allgemeinen nach literaturbekannten Verfahren. Wenn beispielsweise SG für Boc steht, kann die Abspaltung der Boc-Schutzgruppe aus Verbindung XXI wie zuvor für Verbindung V beschrieben, z.B. durch Umsetzung der Verbindung XXI mit einer Säure, erfolgen. Die Durchführung der Entschützungsreaktion kann auch analog zu dem in Tetrahedron 2004, 60, 961-965 beschriebenen Verfahren durchgeführt werden, worauf hiermit in vollem Umfang Bezug genommen wird.

Grundsätzlich lässt sich das erfindungsgemäße Verfahren auch für die Herstellung von Verbindungen I einsetzen, in denen R³ nicht für Wasserstoff stehen muss, sondern auch für C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl stehen kann. In diesem Fall wird anstelle des Aldehyds XII das Keton IX.1 eingesetzt 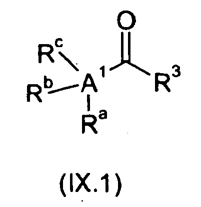 worin R³ für C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl und speziell für C₁-C₆-Alkyl steht.

Die zuvor gemachten Ausführungen zur Herstellung der Piperazinverbindung der Formel I.1 gelten hier entsprechend. Allerdings sind in diesem Fall meist drastischere Reaktionsbedingungen nötig, z.B. stärkere Basen, höhere Temperaturen, längere Reaktionszeiten, etc., so dass die beschriebene Herstellung der Verbindung I über das Phosphonat XXI vorzugsweise für die Herstellung von Verbindungen I.1 angewandt wird.

### Verfahren F

Die Verbindungen der Formel I. mit R¹ ≠ Wasserstoff können auch dadurch hergestellt werden, dass man eine Piperazinverbindung der Formel I, worin R¹ für Wasserstoff steht, mit einem Alkylierungsmittel oder Acylierungsmittel, das den von Wasserstoff verschiedenen Rest R¹ enthält, umsetzt. Derartige Umsetzungen können in Analogie zu literaturbekannten Verfahren erfolgen, beispielsweise nach den von I.O. Donkor et al., Bioorg. Med. Chem. Lett. 11 (19) (2001), 2647-2649, B.B. Snider et al., Tetrahedron 57 (16) (2001), 3301-3307, I. Yasuhiro et al., J. Am. Chem. Soc. 124(47) (2002), 14017-14019, oder M. Falorni et al., Europ. J. Org. Chem. (8) (2000), 1669-1675 beschriebenen Methoden.

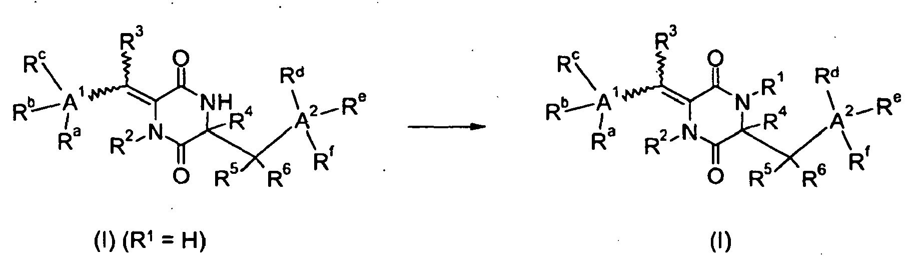

Hierzu wird die Piperazinverbindung der Formel I mit R¹ = Wasserstoff mit einem geeigneten Alkylierungsmittel, im Folgenden Verbindung X¹-R¹, oder Acylierungsmittel, im Folgenden Verbindung X²-R¹, umgesetzt, wobei man eine Piperazinverbindung der Formel I mit R¹ ≠ Wasserstoff erhält.

In den Alkylierungsmitteln X¹-R¹ kann X¹ Halogen oder O-SO₂-R^{m} mit R^{m} in der Bedeutung von C₁-C₄-Alkyl oder Aryl, welche gegebenenfalls durch Halogen, C₁-C₄-Alkyl oder Halo-C₁-C₄-alkyl substituiert sind, bedeuten. In Acylierungsmitteln X²-R¹ kann X² Halogen, insbesondere Cl bedeuten. Dabei ist R¹ ≠ Wasserstoff und hat die oben angegebene Bedeutung und steht insbesondere für C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocyclyl-(C₁-C₆)-alkyl; oder COR²¹ oder SO₂R²⁵, wobei die genannten aliphatischen, cyclischen oder aromatischen Teile von R¹ partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy.

Die Umsetzung erfolgt üblicherweise bei Temperaturen im Bereich von -78°C bis zum Siedepunkt der Reaktionsmischung, bevorzugt von -50°C bis 65°C, insbesondere bevorzugt von -30°C bis 65°C. In der Regel wird die Umsetzung in einem Lösungsmittel vorgenommen, bevorzugt in einem inerten organischen Lösungsmittel.

Geeignete Lösungsmittel sind die unter Verfahren B (Cyclisierung des Dipeptids IV zum Piperazin II) zitierten Verbindungen, unter anderem Toluol, Dichlormethan, Tetrahydrofuran oder Dimethylformamid oder deren Mischungen.

In einer bevorzugten Ausführungsform der Erfindung wird die Reaktion in Tetrahydrofuran durchgeführt.

In einer bevorzugten Ausführungsform wird die Verbindung I mit R¹ = H mit dem Alkylierungs- bzw. Acylierungsmittel in Gegenwart einer Base durchgeführt. Geeignete Basen sind die unter Verfahren B (Cyclisierung des Dipeptids IV zum Piperazin II) zitierten Verbindungen. Die Basen werden im Allgemeinen äquimolar eingesetzt. Sie können auch im Überschuss oder selbst als Lösungsmittel verwendet werden. In einer bevorzugten Ausführungsform wird die Base in äquimolarer Menge oder im Wesentlichen äquimolarer Menge zugesetzt. In einer weiteren bevorzugten Ausführungsform wird Natriumhydrid als Base verwendet.

Die Aufarbeitung erfolgt in der Regel in Analogie zu der unter Verfahren B (Cyclisierung des Dipeptids IV zum Piperazin II) beschriebenen Vorgehensweise.

Die Alkylierung bzw. Acylierung der Gruppe NR¹, worin R¹ für H steht, kann alternativ auch in den Vorstufen erfolgen. So können z.B. Verbindungen II, IV, V, XIII, XIV, XVI, XXII oder XXIII, in denen R¹ für H steht, wie zuvor beschrieben N-alkyliert oder N-acyliert werden.

### Verfahren G

In Analogie zu der oben geschilderten Weise können Verbindungen I oder Vorstufen davon, worin R² für Wasserstoff steht, mit Alkylierungsmitteln R²-X¹ oder Acyclierungsmitteln R²-X² umgesetzt werden, wobei man Verbindungen der Formel I oder deren Vorstufen mit R² ≠ Wasserstoff erhält. Die Reaktionsbedingungen entsprechen den zuvor beschriebenen Bedingungen.

### Verfahren H

Die Verbindungen der Formel I können außerdem an der Gruppe A¹ modifiziert werden. So können sie beispielweise gemäß dem im folgenden Schema skizzierten Verfahren durch Umwandlung des Substituenten R^{a} hergestellt werden, z.B. in Analogie zu den von J. Tsuji, Top. Organomet. Chem. (14) (2005), 332 pp., oder J. Tsuji, Organic Synthesis with Palladium Compounds, (1980), 207 pp. beschriebenen Methoden.

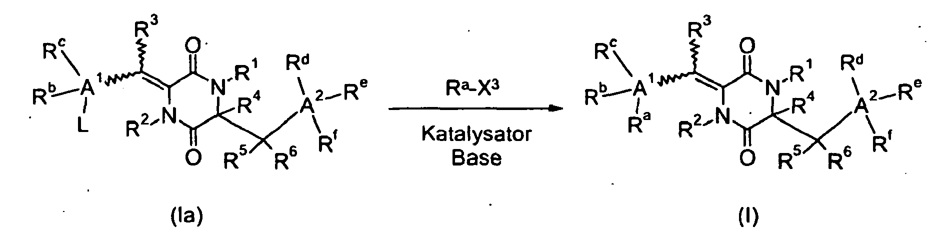

Hierzu wird eine Piperazinverbindung der Formel Ia, die anstelle des Substituenten R^{a} eine geeignete Abgangsgruppe L aufweist, durch Umsetzung mit einem Kupplungspartner, der eine Gruppe R^{a} enthält (Verbindung R^{a}-X³), in ein anderes Piperazinderivat der Formel I überführt.

Die Umsetzung erfolgt üblicherweise in Gegenwart eines Katalysators, bevorzugt in Gegenwart eines Übergangsmetallkatalysators. In der Regel findet die Reaktion in Gegenwart einer Base statt.

Diese Reaktionssequenz ist im Folgenden am Beispiel des Substituenten R^{a} dargestellt und kann selbstredend in analoger Weise für die Umwandlung der Substituenten R^{b} und R^{c} herangezogen werden.

Als Abgangsgruppe L kommen z.B. Halogen oder S(O)ₙR^{k}, mit n = 0, 1, 2 und R^{k} in der Bedeutung von C₁-C₆-Alkyl, Halo-(C₁-C₆)-alkyl oder gegebenenfalls halogeniertem oder mit C₁-C₄-Alkyl substituiertem Aryl in Betracht.

Als Kupplungspartner X³-R^{a} kommen insbesondere solche Verbindungen in Betracht, worin X³ im Falle von R^{a} in der Bedeutung von C₁-C₆-Alkyl, C₂-C₆-Alkenyl, Aryl oder Heteroaryl für eine der folgenden Gruppen steht:
- Zn-R' mit R' in der Bedeutung von Halogen, C₁-C₆-Alkyl, C₂-C₆-Alkenyl, Aryl oder Heteroaryl ;
- B(OR^{m})₂, mit R^{m} in der Bedeutung von H oder C₁-C₆-Alkyl, wobei zwei Alkylsubstituenten zusammen eine C₂-C₄-Alkylenkette bilden können; oder
- SnRⁿ₃, mit Rⁿ in der Bedeutung von C₁-C₆-Alkyl oder Aryl bedeutet.

Sofern R^{a} für C₂-C₆-Alkinyl steht, kann X³ auch Wasserstoff bedeuten.

Zur Herstellung der Verbindung I, worin R^{a} für CN steht, kann man die Verbindung la, worin L für Brom oder Iod steht, auch mit Kupfercyanid in Analogie zu bekannten Verfahren umsetzen (siehe beispielsweise Organikum, 21. Auflage, 2001, Wiley, S. 404 und dort zitierte Literatur).

Nach einer der bevorzugten Ausführungsformen sind dabei L oder R^{a} in den Verbindungen der Formel 1 in ortho-Position zur Verknüpfungsstelle von A¹ an ein C-Atom von A¹ gebunden.

Diese Umsetzung erfolgt üblicherweise bei Temperaturen im Bereich von -78°C bis zum Siedepunkt der Reaktionsmischung, bevorzugt von -30°C bis 65°C, insbesondere bevorzugt bei Temperaturen von 30°C bis 65°C. In der Regel wird die Umsetzung in einem inerten organischen Lösungsmittel in Gegenwart einer Base durchgeführt..

Geeignete Lösungsmittel sind die unter Verfahren B (Cyclisierung des Dipeptids IV zum Piperazin II) zitierten Verbindungen. In einer Ausführungsform des erfindungsgemäßen Verfahrens wird Tetrahydrofuran mit einer katalytischen Menge Wasser verwendet; in einer anderen Ausführungsform wird nur Tetrahydrofuran eingesetzt.

Geeignete Basen sind die unter Verfahren B (Cyclisierung des Dipeptids IV zum Piperazin II) zitierten Verbindungen.

Die Basen werden im Allgemeinen äquimolar eingesetzt. Sie können auch im Überschuss oder selbst als Lösungsmittel verwendet werden.

In einer bevorzugten Ausführungsform des erfindungsgemäßen Verfahrens wird die Base in äquimolarer Menge zugesetzt. In einer weiteren bevorzugten Ausführungsform werden Triethylamin oder Cäsiumcarbonat als Base verwendet, besonders bevorzugt Cäsiumcarbonat.

Als Katalysatoren für das erfindungsgemäße Verfahren sind prinzipiell Verbindungen der Übergangsmetalle Ni, Fe, Pd, oder Cu geeignet. Es ist möglich, organische oder anorganische Verbindungen einzusetzen. Beispielhaft seien genannt: Pd(PPh₃)₂Cl₂, Pd(OAc)₂, PdCl₂, oder Na₂PdCl₄. Ph steht hierbei für Phenyl.

Die verschiedenen Katalysatoren können sowohl einzeln als auch als Gemische eingesetzt werden. In einer bevorzugten Ausführungsform der Erfindung wird Pd(PPh₃)₂Cl₂ verwendet.

Die Aufarbeitung kann analog der in Verfahren B (Cyclisierung des Dipeptids IV zum Piperazin II) beschriebenen Vorgehensweise erfolgen.

Die Umwandlung der Gruppe A¹ kann alternativ auch bei den Vorstufen der Verbindung Ia erfolgen. So können z.B. Verbindungen II, IV, V, VII, VIII, IX, XI und XII, in denen A¹ anstelle des Restes R^{a} eine Gruppe L gebunden enthält, der zuvor beschriebenen Umsetzung unterworfen werden.

### Verfahren I

Piperazinverbindungen der Formel I, worin eine der Gruppen R^{a}, R^{b} oder R^{c} für COOH steht, können außerdem aus Piperazinverbindung der Formel I, worin R^{a}, R^{b} oder R^{c} für COOR^{z} mit R^{z} in der Bedeutung Alkyl, z.B. CH₃, steht, durch Verseifung der Estergruppe hergestellt werden. Die Verseifung gelingt z.B. durch Umsetzung mit (H₃C)₃SnOH, beispielsweise nach K. C. Nicolaou et al., Angew. Chem. Int. Ed. Engl. (44) (2005), 1378. Die so erhaltene Carbonsäure kann dann nach Standardmethoden der organische Synthese, gegebenenfalls nach Überführung in das Säurechlorid, durch Umsetzung mit einem Amin HNR^{u}R^{v} oder einem Alkohol HOR^{w} in den entsprechenden Ester oder das Amid überführt werden ( siehe z.B. Organikum, Autorenkollektiv, Leipzig 1993, 19. Auflage, S. 424, 429). Diese Reaktionssequenz ist im Folgenden am Beispiel des Substituenten R^{a} dargestellt und kann selbstredend in analoger Weise für die Umwandlung der Substituenten R^{b} und R^{c} herangezogen werden.

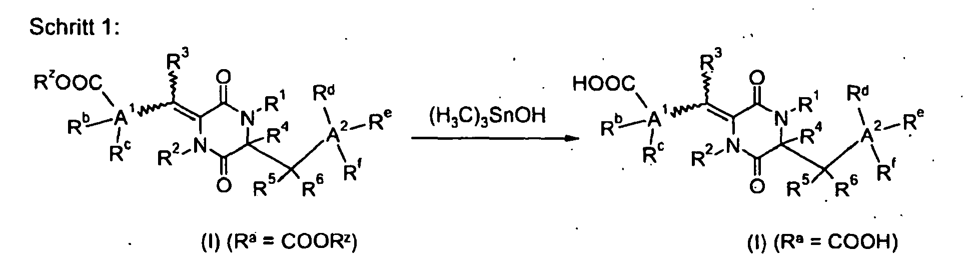

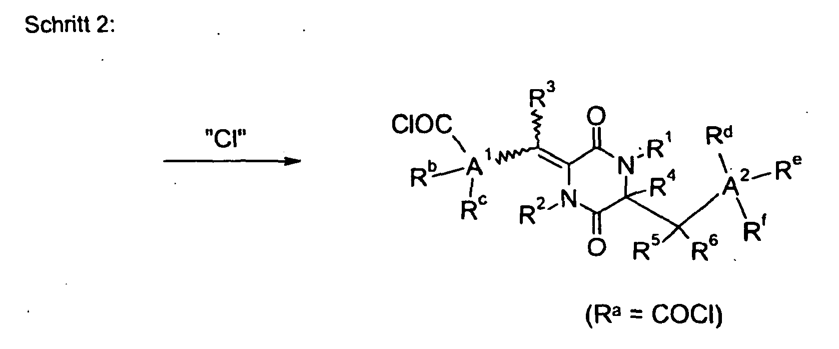

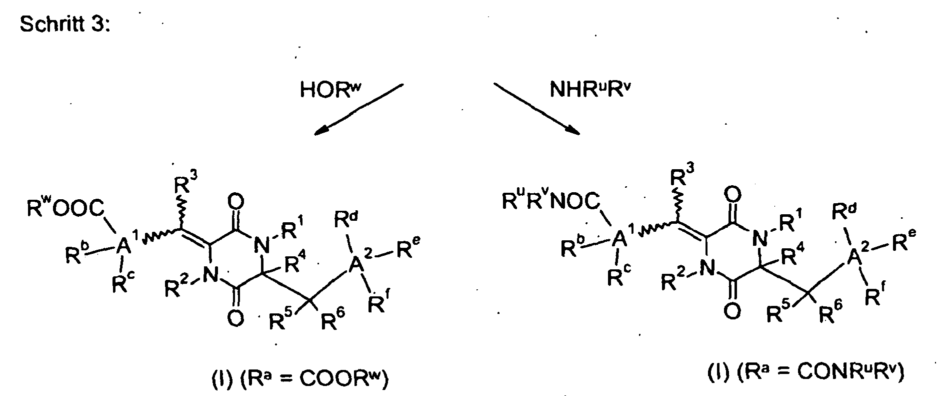

In diesem Schema haben die Variablen A¹, A², R¹-R⁶, R^{b}, R^{c}, R^{d}, Re und R^{f} die zuvor genannten Bedeutungen. R^{u} und R^{v} stehen unabhängig voneinander für Wasserstoff, C₁-C₆-Alkyl, C₂-C₆-Alkenyl, C₂-C₆-Alkinyl, C₁-C₆-Alkoxy; C₁-C₆-Alkylsulfonyl, C₁-C₆-Alkylaminosulfonyl, [Di-(C₁-C₆)-alkylaminolsulfonyl oder ggf. subsituiertes Phenyl. R^{w} steht für C₁-C₆-Alkyl, C₃-C₆-Alkenyl oder C₃-C₆-Alkinyl. R^{z} steht für C₁-C₆-Alkyl, C₃-C₆-Alkenyl, C₃-C₆-Alkinyl , Benzyl, insbesondere für C₁-C₄-Alkyl.

In einem ersten Schritt wird die Estergruppe in der Piperazinverbindung I {R^{a} = COOR^{z}} verseift. Die Verseifung gelingt z.B. durch Umsetzung mit (H₃C)₃SnOH, wobei man die freie Säure von I erhält {R^{a} = COOH}. Die Reaktion zur freien Säure erfolgt üblicherweise mit einem Überschuss an (H₃C)₃SnOH. In der Regel wird die Reaktion in einem inerten organischen Lösungsmittel durchgeführt. Zu den geeigneten Lösungsmitteln zählt insbesondere Dichlorethan. Im Allgemeinen erfolgt die Umsetzung bei erhöhter Temperatur, beispielsweise bei ca. 80°C.

In einem zweiten Schritt wird die Säure I {R^{a} = COOH} in ihr Säurechlorid (R^{a} = COCl) überführt. Die Umsetzung zum Säurechlorid erfolgt üblicherweise bei Temperaturen von 10°C bis 50°C, bevorzugt bei Raumtemperatur, beispielsweise von 25°C. In der Regel wird die Reaktion in einem inerten organischen Lösungsmittel durchgeführt. Zu den geeigneten Lösungsmitteln zählt insbesondere Dichlormethan. In einer bevorzugten Ausführungsform erfolgt die Umsetzung in Dichlormethan und katalytischen Mengen Dimethylformamid. Geeignet sind für die Chlorierung eine Vielzahl von Reagenzien, beispielsweise Oxalylchlorid oder Thionylchlorid. Bevorzugt werden im Wesentlichen äquimolare Mengen des Chlorierungsreagenzes, insbesondere Oxalylchlorid verwendet.

Die Umsetzung mit einem Amin NHR^{u}R^{v} in der Folgereaktion erfolgt üblicherweise durch Zugabe eines Überschusses des jeweiligen Amins. Die Reaktion kann in einem Temperaturbereich von 0°C bis 40°C, bevorzugt bei Raumtemperatur, beispielsweise bei 25°C ausgeführt werden.

Die Umsetzung mit einem Alkohol HOR^{w} in der Folgereaktion erfolgt üblicherweise durch Zugabe eines Überschusses sowohl des jeweiligen Alkohols als auch von Triethylamin.

Die Reaktion kann in einem Temperaturbereich von 0°C bis 40°C, bevorzugt bei Raumtemperatur, beispielsweise bei 25°C ausgeführt werden.

Die Aufarbeitung kann in Analogie zu der bei Verfahren B (Cyclisierung des Dipeptids IV zum Piperazin II) beschriebenen Vorgehensweise erfolgen.

Die Verbindungen I und deren landwirtschaftlich brauchbaren Salze eignen sich - sowohl als Isomerengemische als auch in Form der reinen Isomeren - als Herbizide. Sie eignen sich als solche oder als entsprechend formuliertes Mittel. Die herbiziden Mittel, die die Verbindung I oder Ia enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I oder Ia bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis und prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Darüber hinaus können die Verbindungen I auch in Kulturen, die durch Züchtung einschließlich gentechnischer Methoden gegen Insekten oder Pilzbefall tolerant sind, verwendet werden.

Des Weiteren wurde gefunden, dass die Verbindungen der Formel I auch zur Defoliation und/oder Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Verbindungen der Formel I gefunden.

Als Desikkantien eignet sich die Verbindungen der Formel I insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kem-, Stein- und Schalenobst ermöglicht wird. Derselbe Mechanismus, d.h., die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Qualität der Faser nach der Ernte.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsstoffe.

Beispiele für die Formulierung von Pflanzenschutzmitteln übliche Hilfsmittel sind inerte Hilfsstoffe, feste Trägerstoffe, oberflächenaktive Stoffe (wie Dispergiermittel Schutzkolloide, Emulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide wie Xanthan Gum (Kelzan® der Fa. Kelco), Rhodopol® 23 (Rhone Poulenc) oder Veegum® (Firma R.T. Vanderbilt) sowie organische und anorganische Schichtmineralienwie Attaclay® (Firma Engelhardt).

Beispiele für Antischaummittel sind Silikonemulsionen (wie z.Bsp. Silikon® SRE, Firma Wacker oder Rhodorsil® der Firma Rhodia ), langkettige Alkohole, Fettsäuren, Salze von Fettsäuren, fluororganische Verbindungen und deren Gemische.

Bakterizide können zur Stabilisierung der wäßrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Beispiele für Kleber sind Polyvinylpyrrolidon, Polyvinylacetat, Polyvinylalkohol und Tylose.

### Als inerte Zusatzstoffe kommen beispielsweise in Betracht:

Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Als oberflächenaktive Stoffe (Adjuvantien, Netz-, Haft-, Dispergier- sowie Emulgiermittel) kommen die Alkali-, Erdalkali-, Ammoniumsalze von aromatischen Sulfonsäuren, z.B. Ligninsulfonsäuren (z.B. Borrespers-Typen, Borregaard), Phenolsulfonsäuren, Naphthalinsulfonsäuren (Morwet-Typen, Akzo Nobel) und Dibutylnaphthalinsulfonsäure (Nekal-Typen, BASF AG), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol typen Clariant), Polycarboxylate (BASF AG, Sokalan-Typen), Polyalkoxylate, Polyvinylamin (BASF AG, Lupamin-Typen), Polyethylenimin (BASF AG, Lupasol-Typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I oder Ia als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel I in den anwendungsfertigen Zubereitungen können in weiten Bereichen variiert werden. Die Formulierungen enthalten im Allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:

### 1. Produkte zur Verdünnung in Wasser

### A Wasserlösliche Konzentrate

10 Gew.-Teile Wirkstoff werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate

20 Gew.-Teile Wirkstoff werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%.

### C Emulgierbare Konzentrate

15 Gew.-Teile Wirkstoff werden in 75 Gew.-Teilen eines organisches Lösungsmittel (z.B. Alkylaromaten)-unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen

25 Gew.-Teile Wirkstoff werden in 35 Gew.-Teilen eines organisches Lösungsmittel (z.B. Alkylaromaten) unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen

20 Gew.-Teile Wirkstoff werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F Wasserdispergierbare und wasserlösliche Granulate

50 Gew.-Teile Wirkstoff werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver

75 Gew.-Teile Wirkstoff werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile Wirkstoff, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube

5 Gew.-Teile Wirkstoff werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile Wirkstoff werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile Wirkstoff werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Im Folgenden sind konkrete Formulierungen angegeben:
I 20 Gewichtsteile der Verbindung der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II 20 Gewichtsteile der Verbindung der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
III 20 Gewichtsteile der Verbindung der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
IV 20 Gewichtsteile der Verbindung der Formel I werden mit 3 Gewichtsteilen des Natriumsalzes der Düsobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew. % des Wirkstoffs enthält.
V 3 Gewichtsteile der Verbindung der Formel I werden mit 97 Gewichtsteilen feinteiligem Kaolin vermischt. Man erhält auf diese Weise ein Stäubemittel, das 3 Gew. % des Wirkstoffs enthält.
VI 20 Gewichtsteile der Verbindung der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
VII 1 Gewichtsteil der Verbindung Verbindung der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII 1 Gewichtsteil der Verbindung der Formel I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Die Applikation der Verbindungen I oder der sie enthaltenden herbiziden Mittel kann im Vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel I bzw. der herbiziden Mittel durch Behandlung von Saatgut erfolgen.

Die Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting") basierend auf den erfindungsgemäßen Verbindungen der Formel I bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.). Zur Saatgutbehandlung werden die Verbindungen I üblicherweise in Mengen von 0,001 bis 10kg pro 100kg Saatgut eingesetzt.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, 2-Hetaroyl-1,3-cyclohexandione, Hetaryl-Aryl-Ketone, Benzylisoxazolidinone, meta-CF₃-Phenylderivate, Carbamate, Chinolincarbonsäure und deren Derivate, Chloracetanilide, Cyclohexenonoximether - Derivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile, Phenylpyrazoline, Isoxazoline und deren Derivate in Betracht.

Außerdem kann es von Nutzen sein, die Verbindungen I allein oder in Kombination mit anderen Herbiziden oder auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additve wie nichtphytotoxische Öle und Ölkonzentrate zugesetzt werden.

Die folgenden Beispiele dienen der Erläuterung der Erfindung.

### A Herstellungsbeispiele

Die Produkte wurden durch über ihrer Retentionszeit RT (in min.) in einer HPLC/MS (High Performance Liquid Chromatographie kombiniert mit Massen Spektrometrie), durch NMR oder durch ihren Schmelzpunkt (m.p.) charakterisiert.

HPLC-Säule: RP-18 Säule (Chromolith Speed ROD von Merck KgaA, Deutschland) Eluent: Acetonitril + 0.1% Trifuoroessigsäure (TFA)/ Wasser + 0.1% TFA in einem Gradient von 5:95 bis 95:5 in 5 Minuten bei 40°C.
MS: Quadrupol Elektrospray Ionisation, 80 V (positiv Modus)

### Beispiel 1: 3-Benzyl-1,4-dimethyl-6-(2-nitro-benzylidene)-piperazin-2,5-dion

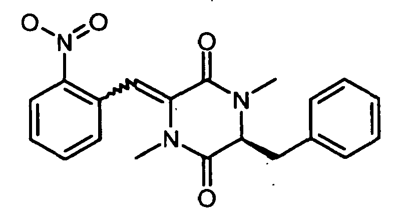

### 1.1 2-(tert-Butoxycarbonyl-methyl-amino)-3-(2-nitro-phenyl)-3-trimethylsilanyloxy-propionsäureethylester

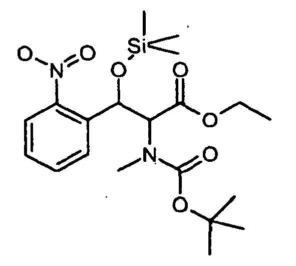

Zu (tert-Butoxycarbonylmethylamino)essigsäureethylester (20 g, 92 mmol) in THF (abs., 50 mL) tropfte man bei - 78 °C langsam Lithium-di-isopropylamid-Lösung (2 M in Tetrahydrofuran/n-Heptan, 46 mL, 92 mmol). Es wird für 3 h bei dieser Temperatur nachgerührt. Dann tropfte man 2-Nitrobenzaldehyd (13.6 g, 90 mmol) in THF (Tetrahydrofuran, absolut, 30 mL) langsam zu. Es wurde 1.5 h bei - 78 °C nachgerührt, bevor man Trimethylsilylchlorid (10 g, 92 mmol) zutropfte. Die Reaktionslösung wurde langsam (12 h) auf Raumtemperatur erwärmt und anschließend am Rotationsverdampfer eingeengt. Der Rückstand wurde in Essigsäureethylester aufgenommen, gewaschen, getrocknet und eingeengt. Der so erhaltene Rückstand wurde anschließend säulenchromatographisch (SiO₂, Hexan/Essigsäureethylester) gereinigt. Es wurden 7.1 g (18 %) eines unpolaren Isomers erhalten, die in der nächsten Stufe weiter umgesetzt wurden.
M+Na (m/z): 463.

### 1.2 3-Hydroxy-2-methylamino-3-(2-nitro-phenyl)-propionsäureethylester

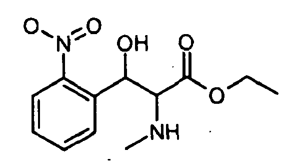

Zu 2-(tert-Butoxycarbonylmethylamino)-3-(2-nitrophenyl)-3-trimethylsilanyloxy-propionsäureethylester (8.6 g, 19.5 mmol) in CH₂Cl₂ (100 mL) gab man Trifluoressigsäure (20 mL) und rührte 12 h bei Raumtemperatur nach. Anschließend wurde mit NaHCO₃-Lösung (gesättigt) neutralisiert, die Phasen wurden getrennt und eingeengt. Der so erhaltene Rückstand wurde säulenchromatographisch (SiO₂, Hexan/Essigsäureethylester) gereinigt. Man erhielt so 1.7 g (32 %) der Zielverbindung als hellgelber Feststoff.
M+1 (m/z): 269.

### 1.3 2-{[2-(tert-Butoxycarbonyl-methyl-amino)-3-phenyl-propionyl]-methyl-amino}-3-hydroxy-3-(2-nitro-phenyl)-propionsäure ethyl ester

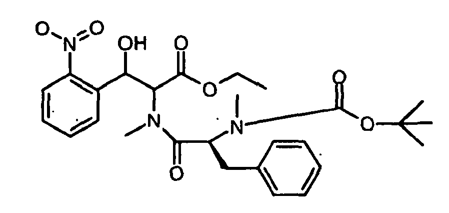

3-Hydroxy-2-methylamino-3-(2-nitrophenyl)-propionsäureethylester (1.7 g, 6.3 mmol), 2-(tert-Butoxycarbonylmethylamino)-3-phenylpropionsäure (2 g, 7 mmol), N-Ethyldiisopropylamin (4.5 g, 35 mmol) und EDAC (3 g, 15,6 mmol) wurden in THF (abs., 50 mL) 3 Tage gerührt. Die Reaktionslösung wurde am Rotationsverdampfer eingeengt. Der Rückstand wurde in Essigester aufgenommen und die erhaltene Lösung wurde gewaschen, getrocknet und eingeengt. Es wurden so 2.1 g (63 %) der Zielverbindung als hellgelbes Öl erhalten.
M+1 (m/z): 530.

### 1.4 3-Hydroxy-2-[methyl-(2-methylamino-3-phenyl-propionyl)-amino]-3-(2-nitrophenyl)-propionsäure ethyl erster.

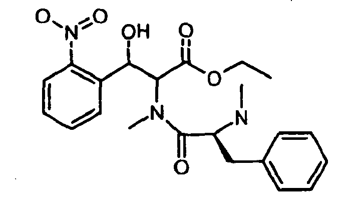

Zu 2-{[2-(tert-Butoxycarbonyl-methyl-amino)-3-phenyl-propionyl]-methyl-amino}-3-hydroxy-3-(2-nitro-phenyl)-propionsäureethylester (2.1 g, 3.9 mmol) in CH₂Cl₂ (20 mL) gab man Trifluoressigsäure (10 mL), rührte 2 h bei Raumtemperatur nach und engte anschließend am Rotationsverdampfer ein. Der so erhaltene Rückstand wurde als Rohprodukt im Folgeschritt umgesetzt.

### 1.5 3-Benzyl-6-[hydroxy-(2-nitro-phenyl)-methyl]-1,4-dimethyl-piperazin-2,5-dion

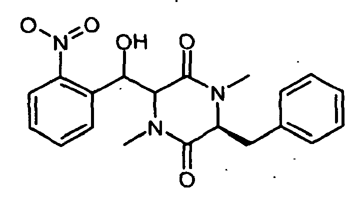

Der unter 1.4 erhaltene Rückstand wird in THF (50 mL) aufgenommen und mit NH₄OH (25 % in H₂O, 10 mL) versetzt. Es wird bei Raumtemperatur für 12 h gerührt. Nach Zugabe von H₂O (100 mL) wird mit Methyl-tert.-Butylether extrahiert, die organische Phase getrocknet und eingeengt. Der so erhaltene Rückstand wird säulenchromatographisch (SiO₂, Hexan/Essigester) gereinigt. Es werden so 0.57 g (38 %) eines polaren Isomers erhalten, die in der nächsten Stufe weiter umgesetzt werden.
M+1 (m/z): 384.

### 1.6 Methanesulfonsäure-[(5-benzyl-1,4-dimethyl-3,6-dioxo-piperazin-2-yl)-(2-nitrophenyl)methyl]ester

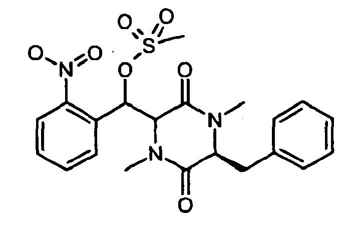

Zu 3-Benzyl-6-[hydroxy-(2-nitro-phenyl)-methyl]-1,4-dimethyl-piperazin-2,5-dion (5.5 g, 14.3 mmoi) in Pyridin (100 mL) gab man DMAP (1.8 g, 14.7 mmol) und Methansulfonylchlorid (30 mL), rührte 12 h bei Raumtemperatur nach und engte anschließend am Rotationsverdampfer ein. Nach Zugabe von H₂O und CH₂Cl₂ wurden die unlöslichen schwarzen Harze über eine Nutsche abfiltriert, die Phasen getrennt und die organische Phase eingeengt. Der so erhaltene Rückstand wurde säulenchromatographisch (SiO₂, Hexan/Essigsäureethylester) gereinigt. Es wurden so 5.1 g (77 %) der Zielverbindung als hellgelber Schaum erhalten.
M+1 (m/z): 462.

### 1.7 3-Benzyl-1,4-dimethyl-6-(2-nitro-benzylidene)-piperazin-2,5-dion

Zu Methanesulfonsäure-[(5-benzyl-1,4-dimethyl-3,6-dioxo-piperazin-2-yl)-(2-nitrophenyl)-methyl]ester (4.25 g, 9 mmol) in THF (100 mL) tropfte man bei 0 °C langsam DBU (1.4 g, 9 mmol) und rührte 4 h bei 0 °C nach. Anschließend wurde bei dieser Temperatur mit Zitronensäure (10 %) auf pH 7 gestellt und man ließ danach langsam auf Raumtemperatur erwärmen. Nach Zugabe von H₂O und Essigsäureethylester wurden die Phasen getrennt und die organische Phase wurde eingeengt. Der so erhaltene Rückstand wurde säulenchromatographisch (SiO₂, Methyl-tert.-Butylether /Essigsäureethylester) gereinigt. Es wurden so 2.5 g (76 %) der Zielverbindung als gelber Schaum erhalten.
Die so erhaltene Z:E-Isomerenmischung wurde mittels einer präparativen MPLC (Kieselgel: Merck Lichroprep RP-18 (40-63 µm), MeOH:H₂O = 60:40) aufgetrennt. ¹H-NMR (CDCl₃) der getrennten Isomere:
a) δ = 2.62 (s, 3H), 3.09 (s, 3H), 3.23 (m, 2H), 4.39 (m, 1H), 6.39 (d, 1H), 7.13 (s, 1H), 7.17 (m, 1H), 7.24 (m, 1H), 7.32 (m, 2H), 7.44 (m, 1H), 7.49 (m, 1H), 8.05 (d, 2H).
b) δ = 2.91 (s, 3H), 3.15 (dm, 1H), 3.33 (s, 3H), 3.29 (dm, 1H), 4.32 (m, 1H), 6.28 (s, 1H), 6.75 (m, 1H), 7.08 (m, 2H), 7.32 (m, 3H), 7.39 (m, 1H), 7.47 (m, 1H), 8.04 (d, 1H).

### Beispiel 105/106: 3-Benzyl-1,4-dimethyl-6-(2-thiazol-2-ylbenzyliden)-piperazin-2,5-dion

In einem Reaktionsgefäß wurden unter Argonatmossphäre 600 mg 3-Benzyl-1,4-dimethyl-6-(2-iodbenzyliden)-piperazin-2,5-dion (Beispiel 4, hergestellt analog Beispiel 1), 250 mg Bis(dibenzylidenaceton)palladium und 500 mg Triphenylarsan in 30 ml Dioxan vorgelegt. Hierzu gab man 1 g 2-(Tributylstannyl)thiazol und erwärmte 16 h auf 60°C. Man engte die Reaktionsmischung im Vakuum ein und chromatographierte den Rückstand an Kieselgel mit Hexan/Methyl-tert.-butylether (2:1 v/v) chromatigraphiert.

Auf diese Weise erhielt man insgesamt 404 mg der Titelverbindung als wachsartigen Feststoff.

### Beispiel 115: 3-Benzyl-1,4-dimethyl-6-(2-cyano-5,6-difluorbenzyliden)-piperazin-2,5-dion

In einem Reaktionsgefäß wurden unter Argonatmossphäre 2,0 g 3-Benzyl-1,4-dimethyl-6-(2-brom-5,6-difluorbenzyliden)-piperazin-2,5-dion (Beispiel 119, hergestellt analog Beispiel 1) mit 1,7 g Kuperf(I)cyanid in 50 ml N-Methylpyrrolidon 18 h bei 155°C umgesetzt. Man engte die Reaktionsmischung im Vakuum ein und nahm den Rückstand in Ethylacetat auf, wusch die erhaltene Lösung 3 mal mit Wasser, trocknete und engte erneut im Vakuum ein. Der Rückstand wurde an Kieselgel mit Hexan/Ethylacetat (1:1 v/v) chromatigraphiert. Auf diese Weise erhielt man 331 mg des Z-Isomers als hellgelben Feststoff mit einem Festpunkt von 175°C und 310 mg des E-Isomers als beige-farbenen Feststoff mit einem Schmelzpunkt von 205°C.

In analoger Weise wurden die in den nachfolgenden Tabellen 2, 3 und 4 angegebenen Verbindungen und die in Tabelle 5 angegebenen Verbindungen hergestellt (Beispiele 2 bis 214).

**Tabelle 2:**

| | | | | | | |
|---|---|---|---|---|---|---|
| 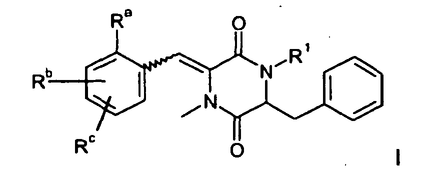 | | | | | | |

| Bsp. | R^{a} | R^{b} | R^{c} | R¹ | RT | Isomer |
|---|---|---|---|---|---|---|
| Nr. | | | | | HPLC/MS oder m.p. | |
| 1 | 2-NO₂ | H | H | CH₃ | 2,831 min | |
| | | | | | m/z= | |
| | | | | | 366,0 | |
| | | | | | [M+H]⁺ | |
| 2 | 2-NO₂ | H | H | H | 2,724 min | Isomer 1 |
| | | | | | m/z= | |
| | | | | | 352,4 | |
| | | | | | [M+H]⁺ | |
| 3 | 2-NO₂ | H | H | H | 2,773 min | Isomer 2 |
| | | | | | m/z= | |
| | | | | | 352,4 | |
| | | | | | [M+H]⁺ | |
| 4 | 2-I | H | H | CH₃ | 3,286 min | |
| | | | | | m/z= | |
| | | | | | 446,9 | |
| | | | | | [M+H]⁺ | |
| 5 | 2-(CH₃)₃Si-C≡C- | H | H | CH₃ | 4,039 min | Isomer 1 |
| | | | | | m/z= | |
| | | | | | 417,5 | |
| | | | | | [M+H]⁺ | |
| 6 | 2-(CH₃)₃Si-C≡C- | H | H | CH₃ | 4,064 min | Isomer 2 |
| | | | | | m/z= | |
| | | | | | 417,5 | |
| | | | | | [M+H]⁺ | |
| 7 | 2-HC≡C- | H | H | CH₃ | 3,053 min | |
| | | | | | m/z= | |
| | | | | | 345,0 | |
| | | | | | [M+H]⁺ | |
| 8 | 2-NO₂ | 5-Cl | H | CH₃ | 3,128 min | Isomer 1 |
| | | | | | m/z= | |
| | | | | | 399,9 | |
| | | | | | [M+H]⁺ | |
| 9 | 2-NO₂ | 5-Cl | H | CH₃ | 3,215 min | Isomer 2 |
| | | | | | m/z= | |
| | | | | | 399,9 | |
| | | | | | [M+H]⁺ | |
| 10 | 2-NO₂ | 4-OCH₃ | 5-OCH₃ | CH₃ | 2,801 min | Isomer 1 |
| | | | | | m/z= | |
| | | | | | 426,0 | |
| | | | | | [M+H]⁺ | |
| 11 | 2-NO₂ | 4-OCH₃ | 5-OCH₃ | CH₃ | 2,828 min | Iso mer 2 |
| | | | | | m/z= | |
| | | | | | 426,0 | |
| | | | | | [M+H]⁺ | |
| 12 | 2-NO₂ | 6-CH₃ | H | CH₃ | 3,088 min | Isomer 1 |
| | | | | | m/z= | |
| | | | | | 380,0 | |
| | | | | | [M+H]⁺ | |
| 13 | 2-NO₂ | 6-CH₃ | H | CH₃ | 3,091 min | Isomer 2 |
| | | | | | m/z= | |
| | | | | | 380,0 | |
| | | | | | [M+H]⁺ | |
| 14 | 2-CN | H | H | CH₃ | 2,721 min | |
| | | | | | m/z= | |
| | | | | | 346,1 | |
| | | | | | [M+H]⁺ | |
| | | | | | 143 °C | |
| 15 | 2-NO₂ | 4-N(CH₃)₂ | H | CH₃ | 3,038 min | |
| | | | | | m/z= | |
| | | | | | 409,0 | |
| | | | | | [M+H]⁺ | |
| 16 | 2-NO₂ | 4,5-(-O-CH₂-O-) | | CH₃ | 2,778 min | Isomer 1 |
| | | | | | m/z= | |
| | | | | | 410,0 | |
| | | | | | [M+H]⁺ | |
| 17 | 2-NO₂ | 4,5-(-O-CH₂-O-) | | CH₃ | 2,856 min | Isomer 2 |
| | | | | | m/z= | |
| | | | | | 410,0 | |
| | | | | | [M+H]⁺ | |
| 18 | 2-NO₂ | 6-Cl | H | CH₃ | 3,105 min | |
| | | | | | m/z=400,1 | |
| | | | | | [M+H]⁺ | |
| 19 | 2-NO₂ | 5-F | H | CH₃ | 2,973 min | Isomer 1 |
| | | | | | m/z=384,4 | |
| | | | | | [M+H]⁺ | |
| 20 | 2-NO₂ | 5-F | H | CH₃ | 3,037 min | Isomer 2 |
| | | | | | m/z=384,4 | |
| | | | | | [M+H]⁺ | |
| 21 | 2-NO₂ | 3-OCH₃ | H | CH₃ | 2,926 min | |
| | | | | | m/z=396,1 | |
| | | | | | [M+H]⁺ | |
| 22 | 2-NO₂ | 5-OCH₃ | 6- | CH₃ | 3,327 min | |
| | | | O-SO₂- | | m/z=552,1 | |
| | | | Ph | | [M+H]⁺ | |
| 23 | 2-NH₂ | H | H | CH₃ | 2,249 min | Isomer 1 |
| | | | | | m/z=336,4 | |
| | | | | | [M+H]⁺ | |
| 24 | 2-NH₂ | H | H | CH₃ | 2,079 min | Isomer 2 |
| | | | | | m/z=336,1 | |
| | | | | | [M+H]⁺ | |
| 25 | 2-NH-C(O)-CH₃ | H | H | CH₃ | 2,372 min | |
| | | | | | m/z=378,1 | |
| | | | | | [M+H]⁺ | |
| 26 | 2-NH-C(O)-Ph | H | H | CH₃ | 2,837 min | |
| | | | | | m/z=440,2 | |
| | | | | | [M+H]⁺ | |
| 27 | 2-NH-C(O)-NH-SO₂-Ph | H | H | CH₃ | 3,095 min | |
| | | | | | m/z=533,2 | |
| | | | | | [M+H]⁺ | |
| 28 | 2-NH-SO₂NHCH₃ | H | H | CH₃ | 2,505 min | |
| | | | | | m/z=429,1 | |
| | | | | | [M+H]⁺ | |
| 29 | 2-NH-C(O)-NH-Ph | H | H | CH₃ | 3,140 min | |
| | | | | | m/z=455,2 | |
| | | | | | [M+H]⁺ | |
| 30 | 2-NH-C(O)-OCH₃ | H | H | CH₃ | 2,667 min | Isomer 1 |
| | | | | | m/z=394,4 | |
| | | | | | [M+H]⁺ | |
| 31 | 2-NH-C(O)-OCH₃ | H | H | CH₃ | 2,590 min | Isomer 2 |
| | | | | | m/z=394,2 | |
| | | | | | [M+H]⁺ | |
| 32 | 2-NH-SO₂-NHCH₂CH₃ | H | H | CH₃ | 2,642 min | |
| | | | | | m/z=443,1 | |
| | | | | | [M+H]⁺ | |
| 33 | 2-NH-SO₂-NHCH(CH₃)₂ | H | H | CH₃ | 2,848 min | |
| | | | | | m/z=457,1 | |
| | | | | | [M+H]⁺ | |
| 34 | 2-NH-CN | H | H | CH₃ | 2,579 min | Isomer 1 |
| | | | | | m/z=361,1 | |
| | | | | | [M+H]⁺ | |
| 35 | 2-NH-CN | H | H | CH₃ | 2,637 min | Isomer 2 |
| | | | | | m/z=361,2 | |
| | | | | | [M+H]⁺ | |
| 36 | 2-F | H | H | CH₃ | 3,009 min | Isomer 1 |
| | | | | | m/z=339,4 | |
| | | | | | [M+H]⁺ | |
| 37 | 2-F | H | H | CH₃ | 2,762 min | Isomer 2 |
| | | | | | m/z=339,4 | |
| | | | | | [M+H]⁺ | |
| 38 | 2-I | 6-F | H | CH₃ | 3,259 min | |
| | | | | | m/z=465,0 | |
| | | | | | [M+H]⁺ | |
| 39 | 2-CN | 6-F | H | CH₃ | 3,033 min | |
| | | | | | m/z=364,1 | |
| | | | | | [M+H]⁺ | |
| | | | | | 138 °C | |
| 40 | 2-I | 6-CI | H | CH₃ | 3,625 min | |
| | | | | | m/z=481,0 | |
| | | | | | [M+H]⁺ | |
| 41 | 2-Br | 6-Cl | H | CH₃ | 3,437 min | |
| | | | | | m/z=434,9 | |
| | | | | | [M+H]⁺ | |
| 42 | 2-CN | H | H | H | 2,656 min | |
| | | | | | m/z=332,1 | |
| | | | | | [M+H]⁺ | |
| | | | | | 158 °C | |
| 43 | 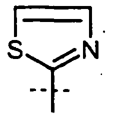 | H | H | CH₃ | 2,936 min | Isomer 1 |
| | | | | | m/z=404,1 | |
| | | | | | [M+H]⁺ | |
| 44 | 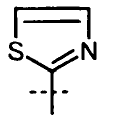 | H | H | CH₃ | 2,806 min | Isomer 2 |
| | | | | | m/z=404,1 | |
| | | | | | [M+H]⁺ | |
| 45 | 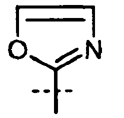 | H | H | CH₃ | 2,878 min | Isomer 1 |
| | | | | | m/z=388,1 | |
| | | | | | [M+H]⁺ | |
| 46 | 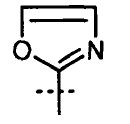 | H | H | CH₃ | 2,867 min | Iso mer 2 |
| | | | | | m/z=388,1 | |
| | | | | | [M+H]⁺ | |
| | | | | | 153 °C | |
| 47 | 2-(Furan-2-yl) | H | H | CH₃ | 3,173 min | Isomer 1 |
| | | | | | m/z=387,1 | |
| | | | | | [M+H]⁺ | |
| 48 | 2-(Furan-2-yl) | H | H | CH₃ | 3,284 min | Isomer 2 |
| | | | | | m/z=387,1 | |
| | | | | | [M+H]⁺ | |
| | | | | | | |
| 49 | 2-(Thiophen-2-yl) | H | H | CH₃ | 3,378 min | |
| | | | | | m/z=403,1 | |
| | | | | | [M+H]⁺ | |
| 50 | 2-(Pyridin-2-yl) | H | H | CH₃ | 2.131 min | |
| | | | | | m/z=398,2 | |
| | | | | | [M+H]⁺ | |
| 51 | 2-(Pyridin-3-yl) | H | H | CH₃ | 2,214 min | |
| | | | | | m/z=398,2 | |
| | | | | | [M+H]⁺ | |
| 52 | 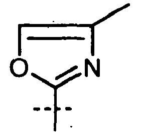 | H | H | CH₃ | 2,963 min | Isomer 1 |
| | | | | | m/z=402,2 | |
| | | | | | [M+H]⁺ | |
| 53 | 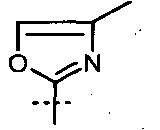 | H | H | CH₃ | 2,989 min | Isomer 2 |
| | | | | | m/z=402,2 | |
| | | | | | [M+H]⁺ | |
| | | | | | 128 °C | |
| 54 | 2-CF₃ | H | H | CH₃ | 3,164 min | |
| | | | | | m/z=389,1 | |
| | | | | | [M+H]⁺ | |
| 55 | 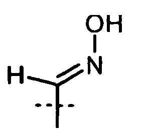 | H | H | CH₃ | 2,601 min | |
| | | | | | m/z=387,4 | |
| | | | | | [M+Na]⁺ | |
| 56 | 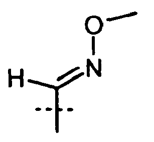 | H | H | CH₃ | 2,985 min | |
| | | | | | m/z=378,2 | |
| | | | | | [M+H]⁺ | |
| 57 | 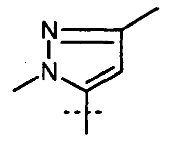 | H | H | CH₃ | 2,803 min | |
| | | | | | m/z=415,5 | |
| | | | | | [M+H]⁺ | |
| 58 | 2-Br | 5-CF₃ | H | CH₃ | 3,435 min | Isomer 1 |
| | | | | | m/z=469,0 | |
| | | | | | [M+H]⁺ | |
| 59 | 2-Br | 5-CF₃ | H | CH₃ | 3,490 min | Isomer 2 |
| | | | | | m/z=469,0 | |
| | | | | | [M+H]⁺ | |
| | | | | | 113°C | |
| 60 | 2-Br | 4-OCH₃ | 5-OCH₃ | CH₃ | 3,074 min | Isomer 1 |
| | | | | | m/z=461,0 | |
| | | | | | [M+H]⁺ | |
| | | | | | 121 °C | |
| 61 | 2-Br | 4-OCH₃ | 5-OCH₃ | CH₃ | 3,131 min | Isomer 2 |
| | | | | | m/z=461,1 | |
| | | | | | [M+H]⁺ | |
| 62 | 2-Br | 5-F | H | CH₃ | 3,380 min | |
| | | | | | m/z=417,0 | |
| | | | | | [M+H]⁺ | |
| | | | | | 106 °C | |
| 63 | 2-CN | 5-CF₃ | H | CH₃ | 3,338 min | Isomer 1 |
| | | | | | m/z=414,1 | |
| | | | | | [M+H]⁺ | |
| 64 | 2-CN | 5-CF₃ | H | CH₃ | 3,376 min | Isomer 2 |
| | | | | | m/z=414,1 | |
| | | | | | [M+H]⁺ | |
| 65 | 2-CN | 4-OCH₃ | 5-OCH₃ | CH₃ | 2,805 min | Isomer 1 |
| | | | | | m/z=406,1 | |
| | | | | | [M+H]⁺ | |
| | | | | | 163 °C | |
| 66 | 2-CN | 4-OCH₃ | 5-OCH₃ | CH₃ | 2,764 min | Isomer 2 |
| | | | | | m/z=406,1 | |
| | | | | | [M+H]⁺ | |
| 67 | 2-CN | 5-F | H | CH₃ | 2,939 min | Isomer 1 |
| | | | | | m/z=364,4 | |
| | | | | | [M+H]⁺ | |
| 68 | 2-CN | 5-F | H | CH₃ | 2,950 min | Isomer 2 |
| | | | | | m/z=364,1 | |
| | | | | | [M+H]⁺ | |
| | | | | | 128 °C | |
| 69 | 2-COOH | H | H | CH₃ | 2,539 min | |
| | | | | | m/z=365,1 | |
| | | | | | [M+H]⁺ | |
| 70 | 2-C(O)NHCH₂CH₂CH₃ | H | H | CH₃ | 2,599 min | |
| | | | | | m/z=406,2 | |
| | | | | | [M+H]⁺ | |
| 71 | 2-C(O)OCH(CH₃)₂ | H | H | CH₃ | 3,271 min | |
| | | | | | m/z=407,2 | |
| | | | | | [M+H]⁺ | |
| 72 | 2-C(O)NH-CH₂CH=CH₂ | H | H | CH₃ | 2,537 min | |
| | | | | | m/z=404,1 | |
| | | | | | [M+H]⁺ | |
| 73 | 2-C(O)OCH₃ | H | H | CH₃ | 2,878 min | |
| | | | | | m/z=379,1 | |
| | | | | | [M+H]⁺ | |
| 74 | 2-COOCH₂CF₃ | H | H | CH₃ | 3,291 min | |
| | | | | | m/z=447,1 | |
| | | | | | [M+H]⁺ | |
| 75 | 2-C(O)N(CH₂CH₃)₂ | H | H | CH₃ | 2,752 min | |
| | | | | | m/z=420,2 | |
| | | | | | [M+H]⁺ | |
| 76 | 2-C(O)OCH₂(o-F- | H | H | CH₃ | 3,516 min | |
| | Ph) | | | | m/z=473,2 | |
| | | | | | [M+H]⁺ | |
| 77 | 2-C(O)NHCH₂-(o, o'-difluor-Ph) | H | H | CH₃ | 2,937 min | |
| | | | | | m/z=490,2 | |
| | | | | | [M+H]⁺ | |
| 78 | 2-C(O)NHOCH₃ | H | H | CH₃ | 2,305 min | |
| | | | | | m/z=394,1 | |
| | | | | | [M+H]⁺ | |
| 79 | 2-C(O)NH-SO₂-N(CH₃)(i-Pr) | H | H | CH₃ | 2,796 min | |
| | | | | | m/z=499,2 | |
| | | | | | [M+H]⁺ | |
| 80 | 2-C(O)O-(o-NO₂-Ph) | H | H | CH₃ | 3,335 min | |
| | | | | | m/z=486,2 | |
| | | | | | [M+H]⁺ | |
| 81 | 2-C(O)NH-SOr N(CH₃)(i-Pr) | H | H | CH₃ | 2,796 min | |
| | | | | | m/z=499,2 | |
| | | | | | [M+H]⁺ | |
| 82 | 2-C(O)NH-(m-Br-Ph) | H | H | CH₃ | 3,286 min | |
| | | | | | m/z=520,0 | |
| | | | | | [M+H]⁺ | |
| 83 | 2-C(O)NH₂ | H | H | CH₃ | 2,220 min | |
| | | | | | m/z=364,1 | |
| | | | | | [M+H]⁺ | |
| 84 | 2-C(O)NH-CH(CH₃)(CH₂-O-CH₃) | H | H | CH₃ | 2,520 min | |
| | | | | | m/z=436,2 | |
| | | | | | [M+H]⁺ | |
| 85 | 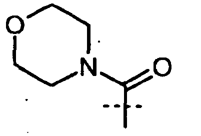 | H | H | CH₃ | 2,416 min | |
| | | | | | m/z=434,2 | |
| | | | | | [M+H]⁺ | |
| 86 | 2-C(O)O-CH₂C≡CH | H | H | CH₃ | 3,003 min | |
| | | | | | m/z=403,1 | |
| | | | | | [M+H]⁺ | |
| 87 | 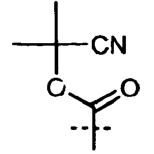 | H | H | CH₃ | 3,084 min | |
| | | | | | m/z=432,1 | |
| | | | | | [M+H]⁺ | |
| | | | | | | |
| | | | | | | |
| 88 | 2-Br | 4,5-(-O-CH₂-O-) | | CH₃ | 3,115 min | |
| | | | | | m/z= | |
| | | | | | 445,0 | |
| | | | | | [M+H]⁺ | |
| 89 | 2-Br | 4-F | H | CH₃ | 3,189 min | Isomer 1 |
| | | | | | m/z= | |
| | | | | | 417,0 | |
| | | | | | [M+H]⁺ | |
| 90 | 2-Br | 4-F | H | CH₃ | 3,197 min | Isomer 2 |
| | | | | | mlz= | |
| | | | | | 417,0 | |
| | | | | | [M+H]⁺ | |
| 91 | 2-CN | 4,5-(-O-CH₂-O-) | | CH₃ | 2,711 min | |
| | | | | | m/z= | |
| | | | | | 412,1 | |
| | | | | | [M+Na]⁺ | |
| 92 | 2-CN | 4-F | H | CH₃ | 2,848 min | |
| | | | | | m/z= | |
| | | | | | 386,1 | |
| | | | | | [M+Na]⁺ | |
| 93 | 2-Br | H | H | CH₃ | 3,086 min | |
| | | | | | m/z= | |
| | | | | | 399,0 | |
| | | | | | [M+H]⁺ | |
| 94 | 2-CN | 4-CH₃ | H | CH₃ | 2,940 min | |
| | | | | | m/z= | |
| | | | | | 360,1 | |
| | | | | | [M+H]⁺ | |
| 95 | 2-C(S)NH₂ | H | H | CH₃ | 2,634 min | |
| | | | | | m/z=380,1 | |
| | | | | | [M+H]⁺ | |
| 96 | 2-CN | H | H | CH₃ | 2,816 min | |
| | | | | | m/z= | |
| | | | | | 346,4 | |
| | | | | | [M+H]⁺ | |
| | | | | | 209 °C | |
| 97 | 2-OH | 5-NO₂ | H | CH₃ | 2,709 min | |
| | | | | | m/z= | |
| | | | | | 382,1 | |
| | | | | | [M+H]⁺ | |
| 98 | 2-CN | 5-NO₂ | H | CH₃ | 3,029 min | Isomer 1 |
| | | | | | m/z= | |
| | | | | | 391,1 | |
| | | | | | [M+H]⁺ | |
| 99 | 2-CN | 5-NO₂ | H | CH₃ | 2,982 min | Isomer 2 |
| | | | | | m/z= | |
| | | | | | 391,1 | |
| | | | | | [M+H]⁺ | |
| 100 | 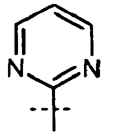 | H | H | CH₃ | 2,660 min | Isomer 1 |
| | | | | | m/z=399,5 | |
| | | | | | [M+H]⁺ | |
| 101 | 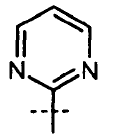 | H | H | CH₃ | 2,736 min | Isomer 2 |
| | | | | | m/z=399,5 | |
| | | | | | [M+H]⁺ | |
| 102 | 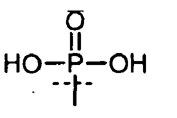 | H | H | CH₃ | 2,089 min | |
| | | | | | m/z=401,4 | |
| | | | | | [M+H]⁺ | |
| | | | | | 105°C | |
| 103 | 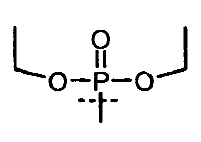 | H | H | CH₃ | 2,780 min | Isomer 1 |
| | | | | | m/z=457,5 | |
| | | | | | [M+H]⁺ | |
| | | | | | 115°C | |
| 104 | 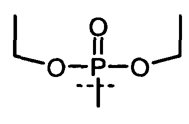 | H | H | CH₃ | 2,845 min | Isomer 2 |
| | | | | | m/z=457,5 | |
| | | | | | [M+H]⁺ | |
| 105 | 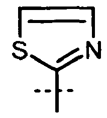 | H | H | CH₃ | 3,074 min | Isomer 1 |
| | | | | | m/z=404,1 | |
| | | | | | [M+H]⁺ | |
| 106 | 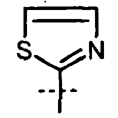 | H | H | CH₃ | 2,886 min | Isomer 2 |
| | | | | | m/z=404,1 | |
| | | | | | [M+H]⁺ | |
| 107 | 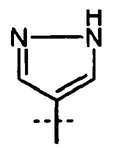 | H | H | CH₃ | 2,563 min | Isomer 1 |
| | | | | | m/z=387,5 | |
| | | | | | [M+H]⁺ | |
| 108 | 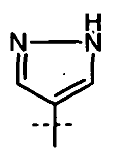 | H | H | CH₃ | 2,700 min | Isomer 2 |
| | | | | | m/z=387,5 | |
| | | | | | [M+H]⁺ | |
| 109 | 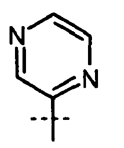 | H | H | CH₃ | 2,680 min | Isomer 1 |
| | | | | | m/z=399,1 | |
| | | | | | [M+H]⁺ | |
| 110 | 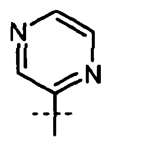 | H | H | CH₃ | 2,785 min | Isomer 2 |
| | | | | | m/z=399,1 | |
| | | | | | [M+H]⁺ | |
| 111 | 2-NO₂ | 6-Br | H | CH₃ | 3,289 min | Isomer 1 |
| | | | | | m/z=444,0 | |
| | | | | | [M+H]⁺ | |
| | | | | | 115 °C | |
| 112 | 2-NO₂ | 6-Br | H | CH₃ | 3,333 min | Isomer 2 |
| | | | | | m/z=444,0 | |
| | | | | | [M+H]⁺ | |
| | | | | | 130 °C | |
| 113 | 2-CN | 6-NH₂ | H | CH₃ | 2,839 min | |
| | | | | | m/z= | |
| | | | | | 361,1 | |
| | | | | | [M+H]⁺ | |
| | | | | | 193 °C | |
| 114 | 2-CN | 5-F | 6-OH | CH₃ | 2,716 min | |
| | | | | | m/z= | |
| | | | | | 380,1 | |
| | | | | | [M+H]⁺ | |
| | | | | | 205 °C | |
| 115 | 2-CN | 5-F | 6-F | CH₃ | 3,153 min | |
| | | | | | m/z= | |
| | | | | | 382,1 | |
| | | | | | [M+H]⁺ | |
| | | | | | 175 °C | |
| 116 | 2-CN | 6-CN | H | CH₃ | 2,903 min | Isomer 1 |
| | | | | | m/z= | |
| | | | | | 371,4 | |
| | | | | | [M+H]⁺ | |
| | | | | | 225 °C | |
| 117 | 2-CN | 6-CN | H | CH₃ | 2,790 min | Isomer 2 |
| | | | | | m/z= | |
| | | | | | 371,4 | |
| | | | | | [M+H]⁺ | |
| | | | | | 235 °C | |
| 118 | 2-CN | 6-Br | H | CH₃ | 3,122 min | |
| | | | | | m/z= | |
| | | | | | 424,4 | |
| | | | | | [M]⁺ | |
| | | | | | 175-180 | |
| | | | | | °C | |
| 119 | 2-Br | 5-F | 6-F | CH₃ | 3,488 min | |
| | | | | | m/z= | |
| | | | | | 435,0 | |
| | | | | | [M]⁺ | |
| 120 | 2-CN- | 6-CH=CH₂ | H | CH₃ | 3,143 min | Isomer 1 |
| | | | | | m/z= | |
| | | | | | 372,1 | |
| | | | | | [M+H]⁺ | |
| 121 | 2-CN | 6-CH=CH₂ | H | CH₃ | 3,261 min | Isomer 2 |
| | | | | | m/z= | |
| | | | | | 372,0 | |
| | | | | | [M+H]⁺ | |
| 122 | 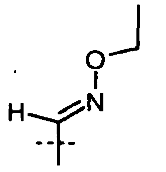 | H | H | CH₃ | 3,343 min | |
| | | | | | m/z=392,5 | |
| | | | | | [M+H]⁺ | |
| 123 | 2-NHC(O)CH₃ | 6-Br | H | CH₃ | 3,035 min | |
| | | | | | m/z=456,2 | |
| | | | | | [M+H]⁺ | |
| 124 | 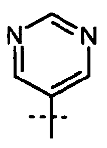 | H | H | CH₃ | 2,654 min | |
| | | | | | m/z=399,1 | |
| | | | | | [M+H]⁺ | |
| 125 | 2-CN | 6-NHC(O)CH₃ | H | CH₃ | 2,676 min | |
| | | | | | m/z=403,5 | |
| | | | | | [M+H]⁺ | |
| 126 | 2-CN | 6-NHC(O)NH(Ph) | H | CH₃ | 3,514 min | |
| | | | | | m/z=480,5 | |
| | | | | | [M+H]⁺ | |
| 127 | 2-CN | 6-N(CH₃)₂ | H | CH₃ | 3,171 min | |
| | | | | | m/z=389,5 | |
| | | | | | [M+H]⁺ | |
| | | | | | 58 °C | |
| 128 | 2-NH-SO₂-NHCH(CH₃)₂ | H | H | CH₃ | 3,129 min | |
| | | | | | m/z=482,0 | |
| | | | | | [M+H]⁺ | |
| | | | | | 103 °C | |
| 129 | 2-CH=CH₂ | H | H | CH₃ | 3,259 min | Isomer 1 |
| | | | | | m/z=347,1 | |
| | | | | | [M+H]⁺ | |
| 130 | 2-CH=CH₂ | H | H | CH₃ | 3,262 min | Isomer 2 |
| | | | | | m/z=347,1 | |
| | | | | | [M+H]⁺ | |
| 131 | 2-CN | 4-Cl | 5-Cl | CH₃ | 3,421 min | |
| | | | | | m/z=413,0 | |
| | | | | | [M]⁺ | |
| 132 | 2-Br | 5-Cl | 6-Cl | CH₃ | 3,744 min | |
| | | | | | m/z=468,8 | |
| | | | | | [M]⁺ | |
| 133 | 2-CN | 5-Cl | 6-Cl | CH₃ | 3,430 min | Isomer 1 |
| | | | | | m/z=413,9 | |
| | | | | | [M]⁺ | |
| 134 | 2-CN | 5-Cl | 6-Cl | CH₃ | 3,408 min | Isomer 2 |
| | | | | | m/z=414,0 | |
| | | | | | [M]⁺ | |
| 135 | 2-Phenyl | H | H | CH₃ | 3,534 min | |
| | | | | | m/z=397,5 | |
| | | | | | [M+H]⁺ | |
| 136 | 2-CN | H | H | CH₂- | 3,198 | |
| | | | | CH=CH₂ | m/z=372,5 | |
| | | | | | [M+H]⁺ | |
| 137 | 2-CN | H | H | C(O)- | 3,648 | |
| | | | | (C₆H₅) | m/z=436,5 | |
| | | | | | [M+H]⁺ | |
| 138 | 2-CN | H | H | CH₂-OH | 2,524 | |
| | | | | | m/z=362,4 | |
| | | | | | [M+H]⁺ | |
| 139 | 2-CN | H | H | CH₂- | 3,233 | |
| | | | | C(O)- | m/z=403,7 | |
| | | | | OCH₃ | [M+H]⁺ | |
| 140 | 2-CN | H | H | C(O)- | 3,322 | |
| | | | | CH₃ | m/z=374,0 | |
| | | | | | [M+H]⁺ | |
| 141 | 2-SO₂CH₃ | H | H | CH₃ | m/z=399,4 | Isomer 1 |
| | | | | | [M+H]⁺ | |
| 142 | 2-SO₂CH₃ | H | H | CH₃ | m/z=399,2 | Isomer 2 |
| | | | | | [M+H]⁺ | |
| 143 | 2-NO₂ | 6-CH=CH₂ | H | CH₃ | m/z=392,3 | Isomer 1 |
| | | | | | [M+H]⁺ | |
| 144 | 2-NO₂ | 6-CH=CH₂ | H | CH₃ | m/z=392,3 | Isomer 2 |
| | | | | | [M+H]⁺ | |
| 145 | 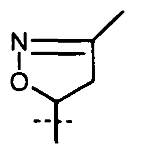 | H | H | CH₃ | m/z=404,4 | |
| | | | | | [M+H]⁺ | |
| 146 | 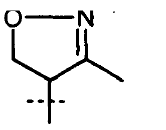 | H | H | CH₃ | m/z=404,4 | |
| | | | | | [M+H]⁺ | |
| 147 | 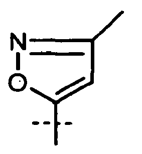 | H | H | CH₃ | m/z=402,4 | Isomer 1 |
| | | | | | [M+H]⁺ | |
| | | | | | 98 °C | |
| 148 | 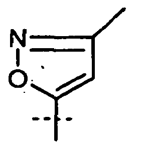 | H | H | CH₃ | m/z=402,4 | Isomer 2 |
| | | | | | [M+H]⁺ | |
| | | | | | 112 °C | |
| 149 | 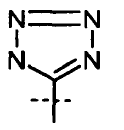 | H | H | CH₃ | m/z=389,4 | Isomer 1 |
| | | | | | [M+H]⁺ | |
| 150 | 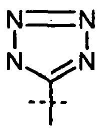 | H | H | CH₃ | m/z=389,4 | Isomer 2 |
| | | | | | [M+H]⁺ | |
| 151 | 2-CN | 6-CF₃ | H | CH₃ | 3,212 | Isomer 1 |
| | | | | | m/z=414,5 | |
| | | | | | [M]⁺ | |
| 152 | 2-CN | 6-CF₃ | H | CH₃ | 3,243 | Isomer 2 |
| | | | | | m/z=414,5 | |
| | | | | | [M]⁺ | |
| 153 | 2-Br | 6-CF₃ | H | CH₃ | 3,522 | |
| | | | | | m/z=469,4 | |
| | | | | | [M+H]⁺ | |
| 154 | 2-CN | 5-Cl | 6-Cl | H | 3,131 min | |
| | | | | | m/z=422,0 | |
| | | | | | [M+Na]⁺ | |
| 155 | 2-Br | 5-Cl | 6-Cl | C(O)- | 4,146 min | |
| | | | | CH₃ | m/z=518,8 | |
| | | | | | [M+Na]⁺ | |
| 156 | 2-Br | 5-Cl | 6-Cl | H | 3,484 min | |
| | | | | | m/z=454,8 | |
| | | | | | [M+H]⁺ | |
| 157 | 2-CN | 6-Cl | H | CH₃ | 3,070 | Isomer 1 |
| | | | | | m/z=380,4 | |
| | | | | | [M+H]⁺ | |
| 158 | 2-CN | 6-Cl | H | CH₃ | 3,117 | Isomer 2 |
| | | | | | m/z=380,4 | |
| | | | | | [M+H]⁺ | |

| | | | | | | |
|---|---|---|---|---|---|---|
| - Bsp. Nr.: Beispiel Nummer - m.p.: Schmelzpunkt - Ph: Phenyl - i-Pr: Isopropyl | | | | | | |

**Tabelle 3:**

| | | | | | | | |
|---|---|---|---|---|---|---|---|
| 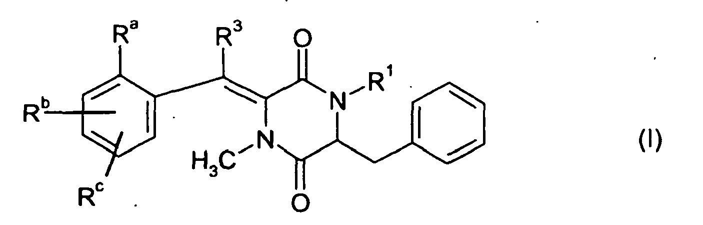 | | | | | | | |

| Bsp. Nr. | R^{a} | R^{b} | R^{c} | R³ | R¹ | RT HPLC/MS oder m.p. | Isomer |
|---|---|---|---|---|---|---|---|
| 159 | 2-NO₂ | H | H | Br | CH₃ | 3,306 min | |
| | | | | | | m/z= 446,0 | |
| | | | | | | [M+H]⁺ | |
| 160 | 2-CN | H | H | Br | CH₃ | 3,113 min | |
| | | | | | | m/z= 424,4 | |
| | | | | | | [M]⁺ | |
| 161 | 2-NO₂ | H | H | CH=CH₂ | CH₃ | 3,172 min | |
| | | | | | | m/z= 392,1 | |
| | | | | | | [M+H]⁺ | |
| 162 | 2-NO₂ | H | H | CH(CH₃)₂ | CH₃ | 3,369 min | Isomer 1 |
| | | | | | | m/z= 408,2 | |
| | | | | | | [M+H]⁺ | |
| 163 | 2-NO₂ | H | H | CH(CH₃)₂ | CH₃ | 3,096 min | Isomer 2 |
| | | | | | | m/z= 408,1 | |
| | | | | | | [M+H]⁺ | |
| 164 | 2-Br | H | H | CN | CH₃ | 3,180 min | |
| | | | | | | m/z= 424,4 | |
| | | | | | | [M]⁺ | |
| 165 | 2-Br | H | H | Br | CH₃ | 3,578 min | Isomer 1 |
| | | | | | | m/z= 478,9 | |
| | | | | | | [M+H]⁺ | |
| 166 | 2-Br | H | H | Br | CH₃ | 3,643 min | Isomer 2 |
| | | | | | | m/z= 478,9 | |
| | | | | | | [M+H]⁺ | |

| | | | | | | | |
|---|---|---|---|---|---|---|---|
| - Bsp Nr.: Beispiel Nummer - m.p.: Schmelzpunkt | | | | | | | |

**Tabelle 4:**

| | | | | | | |
|---|---|---|---|---|---|---|
| 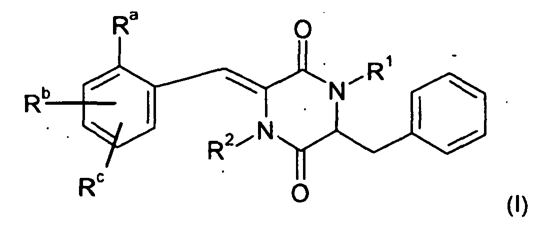 | | | | | | |

| Bsp. Nr. | R^{a} | R^{b} | R^{c} | R² | R¹ | RT HPLC/MS oder m.p. |
|---|---|---|---|---|---|---|
| 167 | 2-NO₂ | H | H | CH₂CH₃ | C(O)CH₃ | 3,578 min |
| | | | | | | m/z= 408,1 |
| | | | | | | [M+H]⁺ |
| 168 | 2-Br | H | H | C(O)CH₃ | C(O)CH₃ | 3,812 min |
| | | | | | | m/z= 456,9 |
| | | | | | | [M+H]⁺ |
| 169 | 2-Br | H | H | CH₂-(C₆H₅) | CH₂-(C₆H₅) | 4,385 min |
| | | | | | | m/z= 552,9 |
| | | | | | | [M+H]⁺ |
| 170 | 2-Br | H | H | CH₂-CH₃ | CH₂-CH₃ | 3,637 min |
| | | | | | | m/z= 428,9 |
| | | | | | | [M+H]⁺ |
| 171 | 2-Br | H | H | CH₂-CH=CH₂ | CH₂-CH=CH₂ | 3,879 min |
| | | | | | | m/z= 453,0 |
| | | | | | | [M+H]⁺ |
| 172 | 2-CN | H | H | CH₂-(C₆H₅) | CH₂-(C₆H₅) | 3,995 min |
| | | | | | | m/z= 498,1 |
| | | | | | | [M+H]⁺ |
| 173 | 2-CN | H | H | CH₂-CH₃ | CH₂-CH₃ | 3,223 min |
| | | | | | | m/z= 374,1 |
| | | | | | | [M+H]⁺ |
| 174 | 2-CN | H | H | CH₂-CH=CH₂ | CH₂-CH=CH₂ | 3,469 min |
| | | | | | | m/z= 398,1 |
| | | | | | | [M+H]⁺ |
| 175 | 2-Br | H | H | CH₂-(C₆H₅) | C(O)CH₃ | 4,235 min |
| | | | | | | m/z= 504,9 |
| | | | | | | [M+H]⁺ |
| | | | | | | 98 °C |
| 176 | 2-Br | H | H | CH₂-CH=CH₂ | C(O)CH₃ | 4,010 min |
| | | | | | | m/z= 454,9 |
| | | | | | | [M+H]⁺ |
| 177 | 2-Br | H | H | CH₂-CH₃ | C(O)CH₃ | 3,921 min |
| | | | | | | m/z= 441,4 |
| | | | | | | [M+H]⁺ |
| 178 | 2-Br | H | H | CH₂-(C₆H₅) | H | 3,620 min |
| | | | | | | m/z= 463,4 |
| | | | | | | [M+H]⁺ |
| 179 | 2-Br | H | H | CH₂-CH=CH₂ | H | 3,322 min |
| | | | | | | m/z= 410,9 |
| | | | | | | [M+H]⁺ |
| | | | | | | 78 °C |
| 180 | 2-Br | H | H | CH₂-CH₃ | H | 3,216 min |
| | | | | | | m/z= 401,0 |
| | | | | | | [M+H]⁺ |
| 181 | 2-CN | H | H | CH₂-(C₆H₅) | H | 3,218 min |
| | | | | | | m/z= 408,0 |
| | | | | | | [M+H]⁺ |
| 182 | 2-CN | H | H | CH₂-CH=CH₂ | H | 2,929 min |
| | | | | | | m/z= 358,4 |
| | | | | | | [M+H]⁺ |
| 183 | 2-CN | H | H | CH₂-CH₃ | H | 2,830 min |
| | | | | | | m/z= 346,4 |
| | | | | | | [M+H]⁺ |

| | | | | | | |
|---|---|---|---|---|---|---|
| - Bsp. Nr.: Beispiel Nummer - m.p.: Schmelzpunkt | | | | | | |

**Tabelle 5:**

| Bsp. Nr. | Name | RT HPLC/MS oder m.p. | Isomer |
|---|---|---|---|
| 184 | 3-Benzyl-1,4-dimethyl-6-(4-nitro-1H-indol-3-ylmethylen)-piperazin-2,5-dion | 2,743 min, | Isomer 1 |
| | | m/z= 405,2 [M+H]⁺ | |
| | | 159 °C | |
| 185 | 3-Benzyl-1,4-dimethyl-6-(4-nitro-1H-indol-3-ylmethylen)-piperazin-2,5-dion | 2,608 min, | Isomer 2 |
| | | m/z= 405,1 [M+H]⁺ | |
| | | 238 °C | |
| 186 | 3-(5-Benzyl-1,4-dimethyl-3,6-dioxo-piperazin-2-ylidenmethyl)-4-nitro-indol-1-carbonsäure tert-butyl ester | 3,776 min, | Isomer 1 |
| | | m/z= 505,0 [M+H]⁺ | |
| 187 | 3-(5-Benzyl-1,4-dimethyl-3,6-dioxo-piperazin-2-ylidenmethyl)-4-nitro-indol-1-carbonsäure-tert-butyl ester | 4,243 min, | Isomer 2 |
| | | m/z= 505,5 [M+H]⁺ | |
| 188 | 1-Methyl-6-(2-nitrobenzyliden)-3-thiophen-2-ylmethyl-piperazin-2,5-dion | 2,579 min, | Isomer 1 |
| | | m/z= 357,9 [M+H]⁺ | |
| 189 | 1-Methyl-6-(2-nitrobenzyliden)-3-thiophen-2-ylmethyl-piperazin-2,5-dion | 2,622 min, | Isomer 2 |
| | | m/z= 358,0 [M+H]⁺ | |
| 190 | 3-Benzyl-6-(5-chlor-1-methyl-3-trifluormethyl-1H-pyrazol-4-ylmethylen)-1,4-dimethyl-piperazin-2,5-dion | 3,108 min, | |
| | | m/z= 427,1 [M+H]⁺ | |
| 191 | 3-Benzyl-1,4-dimethyl-6-(2-nitro-thiophen-3-ylmethylen)-piperazin-2,5-dion | 2,730 min, | Isomer 1 |
| | | m/z= 372,1 [M+H]⁺ | |
| 192 | 3-Benzyl-1,4-dimethyl-6-(2-nitro-thiophen-3-ylmethylen)-piperazin-2,5-dion | 2,784 min, | Isomer 2 |
| | | m/z= 372,1 [M+H]⁺ | |
| 193 | 3-Benzyl-1,4-dimethyl-6-(3-nitro-thiophen-2-ylmethylen)-piperazin-2,5-dion | 2,756 min, | Isomer 1 |
| | | m/z= 372,0 [M+H]⁺ | |
| 194 | 3-Benzyl-1,4-dimethyl-6-(3-nitro-thiophen-2-ylmethylen)-piperazin-2,5-dion | 2,899 min, | Isomer 2 |
| | | m/z= 372,1 [M+H]⁺ | |
| 195 | 1,4-Dimethyl-3-(2-nitro-benzyliden)-6-thiophen-2-ylmethyl-piperazin-2,5-dion | 2,884 min, | |
| | | m/z= 372,1 [M+H]⁺ | |
| 196 | 3-Benzyl-1,4-dimethyl-6-(3-nitro-pyridin-4-ylmethylen)-piperazin-2,5-dion | 2,425 min, | Isomer 1 |
| | | m/z= 367,1 [M+H]⁺ | |
| | | 180 °C | |
| 197 | 3-Benzyl-1,4-dimethyl-6-(3-nitro-pyridin-4-ylmethylen)-piperazin-2,5-dion | 2,418 min, | Isomer 2 |
| | | m/z= 367,1 [M+H]⁺ | |
| | | 175 °C | |
| 198 | 3-Benzyl-1,4-dimethyl-6-(3-nitro-pyridin-2-ylmethylen)-piperazin-2,5-dion | 2,606 min, | |
| | | m/z= 367,1 [M+H]⁺ | |
| | | 165°C | |
| 199 | 3-Benzyl-1,4-dimethyl-6-(4-brom-1H-indol-3-ylmethylen)-piperazin-2,5-dion | 2,845 min, | |
| | | m/z= 440,0 [M+H]⁺ | |
| | | 236 °C | |
| 200 | 3-Benzyl-1,4-dimethyl-6-(4-cyano-1H-indol-3-ylmethylen)-piperazin-2,5-dion | 2,553 min, | Isomer 1 |
| | | m/z= 385,2 [M+H]⁺ | |
| 201 | 3-Benzyl-1,4-dimethyl-6-(4-cyano-1H-indol-3-ylmethylen)-piperazin-2,5-dion | 2,553 min, | Isomer 2 |
| | | m/z= 385,2 [M+H]⁺ | |
| 202 | 3-Benzyl-1,4-dimethyl-6-(4-trimethylsilanylethinyl-1H-indol-3-ylmethylen)piperazin-2,5-dion | 3,887 min, | |
| | | m/z= 456,1 [M+H]⁺ | |
| 203 | 2-(1,4-Dimethyl-3,6-dioxo-5-thiophen-2-ylmethyl-piperazin-2-ylidenmethyl)-benzonitril | 2,743 min, | |
| | | m/z= 352,1 [M+H]⁺ | |
| 204 | 3-Benzyl-6-(2-brom-pyridin-3-ylmethylen)-1,4-dimethyl-piperazin-2,5-dion | 2,615 min, | |
| | | m/z= 400,0 [M]⁺ | |
| 205 | 3-Benzyl-6-(2-cyano-pyridin-3-ylmethylen)-1,4-dimethyl-piperazin-2,5-dion | 2,485 min, | |
| | | m/z= 347,4 [M]⁺ | |
| | | 218 °C | |
| 206 | 3-Benzyl-6-(4-iod-pyridin-3-ylmethylen)-1,4-dimethyl-piperazin-2,5-dion | 3,029 min, | |
| | | m/z= 445,9 [M+H]⁺ | |
| 207 | 2-[5-(4-Fluor-benzyl)-1,4-dimethyl-3,6-dioxo-piperazin-2-ylidenmethyl]-benzonitril | 2,843 min, | |
| | | m/z= 364,0 [M+H]⁺ | |
| 208 | 3-Benzyl-6-(5-brom-3H-imidazol-4-ylmethylen)-1,4-dimethyl-piperazin-2,5-dion | 200 °C | Isomer 1 |
| 209 | 3-Benzyl-6-(5-brom-3H-imidazol-4-ylmethylen)-1,4-dimethyl-piperazin-2,5-dion | m/z= 389,1 [M]⁺ | Isomer 2 |
| 210 | 3-Benzyl-6-(5-cyano-3H-imidazol-4-ylmethylen)-1,4-dimethyl-piperazin-2,5-dion | 170 °C | |
| 211 | 3-Benzyl-6-(5-cyano-3H-imidazol-4-ylmethylen)-1,4-dimethyl-piperazin-2,5-dion | m/z= 336,2 [M+H]⁺ | Isomer 2 |
| 212 | 3-Benzyl-6-(5-brom-3H-[1,2,3]triazol-4-ylmethylen)-1,4-dimethyl-piperazin-2,5-dion | m/z= 390,0 [M]⁺ | Isomer |
| | | | 1 |
| 213 | 3-Benzyl-6-(5-brom-3H-[1,2,3]triazol-4-ylmethylen)-1,4-dimethyl-piperazin-2,5-dion | m/z= 392,0 [M]⁺ | Isomer 2 |
| 214 | 3-(2-Iodbenzyliden)-1,4-dimethyl-6-(thiophen-2-ylmethyl)-piperazin-2,5-dion | 3,303 min | |
| | | m/z 453,0 [M+H]⁺ | |

| | | | |
|---|---|---|---|
| - Bsp. Nr.: Beispiel Nummer - m.p.: Schmelzpunkt | | | |

### Teil B: Anwendungsbeispiele

Die herbizide Wirkung der Verbindungen der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen, wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.
Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine gute herbizide Aktivität ist bei Werten von wenigstens 70 und eine sehr gute herbizide Aktivität ist bei Werten von wenigstens 85 gegeben.

Die in den Gewächshausversuchen verwendeten Pflanzen setzten sich aus folgenden Arten zusammen:

| Lateinischer Name | Deutscher Name | Englischer Name |
|---|---|---|
| Lulium perenne (LOLMU) | Italienisches Raygras | annual ryegrass |
| Amaranthus retroflexus (AMARE) | Fuchsschwanz | pig weed |
| Abutilon theophrasti (ABUTH) | Chinesischer Hanf | velvet leaf |
| Apera spica-venti (APESV) | Gemeiner Windhalm | windgrass |
| Avena fatua (AVEFA) | Flughafer | wild oat |
| Echinochloa crus galli (ECHCG) | Hühnerhirse | barnyard grass |
| Setaria faberi (SETFA) | Fabers Borstenhirse | giant foxtail |
| Setaria viridis (SETVI) | Grüne Borstenhirse | green foxtail |

Die Verbindungen der Beispiele 1, 13, 16, 44, 47, 48, 49, 51, 52, 129, 132, 160 und 170 zeigen gute bis sehr gute herbizide Wirkung im Nachauflaufverfahren.

Die Verbindungen der Beispiele 44 und 160 zeigen bei einer Aufwandmenge von 3 kg/ha im Nachauflauf gegen ABUTH eine gute herbizide Wirkung.

Die Verbindungen der Beispiele 1, 16 und 129 zeigen bei einer Aufwandmenge von 1 kg/ha im Nachaulflauf gegen AMARE eine sehr gute herbizide Wirkung. Die Verbindung des Beispiels 13 zeigt bei einer Aufwandmenge von 0,5 kg/ha im Nachauflauf gegen AMARE eine sehr gute herbizide Wirkung.

Die Verbindung des Beispiels 132 zeigt bei einer Aufwandmenge von 3 kg/ha im Nachauflauf gegen AVEFA eine sehr gute herbizide Wirkung.

Die Verbindung des Beispiels 1 zeigt bei einer Aufwandmenge von 1 kg/ha im Nachauflauf gegen LOLMU eine sehr gute herbizide Wirkung.

Die Verbindungen der Beispiele 44, 47/48 (Gemisch), 49, 51 und 160 zeigen bei einer Aufwandmenge von 3 kg/ha im Nachauflauf eine sehr gute herbizide Wirkung und die Verbindungen der Beispiele 52 und 132 eine gute herbizide Wirkung gegen SETFA. Die Verbindung des Beispiels 170 zeigt bei einer Aufwandmenge von 1 kg/ha im Nachauflauf gegen SETFA eine gute herbizide Wirkung.

Die Verbindungen der Beispiele 38, 40, 45, 54, 62, 73, 100, 101, 109, 110, 119, 124, 137, 140, 147, 148, 191, 198, 204, 206, 214 gute bis sehr gute herbizide Wirkung im Vorauflaufverfahren.

Die Verbindung des Beispiels 73 zeigt bei einer Aufwandmenge von 3 kg/ha im Vorauflauf gegen ABUTH eine gute herbizide Wirkung.

Die Verbindung des Beispiels 137 zeigt bei einer Aufwandmenge von 1 kg/ha im Vorauflauf gegen AMARE eine gute herbizide Wirkung.

Die Verbindung des Beispiels 206 zeigt bei einer Aufwandmenge von 1 kg/ha im Vorauflauf gegen AVEFA eine gute herbizide Wirkung.

Die Verbindungen der Beispiele 38, 45, 54, 124, 140, 147 und 198 zeigen bei einer Aufwandmenge von 1 kg/ha im Vorauflauf eine sehr gute herbizide Wirkung gegen APSEV. Die Verbindungen der Beispiele 119 und 191 zeigen bei einer Aufwandmenge von 0,5 kg/ha im Vorauflauf eine gute herbizide Wirkung gegen APSEV.

Die Verbindungen der Beispiele 38, 45, 100/101 (Gemisch) und 109/110 (Gemisch) zeigen bei einer Aufwandmenge von 1 kg/ha im Vorauflauf eine sehr gute herbizide Wirkung und die Verbindung 147 eine gute herbizide Wirkung gegen ECHCG. Die Verbindungen der Beispiele 40 und 204 zeigen bei einer Aufwandmenge von 3 kg/ha im Vorauflauf eine sehr gute herbizide Wirkung und die Verbindung des Beispiels 214 eine gute herbizide Wirkung gegen ECHCG.

Die Verbindungen der Beispiele 109/110 (Gemisch) und 140 zeigen bei einer Aufwandmenge von 1 kg/ha im Vorauflauf eine sehr gute herbizide Wirkung gegen SETFA. Die Verbindung des Beispiels 62 bei einer Aufwandmenge von 3 kg/ha und die Verbindung des Beispiels 206 bei einer Aufwandmenge von 2 kg/ha zeigen jeweils eine gute herbizide Wirkung gegen SETFA.

Die Verbindungen der Beispiele 100/101 (Gemisch) zeigen bei einer Aufwandmenge von 1 kg/ha im Vorauflauf eine sehr gute herbizide Wirkung gegen SETIT. Die Verbindungen der Beispiele 40 und 204 zeigen bei einer Aufwandmenge von 3 kg/ha im Vorauflauf eine sehr gute herbizide Wirkung gegen SETIT. Die Verbindung des Beispiels 214 zeigt bei einer Aufwandmenge von 3 kg/ha im Vorauflauf gegen SETIT eine gute herbizide Wirkung.

## Patentansprüche

1. Verwendung von Piperazinverbindungen der Formel I 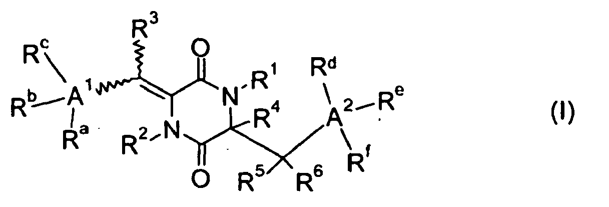 oder der landwirtschaftlich brauchbaren Salze von Piperazinverbindungen der Formel I als Herbizide, wobei in Formel I die Variablen die folgenden Bedeutungen haben:
R¹ und R² unabhängig voneinander
Cyano, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocyclyl-(C₁-C₆)-alkyl; oder
COR²¹, wobei
R²¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet; oder
NR²²R²³, wobei
R²² und R²³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl oder C₁-C₆-Alkylcarbonyl bedeuten; oder
OR²⁴, wobei
R²⁴ C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl oder Phenyl-(C₁-C₆)-Alkyl bedeutet; oder
SO₂R²⁵, wobei
R²⁵ C₁-C₆-Alkyl oder Phenyl bedeutet;
wobei die genannten aliphatischen, cyclischen oder aromatischen Teile der Substituenten von R¹ und R² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl , Aminocarbonyl, C₁-C₄-Alkylaminocarbonyl, [Di-(C₁-C₄)-alkyl]aminocarbonyl oder C₁-C₄-Alkylcarbonyloxy;
und wobei R¹ zusätzlich Wasserstoff bedeuten kann;
R³ Wasserstoff, Halogen, Cyano, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Phenyl, Phenyl-(C₁-C₆)-Alkyl, Heterocyclyl, Heterocyclyl-(C₁-C₆)-alkyl; Phenyl-[C₁-C₆-alkoxycarbonyl]-(C₁-C₆)-alkyl oder Phenylheterocyclyl-(C₁-C₆)-alkyl; oder ein Rest COR²⁶, NR²⁷R²⁸, OR²⁹, SO₂R³⁰ oder N(OR³¹)R³², wobei
R²⁶ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet;
R²⁷ und R²⁸ unabhängig voneinander Wasserstoff, C₁-C₆-Alkyl, Aryl oder Heteroaryl bedeuten;
R²⁹ C₁-C₆-Alkyl bedeutet;
R³⁰ C₁-C₆-Alkyl oder Phenyl bedeutet;
R³¹ Wasserstoff, C₁-C6-Alkyl, Phenyl oder Phenyl-(C₁-C₆)-alkyl bedeutet;
R³² C₁-C₆-Alkyl, Phenyl oder Phenyl-(C₁-C₆)-alkyl bedeutet;
wobei die genannten aliphatischen, cyclischen oder aromatischen Teile der Substituenten von R³ bzw. R26, R²⁷, R²⁸, R²⁹, R³⁰, R³¹ und R³² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxcarbonyl, Aminocarbonyl, C₁-C₄-Alkylaminocarbonyl, [Di-(C₁-C₄)-alkyl]aminocarbonyl oder C₁-C₄-Alkylcarbonyloxy;
R⁴, R⁵, R⁶ unabhängig voneinander Wasserstoff, Hydroxy, C₁-C₆-Alkyl, C₁-C₆-Alkoxy, wobei die genannten aliphatischen Teile der Substituenten von R⁴, R⁵ oder R⁶ partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
A¹ Aryl oder Heteroaryl;
A² Aryl oder Heteroaryl, ausgenommen Indolyl;
R^{a} Halogen, Cyano, Nitro, C₁-C₆-Alkyl, C₃-C₆-Cycloalkyl, C₂-C₆-Alkenyl, C₃-C₆-Cycloalkenyl, C₄-C₁₀-Alkadienyl, C₂-C₆-Alkinyl, [Tri-(C₁-C₆)-alkylsilyl]-(C₂-C₆)-alkinyl, C₃-C₆-Cycloalkinyl, C₁-C₆-Alkylthio, C₁-C₆-Alkylsulfinyl, Aryl, Phenyl-(C₁-C₆)-Alkyl, Phenyl-(C₂-C₆)-Alkenyl, Phenylsulfonyl-(C₁-C₆)-Alkyl, Heterocyclyl, Heterocyclyl-(C₁-C₆)-Alkyl oder Phenyl-[C₁-C₆-alkoxycarbonyl]-(C₁-C₆)-alkyl,
Z¹P(O)(OR⁹)₂, Z²B(OR¹⁰)₂, wobei
R⁹ und R¹⁰ jeweils Wasserstoff oder C₁-C₆-Alkyl bedeuten und R¹⁰ in Z²B(OR¹⁰)₂ zusammen eine C₂-C₄-Alkylenkette bilden können; oder
Z³COR¹¹, wobei
R¹¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino. [Di-(C₁-C₆)-Alkoxy]amino, C₁-C₆-Alkylsulfonylamino, C₁-C₆-Alkylaminosulfonylamino, [Di-(C₁-C₆)-alkylamino)sulfonylamino, C₃-C₆-Alkenylamino, C₃-C₆-Alkinylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenoxy, Phenylamino, Naphthyl oder Heterocyclyl bedeutet; oder
Z⁴NR¹²R¹³, wobei
R¹² und R¹³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₃-C₆-Cycloalkylcarbonyl, [Di-(C₁-C₆)-alkylamino]carbonyl, C₁-C₆-Alkoxycarbonyl, C₁-C₆-Alkoxycarbonyl-(C₁-C₆)-alkyl, C₁-C₆-Alkylsulfonyl, C₁-C₆-Alkylaminosulfonyl, [Di,(C₁-C₆)-alkylamino]sulfonyl, Phenylcarbonyl, Phenylaminocarbonyl, Phenylsulfonyl, Phenylsulfonylaminocarbonyl der Heterocyclylcarbonyl bedeuten; oder
Z⁵CH=N-O-R¹⁴, wobei R¹⁴ Wasserstoff oder C₁-C₆-Alkyl bedeutet; oder
Z⁶OR¹⁵, wobei
R¹⁵ Wasserstoff, C₁-C₆-Alkyl, C₃-C₆-Cycloalkyl, C₃-C₆-Alkenyl , C₃-C₆-Cycloalkenyl, C₃-C₆-Alkinyl, C₃-C₆-Cycloalkinyl, C₁-C₆-Alkylcarbonyl, C₁-C₆-Alkoxycarbonyl-(C₁-C₆)-alkyl, [Di-(C₁-C₆)-Alkoxycarbonyl]-(C₁-C₆)-alkyl, Phenyl oder Phenyl-(C₁-C₆)-Alkyl bedeutet; oder
Z⁷SO₂R¹⁶, wobei R¹⁶ C₁-C₆-Alkyl oder Phenyl bedeutet; und wobei
Z¹, Z², Z³, Z⁴, Z⁵, Z⁶, Z⁷ unabhängig voneinander eine Bindung, -CH₂-, -CH₂-CH₂-, -O-CH(R17)-, -S-CH(R¹⁸)-, -S(O)-CH(R¹⁹)- oder -SO₂CH(R²⁰)-bedeutet, und wobei R¹⁷, R¹⁸, R¹⁹ und R²⁰ unabhängig voneinander Wasserstoff oder C₁-C₆-Alkyl bedeuten; und
wobei die genannten aliphatischen, cyclischen oder aromatischen Teile des Substituenten R^{a} partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, [Di-(C₁-C₄)-alkyl]amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁₋C₄-Alkylcarbonyloxy; und
R^{b}, R^{c}, R^{d}, R^{e} und R^{f} jeweils unabhängig voneinander für Wasserstoff stehen oder eine der für R^{a} angegebenen Bedeutungen aufweisen; und
worin zwei an benachbarte Ringatome von A¹ gebundene Reste R^{a}, R^{b} oder R^{c} oder zwei an benachbarte Ringatome von A² gebundene Reste R^{d}, R^{e} oder R^{f} auch für lineares C₃-C₆-Alkylen stehen können, das teilweise oder vollständig halogeniert sein kann und das eine bis drei der folgenden Gruppen tragen kann: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy, worin eine CH₂-Gruppe in C₃-C₆-Alkylen durch eine Carbonylgruppe, Thiocarbonylgruppe oder Sulfonylgruppe ersetzt sein kann und worin eine oder zwei nicht benachbarte CH₂-Gruppen in C₃-C₆-Alkylen jeweils durch Sauerstoff, Schwefel oder eine Gruppe NR³⁴ ersetzt sein können, wobei R³⁴ eine für R¹² angegebenen Bedeutungen aufweist.

2. Verwendung von Piperazinverbindungen der Formel I nach Anspruch 1, worin R^{a} in der ortho-Postion zur Verknüpfungsstelle von A¹ gebunden ist.

3. Verwendung von Piperazinverbindungen der Formel I nach Anspruch 1, worin A¹ bicyclisches Aryl oder Hetaryl ist.

4. Verwendung von Piperazinverbindungen der Formel I nach einem der vorhergehenden Ansprüche, worin A¹ und A² unabhängig voneinander ausgewählt sind aus der Gruppe Phenyl, Naphthyl, Furyl, Thienyl, Pyrrolyl, Pyrazolyl, Thiazolyl, Isothiazolyl, Oxazolyl, Isoxazolyl, Triazolyl, Tetrazolyl, Pyridinyl, Pyridazinyl, Pyrimidinyl, Pyrazinyl, Triazinyl und Tetrazinyl.

5. Verwendung von Piperazinverbindungen der Formel I nach einem der Ansprüche 1, 2 oder 4, worin A¹ und A² unabhängig voneinander ausgewählt sind aus der Gruppe Phenyl, Furyl, Thienyl, Triazolyl, Tetrazolyl und Pyridinyl.

6. Verwendung von Piperazinverbindungen der Formel I nach einem der Ansprüche 1, 2, 4 oder 5, worin A¹ für Phenyl oder Pyridinyl steht.

7. Verwendung von Piperazinverbindungen der Formel I nach einem der vorhergehenden Ansprüche, worin A² für Phenyl oder Thienyl steht.

8. Verwendung von Piperazinverbindungen der Formel I mach nach einem der vorhergehenden Ansprüche, worin:
R^{a} ausgewählt ist unter Halogen, Cyano, Nitro, C₁-C₆-Alkyl, C₂-C₆-Alkenyl, C₂-C₆-Alkinyl, C₃-C₆-Cycloalkyl, [Tri-(-C₁-C₆-)alkylsilyl]-C₂-C₆-alkinyl, Z¹P(O)(OR⁹)₂, Z³COR¹, Z⁴NR¹²R¹³, Z⁵CH=N-O-R¹⁴, Z⁶OR¹⁵, Z⁷SO₂R¹⁶, C₁-C₆-Alkylthio, C₁-C₆-Alkylsulfinyl, Aryl und Heterocyclyl, wobei
Z¹ eine Bindung oder CH₂ bedeutet und R⁹ und R¹⁰ jeweils Wasserstoff oder C₁-C₆-Alkyl bedeuten;
Z³ eine Bindung bedeutet und R¹¹ Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, (C₁-C₆)-Alkoxy-(C₁-C₆)-Alkylamino, C₁-C₆-Alkylsulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl bedeutet; oder
Z⁴ eine Bindung oder CH₂ bedeutet und R¹² und R¹³ unabhängig voneinander Wasserstoff, C₁-C₆-Alkyl, C₃-C₆-Cycloalkyl, C₃-C₆-Alkenyl, C₃-C₆-Alkinyl, C₁-C₆-Alkylcarbonyl, [Di-(C₁-C₆)-alkylamino]carbonyl, C₁-C₆-Alkoxycarbonyl, C₁-C₆-Alkylsulfonyl, Phenylcarbonyl, Phenylsulfonyl, oder Heterocyclylcarbonyl bedeuten; oder
Z⁵ eine Bindung bedeutet und R¹⁴ Wasserstoff oder C₁-C₆-Alkyl bedeutet; oder
Z⁶ eine Bindung oder CH₂ bedeutet und R¹⁵ Wasserstoff, C₁-C₆-Alkyl, C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Phenyl oder Phenyl-(C₁-C₆)-Alkyl bedeutet; oder
Z⁷ eine Bindung bedeutet und R¹⁶ C₁-C₆-Alkyl oder Phenyl;
R^{b}, R^{c}, R^{d}, R^{e} und R^{f} unabhängig voneinander für Wasserstoff stehen oder eine der für R^{a} genannten Bedeutungen haben,
und wobei die genannten aliphatischen, cyclischen oder aromatischen Teile der Substituenten R^{a}, R^{b}, R^{e}, R^{d}, R^{e} und R^{f} partiell oder vollständig halogeniert sein können.

9. Verwendung von Piperazinverbindungen der Formel I mach einem der vorhergehenden Ansprüche, worin
R¹ Wasserstoff oder C₁-C₆-Alkyl; und
R² C₁-C₆-Alkyl;
bedeuten, wobei C₁-C₆-Alkyl in R¹ und R² partiell oder vollständig halogeniert sein kann.

10. Verwendung von Piperazinverbindungen der Formel I nach einem der vorhergehenden Ansprüche, worin R³ Wasserstoff, Halogen, C₁-C₆-Alkyl oder Halo-C₁-C₆-alkyl bedeutet.

11. Verwendung von Piperazinverbindungen der Formel I nach einem der vorhergehenden Ansprüche, worin R⁴, R⁵ und R⁶ für Wasserstoff stehen.

12. Verwendung von Piperazinverbindungen der Formel I nach einem der vorhergehenden Ansprüche, wobei das Chiralitätszentrum im Piperazinring die (S)-Konfiguration aufweist.

13. Verwendung von Piperazinverbindungen der Formel I nach einem der vorhergehenden Ansprüche, wobei die exo-Doppelbindung am Piperazinring die (Z)-Konformation aufweist.

14. Mittel, enthaltend eine herbizid wirksame Menge mindestens einer Piperazinverbindung der Formel I oder eines landwirtschaftlich brauchbaren Salzes von I nach einem der Ansprüche 1 bis 13 und für die Formulierung von Pflanzenschutzmitteln übliche Hilfsmittel.

15. Verfahren zur Bekämpfung von unerwünschtem Pflanzenwuchs, **dadurch gekennzeichnet, dass** man eine herbizid wirksame Menge mindestens einer Piperazinverbindung der Formel I oder eines landwirtschaftlich brauchbaren Salzes von I nach einem der Ansprüche 1 bis 13 auf Pflanzen, deren Samen und/oder deren Lebensraum einwirken läßt.

16. Piperazinverbindungen der allgemeinen. Formel I gemäß einem der Ansprüche 1 bis 13,
ausgenommen Verbindungen der Formel I, worin A¹ für Phenyl steht und A² 4-Imidazolyl bedeutet oder A¹ für 4-Imidazolyl steht und A² Phenyl bedeutet,
weiterhin ausgenommen die Verbindung der Formel I, worin R¹ für Wasserstoff steht und R² Methyl bedeutet, R³, R⁴, R⁵ und R⁶ Wasserstoff bedeuten, und die Gruppe A¹(R^{a}R^{b}R^{c}) für 4-Methoxyphenyl steht und die Gruppe A²(R^{d}R^{e}R^{f}) für Phenyl steht,
weiterhin ausgenommen Verbindungen der Formel I, worin A¹ für Phenyl steht, R¹ und R² für Methyl stehen, R³, R⁴, R⁵ und R⁶ Wasserstoff bedeuten, R^{a} Benzyloxy, das in der 3-Position gebunden ist, bedeutet, R^{b} und R^{c} für Wasserstoff stehen, und die Gruppe A²(R^{d}R^{e}R^{f}) für Phenyl oder 3-Nitrophenyl steht,
weiterhin ausgenommen Verbindungen der Formel I, worin A¹ für Phenyl steht, R¹ für Wasserstoff, Acetyl oder Isopropyloxycarbonyl und R² für Wasserstoff oder Benzyl stehen, R³, R⁴, R⁵ und R⁶ Wasserstoff bedeuten, R^{a} Benzyloxy, das in der 2-Position gebunden ist, bedeutet, R^{b} und R^{c} gemeinsam für eine Gruppe OCH₂-O stehen, die an die Kohlenstoffatome der 4- und der 5-Position von Phenyl gebunden sind , und die Gruppe A²(R^{d}R^{e}R^{f}) für 3-Methyl-4-methoxyphenyl steht,
weiterhin ausgenommen die Verbindung der Formel I, worin R¹ für Isopropyloxycarbonyl steht und R² für Benzyl steht, R³, R⁴, R⁵ und R⁶ Wasserstoff bedeuten, und die Gruppen A¹(R^{a}R^{b}R^{e}) und A²(R^{d}R^{e}R^{f}) jeweils für für 3,4,5-Trimethoxyphenyl stehen.

## Claims

1. The use of piperazine compounds of the formula I 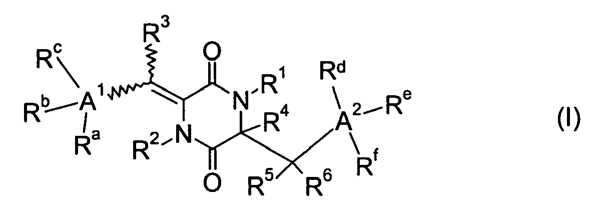 or of the agriculturally useful salts of piperazine compounds of the formula I as herbicides, where in formula I the variables are as defined below:
R¹ and R² independently of one another are:
cyano, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, phenyl, phenyl-(C₁-C₆) -alkyl, heterocyclyl, heterocyclyl-(C₁-C₆)-alkyl; phenyl- [C₁-C₆-alkoxycarbonyl]-(C₁-C₆)-alkyl or phenylheterocyclyl-(C₁-C₆)-alkyl; or
COR²¹, where
R²¹ is hydrogen, C₁-C₆-alkyl, C₃-C₆-cycloalkyl, C₂-C₆-alkenyl, C₃-C₆-cycloalkenyl, C₂-C₆-alkynyl, C₃-C₆-cycloalkynyl, hydroxyl, C₁-C₆-,alkoxy, C₃-C₆-alkenyloxy, C₃-C₆-alkynyloxy, amino, C₁-C₆-alkylamino, [di- (C₁-C₆) -alkyl] amino, C₃-C₆-alkenylamino, C₃-C₆-alkynylamino, C₁-C₆-alkylsulfonylamino, -(C₂-C₆-alkenyl)-N-(C₁-C₆-alkyl) amino, N-(C₂-C₆-alkynyl)-N-(C₁-C₆-alkyl) amino, N-(C₁-C₆-alkoxy)-N- (C₁-C₆-alkyl) amino, N-(C₂-C₆-alkenyl)-N- (C₁-C₆-alkoxy) amino, N-(C₂-C₆-alkynyl) -N-(C₁-C₆-alkoxy)amino, phenyl, phenylamino, phenoxy, naphthyl or heterocyclyl; or
NR²²R²³, where
R²² and R²³ independently of one another are hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl or C₁-C₆-alkylcarbonyl; or
OR²⁴, where
R²⁴ is C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, phenyl or phenyl-(C₁-C₆)-alkyl; or
SO₂R²⁵, where
R²⁵ is C₁-C₆-alkyl or phenyl;
where the abovementioned aliphatic, cyclic or aromatic moieties of the substituents of R¹ and R² may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di- (C₁-C₄) -alkyl] amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy;
and where R¹ may additionally be hydrogen;
R³ is hydrogen, halogen, cyano, C₁-C₆-alkyl, C₃-C₆-cycloalkyl, C₂-C₆-alkenyl, C₃-C₆-cycloalkenyl, C₂-C₆-alkynyl, C₃-C₆-cycloalkynyl, phenyl, phenyl-(C₁-C₆)-alkyl, heterocyclyl, heterocyclyl-(C₁-C₆)-alkyl; phenyl- [C₁-C₆-alkoxycarbonyl]-(C₁-C₆)-alkyl or phenylheterocyclyl-(C₁-C₆)-alkyl; or a radical COR²⁶, NR²⁷R²⁸, OR²⁹, SO₂R³⁰ or N(OR³¹)R³², where
R²⁶ is hydrogen, C₁-C₆-alkyl, C₃-C₆-cycloalkyl, C₂-C₆-alkenyl, C₃-C₆-cycloalkenyl, C₂-C₆-alkynyl, C₃-C₆-cycloalkynyl, hydroxyl, C₁-C₆-alkoxy, C₃-C₆-alkenyloxy, C₃-C₆-alkynyloxy, amino, C₁-C₆-alkylamino, [di- (C₁-C₆)-alkyl]amino, C₃-C₆-alkenylamino, C₃-C₆-alkynylamino, C₁-C₆-alkylsulfonylamino, N-(C₂-C₆-alkenyl) -N- (C₁-C₆-alkyl) amino, N- (C₂-C₆-alkynyl) -N- (C₁-C₆-alkyl) amino, N-(C₁-C₆-alkoxy)-N-(C₁-C₆-alkyl) amino, N-(C₂-C₆-alkenyl) -N- (C₁-C₆-alkoxy) amino, N-(C₂-C₆-alkynyl)-N-(C₁-C₆-alkoxy) amino, phenyl, phenylamino, phenoxy, naphthyl or heterocyclyl;
R²⁷ and R²⁸ independently of one another are hydrogen, C₁-C₆-alkyl, aryl or heteroaryl;
R²⁹ is C₁-C₆-alkyl ;
R³⁰ is C₁-C₆-alkyl or phenyl ;
R³¹ is hydrogen, C₁-C₆-alkyl, phenyl or phenyl-(C₁-C₆)-alkyl ;
R³² is C₁-C₆-alkyl, phenyl or phenyl-(C₁-C₆)-alkyl;
where the abovementioned aliphatic, cyclic or aromatic moieties of the substituents of R³ or R²⁶, R²⁷, R²⁸, R²⁹, R³⁰, R³¹ and R³² may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄) -alkyl] amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄) -alkyl] aminocarbonyl or C₁-C₄-alkylcarbonyloxy;
R⁴, R⁵, R⁶ independently of one another are hydrogen, hydroxyl, C₁-C₆-alkyl, C₁-C₆-alkoxy, where the abovementioned aliphatic moieties of the substituents of R⁴, R⁵ or R⁶ may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄) -alkyl] amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄) -alkyl] aminocarbonyl or C₁-C₄-alkylcarbonyloxy;
A¹ is aryl or heteroaryl;
A² is aryl or heteroaryl, except for indolyl;
R^{a} is halogen, cyano, nitro, C₁-C₆-alkyl, C₃-C₆-cycloalkyl, C₂-C₆-alkenyl, C₃-C₆-cycloalkenyl, C₄-C₁₀-alkadienyl, C₂-C₆-alkynyl, [tri-(C₁-C₆)-alkylsilyl]-(C₂-C₆)-alkynyl, C₃-C₆-cycloalkynyl, C₁-C₆-alkylthio, C₁-C₆-alkylsulfinyl, aryl, phenyl-(C₁-C₆)-alkyl, phenyl-(C₂-C₆)-alkenyl, phenylsulfonyl-(C₁-C₆)-alkyl, heterocyclyl, heterocyclyl-(C₁-C₆)-alkyl or phenyl-[C₁-C₆-alkoxycarbonyl]-(C₁-C₆)-alkyl,
Z¹P(O)(OR⁹)2, Z²B(OR¹⁰)₂, where
R⁹ and R¹⁰ are each hydrogen or C₁-C₆-alkyl and the radicals R¹⁰ in Z²B(OR¹⁰) ₂ together may form a C₂-C₄-alkylene chain; or
Z³COR¹¹, where
R¹¹ is hydrogen, C₁-C₆-alkyl, C₃-C₆-cycloalkyl, C₂-C₆-alkenyl, C₃-C₆-cycloalkenyl, C₂-C₆-alkynyl, C₃₋C₆-cycloalkynyl, hydroxyl, C₁-C₆-alkoxy, C₃-C₆-alkenyloxy, C₃-C₆-alkynyloxy, amino, C₁-C₆-alkylamino, [di- (C₁-C₆)-alkyl]amino, C₁-C₆-alkoxyamino, [di- (C₁-C₆)-alkoxy]amino, C₁-C₆-alkylsulfonylamino, C₁-C₆-alkylaminosulfonylamino, [di-(C₁-C₆)-alkylamino]sulfonylamino, C₃-C₆-alkenylamino, C₃-C₆-alkynylamino, N-(C₂-C₆-alkenyl)-N-(C₁-C₆-alkyl)amino, N-(C₂-C₆-alkynyl)-N-(C₁-C₆-alkyl)amino, N-(C₁-C₆-alkoxy)-N-(C₁-C₆-alkyl)amino, N-(C₂-C₆-alkenyl)-N-(C₁-C₆-alkoxy)amino, N-(C₂-C₆-alkynyl)-N-(C₁-C₆-alkoxy)amino, phenyl, phenoxy, phenylamino, naphthyl or heterocyclyl; or
Z⁴NR¹²R¹³, where
R¹² and R¹³ independently of one another are hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, C₁-C₆-alkylcarbonyl, C₃-C₆-cycloalkylcarbonyl, [di- (C₁-C₆) -alkylamino] carbonyl, C₁-C₆-alkoxycarbonyl, C₁-C₆-alkoxycarbonyl-(C₁-C₆)-alkyl, C₁-C₆-alkylsulfonyl, C₁-C₆-alkylaminosulfonyl, [di-(C₁-C₆)-alkylamino]sulfonyl, phenylcarbonyl, phenylaminocarbonyl, phenylsulfonyl, phenylsulfonylaminocarbonyl or heterocyclylcarbonyl; or
Z⁵CH=N-O-R¹⁴, where R¹⁴ is hydrogen or C₁-C₆-alkyl; or
Z⁶OR¹⁵, where
R¹⁵ is hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, C₁-C₆-alkylcarbonyl, C₁-C₆-alkoxycarbonyl-(C₁-C₆)-alkyl, [di-(C₁-C₆)-alkoxycarbonyl]-(C₁-C₆)-alkyl, phenyl or phenyl-(C₁-C₆)-alkyl; or
Z⁷SO₂R¹⁶, where R¹⁶ is C₁-C₆-alkyl or phenyl; and where
Z¹, Z², Z³, Z⁴, Z⁵, Z⁶, Z⁷ independently of one another are a bond, -CH₂-, -CH₂-CH₂-, -O-CH(R¹⁷)-, -S-CH(R¹⁸)-, -S(O)-CH(R¹⁹)- or -SO₂CH(R²⁰)-, and where R¹⁷, R¹⁸, R¹⁹ and R²⁰ independently of one another are hydrogen or C₁-C₆-alkyl; and
where the abovementioned aliphatic, cyclic or aromatic moieties of the substituent R^{a} may be partially or fully halogenated and/or may carry one to three of the following groups: cyano, hydroxyl, C₁-C₄-alkyl, C₁-C₄-haloalkyl, C₃-C₆-cycloalkyl , C₁-C₄-alkoxy, C₁-C₄-haloalkoxy, C₁-C₄-alkylthio, [di- (C₁-C₄) -alkyl] amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy; and
R^{b}, R^{c}, R^{d}, R^{e} and R^{f} are each independently of one another hydrogen or have one of the meanings given for R^{a}; and
where two radicals R^{a}, R^{b} or R^{c} attached to adjacent ring atoms of A¹ or two radicals R^{d}, R^{e} or R^{f} attached to adjacent ring atoms of A² may also be straight-chain C₃-C₆-alkylene which may be partially or fully halogenated and which may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di- (C₁-C₄) -alkyl] amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy, where one CH₂ group in C₃-C₆-alkylene may be replaced by a carbonyl group, thiocarbonyl group or sulfonyl group and in which one or two non-adjacent CH₂ groups in C₃-C₆-alkylene may in each case be replaced by oxygen, sulfur or a group NR³⁴, where R³⁴ has one of the meanings given for R¹².

2. The use of piperazine compounds of the formula I according to claim 1 in which R^{a} is attached in the ortho-position to the point of attachment of A¹.

3. The use of piperazine compounds of the formula I according to claim 1 in which A¹ is bicyclic aryl or hetaryl.

4. The use of piperazine compounds of the formula I according to any of the preceding claims in which A¹ and A² independently of one another are selected from the group consisting of phenyl, naphthyl, furyl, thienyl, pyrrolyl, pyrazolyl, thiazolyl, isothiazolyl, oxazolyl, isoxazolyl, triazolyl, tetrazolyl, pyridinyl, pyridazinyl, pyrimidinyl, pyrazinyl, triazinyl and tetrazinyl.

5. The use of piperazine compounds of the formula I according to any of claims 1, 2 or 4 in which A¹ and A² independently of one another are selected from the group consisting of phenyl, furyl, thienyl, triazolyl, tetrazolyl and pyridinyl.

6. The use of piperazine compounds of the formula I according to any of claims 1, 2, 4 or 5 in which A¹ is phenyl or pyridinyl.

7. The use of piperazine compounds of the formula I according to any of the preceding claims in which A² is phenyl or thienyl.

8. The use of piperazine compounds of the formula I according to any of the preceding claims in which:
R^{a} is selected from the group consisting of halogen, cyano, nitro, C₁-C₆-alkyl, C₂-C₆-alkenyl, C₂-C₆-alkynyl, C₃-C₆-cycloalkyl, [tri-(C₁-C₆)-alkylsilyl]-C₂-C₆-alkynyl, Z¹P(O) (OR⁹)₂, Z³COR¹¹, Z⁴NR¹²R¹³, Z⁵CH=N-O-R¹⁴, Z⁶OR¹⁵ Z⁷SO₂R¹⁶, C₁-C₆-alkylthio, C₁-C₆-alkylsulfinyl, aryl and heterocyclyl, where
Z¹ is a bond or CH₂ and R⁹ and R¹⁰ are each hydrogen or C₁-C₆-alkyl ;
Z³ is a bond and R¹¹ is hydrogen, C₁-C₆-alkyl, hydroxyl, C₁-C₆-alkoxy, C₃-C₆-alkenyloxy, C₃-C₆-alkynyloxy, amino, C₁-C₆-alkylamino, [di-(C₁-C₆)-alkyl] amino, C₁-C₆-alkoxyamino, (C₁-C₆)-alkoxy-(C₁-C₆)-alkylamino, C₁-C₆-alkylsulfonylamino, C₁-C₆-alkylaminosulfonylamino, [di-(C₁-C₆)-alkylamino]sulfonylamino, phenyl, phenoxy, phenylamino, naphthyl or heterocyclyl; or
Z⁴ is a bond or CH₂ and R¹² and R¹³ independently of one another are hydrogen, C₁-C₆-alkyl, C₃-C₆-cycloalkyl, C₃-C₆-alkenyl, C₃-C₆-alkynyl, C₁-C₆-alkylcarbonyl, [di-(C₁-C₆)-alkylamino]carbonyl, C₁-C₆-alkoxycarbonyl , C₁-C₆-alkylsulfonyl, phenylcarbonyl, phenylsulfonyl, or heterocyclylcarbonyl; or
Z⁵ is a bond and R¹⁴ is hydrogen or C₁-C₆-alkyl; or
Z⁶ is a bond or CH₂ and R¹⁵ is hydrogen, C₁-C₆-alkyl, C₃-C₆-cycloalkyl, C₃-C₆-alkenyl, C₃-C₆-alkynyl, C₁-C₆-alkylcarbonyl , C₁-C₆-alkoxycarbonyl-(C₁-C₆)-alkyl, phenyl or phenyl-(C₁-C₆)-alkyl; or
Z⁷ is a bond and R¹⁶ is C₁-C₆-alkyl or phenyl;
R^{b}, R^{c}, R^{d}, R^{e} and R^{f} independently of one another are hydrogen or have one of the meanings mentioned for R^{a},
and where the abovementioned aliphatic, cyclic or aromatic moieties of the substituents R^{a}, R^{b}, R^{c}, R^{d}, R^{e} and R^{f} may be partially or fully halogenated.

9. The use of piperazine compounds of the formula I according to any of the preceding claims in which
R¹ is hydrogen or C₁-C₆-alkyl; and
R² is C₁-C₆-alkyl ;
where C₁-C₆-alkyl in R¹ and R² may be partially or fully halogenated.

10. The use of piperazine compounds of the formula I according to any of the preceding claims in which R³ is hydrogen, halogen, C₁-C₆-alkyl or halo-C₁-C₆-alkyl.

11. The use of piperazine compounds of the formula I according to any of the preceding claims in which R⁴, R⁵ and R⁶ are hydrogen.

12. The use of piperazine compounds of the formula I according to any of the preceding claims in which the center of chirality in the piperazine ring has the (S)-configuration.

13. The use of piperazine compounds of the formula I according to any of the preceding claims in which the exo double bond at the piperazine ring has the (Z)-conformation.

14. A composition comprising a herbicidally effective amount of at least one piperazine compound of the formula I or an agriculturally useful salt of I according to any of claims 1 to 13 and auxiliaries customary for formulating crop protection agents.

15. A method for controlling unwanted vegetation which comprises allowing a herbicidally effective amount of at least one piperazine compound of the formula I or an agriculturally useful salt of I according to any of claims 1 to 13 to act on plants, their seeds and/or their habitat.

16. A piperazine compound of the general formula I according to any of claims 1 to 13,
except for compounds of the formula I in which A¹ is phenyl and A² is 4-imidazolyl or A¹ is 4-imidazolyl and A² is phenyl,
furthermore except for the compound of the formula I in which R¹ is hydrogen and R² is methyl, R³, R⁴, R⁵ and R⁶ are hydrogen and the group A¹( R^{a}R^{b}R^{c} ) is 4-methoxyphenyl and the group A² (R^{d}R^{e}R^{f}) is phenyl,
furthermore except for compounds of the formula I in which A¹ is phenyl, R¹ and R² are methyl, R³, R⁴, R⁵ and R⁶ are hydrogen, R^{a} is benzyloxy which is attached in the 3-position, R^{b} and R^{c} are hydrogen and the group A²(R^{d}R^{e}R^{f}) is phenyl or 3-nitrophenyl,
furthermore except for compounds of the formula I in which A¹ is phenyl, R¹ is hydrogen, acetyl or isopropyloxycarbonyl and R² is hydrogen or benzyl, R³, R⁴, R⁵ and R⁶ are hydrogen, R^{a} is benzyloxy which is attached in the 2-position, R^{b} and R^{c} together are a group OCH₂-O which is attached to the carbon atoms in the 4- and the 5-position of phenyl and the group A²(R^{d}R^{e}R^{f}) is 3-methyl-4-methoxyphenyl,
furthermore except for the compound of the formula I in which R¹ is isopropyloxycarbonyl and R² is benzyl, R³, R⁴, R⁵ and R⁶ are hydrogen and the groups A¹(R^{a}R^{b}R^{c}) and A²(R^{d}R^{e}R^{f}) are each 3,4,5-trimethoxyphenyl.

## Revendications

1. Utilisation de composés de type pipérazine de formule I 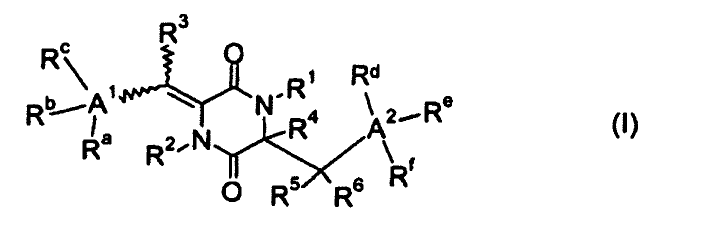 ou des sels utilisables en agriculture des composés de type pipérazine de formule I, en tant qu'herbicides, les variables dans la formule I ayant les significations suivantes :
R¹ et R² représentent, indépendamment l'un de l'autre,
un groupe cyano, alkyle en C₁-C₆, cycloalkyle en C₃-C₆, alcényle en C₃-C₆, cycloalcényle en C₃-C₆, alcynyle en C₃-C₆, cycloalcynyle en C₃-C₆, phényle, phényl-alkyle(C₁-C₆), hétérocyclyle, hétérocyclyl-alkyle(C₁-C₆) ; phényl- [alcoxy(C₁-C₆)-carbonyl]-alkyle (C₁-C₆) ou phénylhétérocyclyl-alkyle(C₁-C₆) ; ou
COR²¹, où
R²¹ représente un atome d'hydrogène, un groupe alkyle en C₁-C₆, cycloalkyle en C₃-C₆, alcényle en C₂-C₆, cycloalcényle en C₃-C₆, alcynyle en C₂-C₆, cycloalcynyle en C₃-C₆, hydroxy, alcoxy en C₁-C₆, alcényl (C₃-C₆)oxy, alcynyl(C₃-C₆)oxy, amino, alkyl(C₁-C₆)-amino, [dialkyl(C₁-C₆)]amino, alcényl-(C₃-C₆)amino, alcynyl(C₃-C₆)amino, alkyl-(C₁-C₆)sulfonylamino, N-[alcényl(C₂-C₆)]-N-[alkyl(C₁-C₆)]-amino, N-[alcynyl-(C₂-C₆)]-N-[alkyl(C₁-C₆)]-amino, N-[alcoxy(C₁-C₆)]-N-[alkyl(C₁-C₆)]-amino, N-[alcényl(C₂-C₆)]-N-[alcoxy(C₁-C₆)]-amino, N-[alcynyl(C₂-C₆)]-N-[alcoxy(C1-C6)]-amino, phényle, phénylamino, phénoxy, napthyle ou hétérocyclyle ; ou
NR²²R²³ où
R²² et R²³ représentent, indépendamment l'un de l'autre, un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆ ou alkyl(C₁-C₆)-carbonyle; ou
OR²⁴ où
R²⁴ représente un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆, phényle ou phényl-alkyle(C₁-C₆) ; ou
SO₂R²⁵, où
R²⁵ représente un groupe alkyle en C₁-C₆ ou phényle ;
les fragments aliphatiques, cycliques ou aromatiques nommés des substituants de R¹ et R² pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄)carbonyle, aminocarbonyle, alkyl(C₁-C₄)amino-carbonyle, [dialkyl(C₁-C₄)]amino-carbonyle ou alkyl (C₁-C₄)carbonyloxy ;
et R¹ pouvant représenter en outre un atome d'hydrogène ;
R³ représente un atome d'hydrogène ou d'halogène ou un groupe cyano, alkyle en C₁-C₆, cycloalkyle en C₃-C₆, alcényle en C₂-C₆, cycloalcényle en C₃-C₆, alcynyle en C₂-C₆, cycloalcynyle en C₃-C_{6,} phényle, phényl-alkyle(C₁-C₆), hétérocyclyle, hétérocyclyl-alkyle (C₁-C₆), phényl-[alcoxy(C₁-C₆)-carbonyle]-alkyle(C₁-C₆) ou phényl-hétérocyclyl-alkyle(C₁-C₆) ; ou un radical COR²⁶, NR²⁷R²⁸, OR²⁹, SO₂R³⁰ ou N(OR³¹)R³²,
R²⁶ représentant un atome d'hydrogène, un groupe alkyle en C₁-C₆, cycloalkyle en C₃-C₆, alcényle en C₂-C₆, cycloalcényle en C₃-C₆, alcynyle en C₂-C₆, cycloalcynyle en C₃-C_{6,} hydroxy, alcoxy en C₁-C₆, alcényl(C₃-C₆)oxy, alcynyl(C₃-C₆)oxy, amino, alkyl(C₁-C₆)amino, [dialkyl(C₁-C₆)]amino, alcényl(C₃-C₆)amino, alcynyl(C₃-C₆)amino, alkyl(C₁-C₆)sulfonylamino, N-[alcényl(C₂-C₆)]-N-[alkyl(C₁-C₆)]-amino, N-[alcynyl(C₂-C₆)]-N-[alkyl (C1-C₆)]-amino, N-[alcoxy(C₁-C₆)]-N-[alkyl(C₁-C₆)]-amino, N-[alcényl(C₂-C₆)]-N-[alcoxy(C₁-C₆)]amino, N-[alcynyl(C₂-C₆)]-N-[alcoxy(C₁-C₆)]-amino, phényle, phénylamino, phénoxy, naphtyle ou hétérocyclyle ;
R²⁷ et R²⁸ représentant, indépendamment l'un de l'autre, un atome d'hydrogène, un groupe alkyle en C₁-C₆, aryle ou hétéroaryle ;
R²⁹ représentant un groupe alkyle en C₁-C₆,
R³⁰ représentant un groupe alkyle en C₁-C₆ ou phényle ;
R³¹ représentant un atome d'hydrogène, un groupe alkyle en C₁-C₆, phényle ou phényl-alkyle(C₁-C₆) ;
R³² représentant un groupe alkyle en C₁-C₆, phényle ou phényl-alkyle(C₁-C₆) ;
les fragments aliphatiques, cycliques ou aromatiques nommés des substituants de R³ ou R²⁶ , R²⁷ ,R²⁸ , R²⁹ , R³⁰ , R³¹ et R³² pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄) carbonyle, aminocarbonyle, alkyl(C₁-C₄)amino-carbonyle, [dialkyl(C₁-C₄)]amino-carbonyle ou alkyl(C₁-C₄)carbonyloxy ;
R⁴ , R⁵ , R⁶ représentent, chacun indépendamment, un atome d'hydrogène, un groupe hydroxy, alkyle en C₁-C₆, alcoxy en C₁-C₆, les fragments aliphatiques nommés des substituants de R⁴, R⁵ ou R⁶ pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁₋C₄) carbonyle, aminocarbonyle, alkyl(C₁-C₄)amino-carbonyle, [dialkyl(C₁-C₄)]amino-carbonyle ou alkyl(C₁-C₄)carbonyloxy ;
A¹ représente un groupe aryle ou hétéroaryle ;
A² représente un groupe aryle ou hétéroaryle, hormis indolyle ;
R^{a} représente un atome d'halogène, un groupe cyano, nitro, alkyle en C₁-C₆, cycloalkyle en C₃-C₆, alcényle en C₂-C₆, cycloalcényle en C₃-C₆, alcadiényle en C₄-C₁₀, alcynyle en C₂-C₆, [trialkyl (C₁-C₆) silyl] -alcynyle (C₂-C₆), cycloalcynyle en C₃-C₆, alkyl (C₁-C₆) thio, alkyl(C₁-C₆)sulfinyle, aryle, phényl-alkyle (C₁-C₆) , phényl-alcényle (C₂-C₆) , phénylsulfonyl-alkyle(C₁-C₆), hétérocyclyle, hétérocyclyl-alkyle(C₁-C₆) ou phényl-[alcoxy(C₁-C₆)carbonyl]-alkyle(C₁-C₆),
Z¹P(O)(OR⁹)₂, Z²B(OR¹⁰)₂,
R⁹ et R¹⁰ représentant chacun un atome d'hydrogène ou un groupe alkyle en C₁-C₆ et les radicaux R¹⁰ dans Z²B(OR¹⁰)₂ pouvant former ensemble une chaîne alkylène en C₂-C₄ ; ou
Z³COR¹¹,
R¹¹ représentant un atome d'hydrogène, un groupe alkyle en C₁-C₆, cycloalkyle en C₃-C₆, alcényle en C₂-C₆, cycloalcényle en C₃-C₆, alcynyle en C₂-C₆, cycloalcynyle en C₃-C₆, hydroxy, alcoxy en C₁-C₆, alcényl(C3-C6)oxy, alcynyl (C3-C6)oxy, amino, alkyl(C₁-C₆)amino, [dialkyl(C₁-C₆)]amino, alcoxy(C₁-C₆)amino, [dialcoxy(C₁-C₆)]amino, alkyl(C₁-C₆)sulfonylamino, alkyl(C₁-C₆)amino-sulfonylamino, [dialkyl(C₁-C₆)amino]sulfonylamino, alcényl(C₃-C₆)amino, alcynyl(C₃-C₆)amino, N-[alcényl(C₂-C₆)]-N-[alkyl(C₁-C₆)]-amino, N-[alcynyl(C₂-C₆)]-N-[alkyl(C₁-C₆)]-amino, N-[alcoxy(C₁-C₆)]-N-[alkyl(C₁-C₆)]-amino, N-[alcényl(C₂-C₆)]-N-[alcoxy(C₁-C₆)]-amino, N-[alcynyl(C₂-C₆)]-N-[alcoxy(C₁₋C₆)]amino, phényle, phénoxy, phénylamino, naphtyle ou hétérocyclyle ; ou
Z⁴NR¹²R¹³,
R¹² et R¹³ représentant, indépendamment l'un de l'autre, un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆, alkyl(C₁-C₆)carbonyle, cycloalkyl(C₃-C₆)carbonyle, [dialkyl(C₁-C₆)amino]carbonyle, alcoxy(C₁-C₆)carbonyle, alcoxy(C₁-C₆)carbonyl-alkyle(C₁-C₆) , alkyl(C₁-C₆)sulfonyle, alkyl(C₁-C₆)aminosulfonyle, [dialkyl(C₁-C₆)amino]sulfonyle, phénylcarbonyle, phénylaminocarbonyle, phénylsulfonyle, phénylsulfonylaminocarbonyle ou hétérocyclylcarbonyle ; ou
Z⁵CH=N-O-R¹⁴, R¹⁴ représentant un atome d'hydrogène ou un groupe alkyle en C₁-C₆ ; ou
Z⁶OR¹⁵,
R¹⁵ représentant un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆, alkyl(C₁-C₆)carbonyle, alcoxy(C₁-C₆)carbonyl-alkyle(C₁-C₆), [dialcoxy(C₁-C₆)carbonyl]-alkyle(C₁-C₆) , phényle ou phényl-alkyle(C₁-C₆) ; ou
Z⁷SO₂R¹⁶, R¹⁶ représentant un groupe alkyle en C₁-C₆ ou phényle ; et
Z¹, Z², Z³, Z⁴, Z⁵, Z⁶, Z⁷ représentant, indépendamment les uns des autres, une liaison, -CH₂-, -CH₂-CH₂-, -O-CH(R¹⁷)-, -S-CH(R¹⁸)-, -S(O)-CH(R¹⁹) - ou -SO₂CH(R²⁰) -, et R¹⁷, R¹⁸, R¹⁹ et R²⁰ représentant, indépendamment les uns des autres, un atome d'hydrogène ou un groupe alkyle en C₁-C₆ ;
les fragments aliphatiques, cycliques ou aromatiques nommés du substituant R^{a} pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, halogénoalcoxy en C₁-C₄, alkyl(C₁-C₄) thio, [dialkyl(C₁-C₄)]-amino, alkyl(C₁-C₄)carbonyle, hydroxycarbonyle, alcoxy(C₁-C₄) carbonyle, aminocarbonyle, alkyl (C₁-C₄) amino-carbonyle, [dialkyl(C₁-C₄)]amino-carbonyle ou alkyl(C₁-C₄)carbonyloxy ; et
R^{b}, R^{c}, R^{d}, R^{e} et R^{f}, indépendamment les uns des autres, représentent chacun un atome d'hydrogène ou ont chacun l'une des significations indiquées pour R^{a} ; et
dans laquelle deux radicaux R^{a}, R^{b} ou R^{c} liés à des atomes contigus formant le cycle de A1 ou deux radicaux R^{d}, R^{e} ou R^{f} liés à des atomes contigus formant le cycle de A² peuvent également représenter un groupe alkylène linéaire en C₃-C₆ qui peut être partiellement ou totalement halogéné et qui peut porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)] - amino, alkyl(C₁-C₄)carbonyle, hydroxycarbonyle, alcoxy(C₁-C₄)carbonyle, aminocarbonyle, alkyl(C₁-C₄)amino-carbonyle, [dialkyl(C₁-C₄)]amino-carbonyle ou alkyl(C₁-C₄)carbonyloxy, un groupe CH₂ dans le groupe alkylène en C₃-C₆ pouvant être remplacé par un groupe carbonyle, un groupe thiocarbonyle ou un groupe sulfonyle et un ou deux groupes CH₂ non contigus dans le groupe alkylène en C₃-C₆ pouvant être remplacés chacun par un atome d'oxygène ou de soufre ou par un groupe NR³⁴ , R³⁴ ayant l'une des significations données pour R¹².

2. Utilisation des composés de type pipérazine de formule I selon la revendication 1, dans lesquels R^{a} est lié en position ortho par rapport au point de liaison de A¹.

3. Utilisation des composés de type pipérazine de formule I selon la revendication 1, dans lesquels A¹ est un groupe aryle ou hétéroaryle bicyclique.

4. Utilisation des composés de type pipérazine de formule I selon l'une quelconque des revendications précédentes, dans lesquels A¹ et A² sont choisis, indépendamment l'un de l'autre, dans l'ensemble constitué par les groupes phényle, naphtyle, furyle, thiényle, pyrrolyle, pyrazolyle, thiazolyle, isothiazolyle, oxazolyle, isoxazolyle, triazolyle, tétrazolyle, pyridinyle, pyridazinyle, pyrimidinyle, pyrazinyle, triazinyle et tétrazinyle.

5. Utilisation des composés de type pipérazine de formule I selon l'une quelconque des revendications 1, 2 et 4, dans lesquels A¹ et A² sont choisis, indépendamment l'un de l'autre, dans l'ensemble constitué par les groupes phényle, furyle, thiényle, triazolyle, tétrazolyle et pyridinyle.

6. Utilisation des composés de type pipérazine de formule I selon l'une quelconque des revendications 1, 2, 4 et 5, dans lesquels A¹ représente le groupe phényle ou pyridinyle.

7. Utilisation des composés de type pipérazine de formule I selon l'une quelconque des revendications précédentes, dans lesquels A² représente le groupe phényle ou thiényle.

8. Utilisation des composés de type pipérazine de formule I selon l'une quelconque des revendications précédentes, dans lesquels
R^{a} est choisi parmi un atome d'halogène, un groupe cyano, nitro, alkyle en C₁-C₆, alcényle en C₂-C₆, alcynyle en C₂-C₆, cycloalkyle en C₃-C₆, [trialkyl(C₁-C₆)silyl]-alcynyle(C₂-C₆), Z¹P(O) (OR⁹)₂, Z³COR¹¹, Z⁴NR¹²R¹³, Z⁵CH=N-O-R¹⁴, Z⁶OR¹⁵, Z⁷SO₂R¹⁶, alkyl(C₁-C₆)thio, alkyl(C₁-C₆)sulfinyle, aryle et hétérocyclyle,
Z¹ représentant une liaison ou CH₂ et R⁹ et R¹⁰ représentant chacun un atome d'hydrogène ou un groupe alkyle en C₁-C₆ ;
Z³ représentant une liaison et R¹¹ représentant un atome d'hydrogène, un groupe alkyle en C₁-C₆, hydroxy, alcoxy en C₁-C₆, alcényl(C₃-C₆)oxy, alcynyl (C₃-C₆)oxy, amino, alkyl(C₁-C₆)amino, [dialkyl(C₁-C₆)]amino, alcoxy(C₁-C₆)amino, alcoxy(C₁-C₆)-alkyl(C₁-C₆)amino, alkyl(C₁-C₆)sulfonylamino, alkyl(C₁-C₆)aminosulfonylamino, [dialkyl(C₁-C₆)amino]sulfonylamino, phényle, phénoxy, phénylamino, naphtyle ou hétérocyclyle ; ou
Z⁴ représentant une liaison ou CH₂ et R¹² et R¹³ représentant, indépendamment l'un de l'autre, un atome d'hydrogène ou un groupe alkyle en C₁-C₆, cycloalkyle en C₃-C₆, alcényle en C₃-C₆, alcynyle en C₃-C₆, alkyl(C₁-C₆)carbonyle, [dialkyl(C₁-C₆)amino]carbonyle, alcoxy(C₁-C₆) carbonyle, alkyl(C₁-C₆)sulfonyle, phénylcarbonyle, phénylsulfonyle ou hétérocyclylcarbonyle ; ou
Z⁵ représentant une liaison et R¹⁴ représentant un atome d'hydrogène ou un groupe alkyle en C₁-C₆ ; ou
Z⁶ représentant une liaison ou CH₂ et R¹⁵ représentant un atome d'hydrogène ou un groupe alkyle en C₁-C₆, cycloalkyle en C₃-C₆, alcényle en C₃-C₆, alcynyle en C₃-C₆, alkyl(C₁-C₆)carbonyle, alcoxy(C₁-C₆)carbonyl-alkyle(C₁-C₆), phényle ou phényl-alkyle(C₁-C₆) ; ou
Z⁷ représentant une liaison et R¹⁶ représentant un groupe alkyle en C₁-C₆ ou phényle ;
R^{b}, R^{c}, R^{d}, R^{e} et R^{f}, indépendamment les uns des autres, représentent un atome d'hydrogène ou ont l'un des significations indiquées pour R^{a} ;
et les fragments aliphatiques, cycliques ou aromatiques nommés des substituants R^{a}, R^{b}, R^{c}, R^{d}, R^{e} et R^{f} pouvant être partiellement ou totalement halogénés.

9. Utilisation des composés de type pipérazine de formule I selon l'une quelconque des revendications précédentes, dans lesquels
R¹ représente un atome d'hydrogène ou un groupe alkyle en C₁-C₆ ;
R² représente un groupe alkyle en C₁-C₆ ;
les groupes alkyle en C₁-C₆ dans R¹ et R² pouvant être partiellement ou totalement halogénés.

10. Utilisation des composés de type pipérazine de formule I selon l'une quelconque des revendications précédentes, dans lesquels R³ représente un atome d'hydrogène ou d'halogène ou un groupe alkyle en C₁-C₆ ou halogénoalkyle en C₁₋C₆.

11. Utilisation des composés de type pipérazine de formule I selon l'une quelconque des revendications précédentes, dans lesquels R⁴, R⁵ et R⁶ représentent un atome d'hydrogène.

12. Utilisation des composés de type pipérazine de formule I selon l'une quelconque des revendications précédentes, dans lesquels le centre de chiralité dans le cycle pipérazine présente la configuration (S).

13. Utilisation des composés de type pipérazine de formule I selon l'une quelconque des revendications précédentes, dans lesquels la double liaison exo sur le cycle pipérazine présente la configuration (Z).

14. Composition contenant une quantité, efficace en tant qu'herbicide, d'au moins un composé pipérazine de formule I ou d'un sel de I utilisable en agriculture, selon l'une quelconque des revendications 1 à 13, et des adjuvants usuels pour la formulation de produits phytosanitaires.

15. Procédé pour la lutte contre la croissance de plantes indésirables, **caractérisé en ce qu'**on laisse agir sur des plantes, leurs semences et/ou leur habitat une quantité, efficace en tant qu'herbicide, d'au moins un composé de type pipérazine de formule I ou d'un sel de I utilisable en agriculture, selon l'une quelconque des revendications 1 à 13.

16. Composés de type pipérazine de formule générale I selon l'une quelconque des revendications 1 à 13,
à l'exclusion des composés de formule I dans lesquels A¹ représente le groupe phényle et A² représente le groupe 4-imidazolyle ou A¹ représente le groupe 4-imidazolyle et A² représente le groupe phényle,
en outre à l'exclusion du composés de formule I dans lequel R¹ représente un atome d'hydrogène et R² représente le groupe méthyle, R³, R⁴, R⁵ et R⁶ représentent un atome d'hydrogène, et le groupe A¹(R^{a}R^{b}R^{c}) représente le groupe 4-méthoxyphényle, et le groupe A²(R^{d}R^{e}R^{f}) représente le groupe phényle,
en outre à l'exclusion des composés de formule I dans lesquels A¹ représente le groupe phényle, R¹ et R² représentent le groupe méthyle, R³, R⁴, R⁵ et R⁶ représente un atome d'hydrogène, R^{a} représente le groupe benzyloxy qui est lié en la position 3, R^{b} et R^{c} représentent un atome d'hydrogène, et le groupe A²(R^{d}R^{e}R^{f}) représente le groupe phényle ou 3-nitrophényle,
en outre à l'exclusion des composés de formule I dans lesquels A¹ représente le groupe phényle, R¹ représente un atome d'hydrogène, le groupe acétyle ou isopropyloxycarbonyle et R² représente un atome d'hydrogène ou le groupe benzyle, R³, R⁴ R⁵ et R⁶ représentent un atome d'hydrogène, R^{a} représente le groupe benzyloxy qui est lié en position 2, R^{b} et R^{c} représentent ensemble un groupe OCH₂-O en étant liés à la position 4 et à la position 5 du groupe phényle, et le groupe A²(R^{d}R^{e}R^{f}) représente le groupe 3-méthyl-4-méthoxyphényle,
en outre à l'exclusion des composés de formule I dans lesquels R¹ représente le groupe isopropyloxycarbonyle et R² représente le groupe benzyle, R³, R⁴ R⁵ et R⁶ représentent un atome d'hydrogène, et les groupes A¹(R^{a}R^{b}R^{c}) et A²(R^{d}R^{e}R^{f}) représentent chacun le groupe 3,4,5-triméthoxyphényle.
